# EUROPEAN PATENT SPECIFICATION

(11) **EP 0 989 805 B1**
(45) Date of publication and mention of the grant of the patent: **11.01.2006**
(21) Application number: 98911434.3
(22) Date of filing: 27.02.1998
(51) Int. Cl.: A01N 43/04, C07H 21/02, C07H 21/04, C12N 5/10, C12N 15/00, C12N 15/09, C12N 15/11, C12N 15/12, C12N 15/63, C12N 15/70, C12N 15/74, C12N 15/79

(54) **NUCLEIC ACID SEQUENCES FOR ATP-BINDING CASSETTE TRANSPORTER**
NUKLEINSÄURESEQUENZEN FÜR ATP-BINDUNGS CASSETTE-TRANSPORTER
SEQUENCES D'ACIDES NUCLEIQUES POUR TRANSPORTEUR A CASSETTE DE FIXATION A L'ATP

(30) Priority: 27.02.1997 US 39388 P
(43) Date of publication of application: 05.04.2000
(73) Proprietor: BAYLOR COLLEGE OF MEDICINE, Houston, TX 77030 (US); Johns Hopkins University, Baltimore, MD 21205 (US); THE UNITED STATES OF AMERICA, as represented by the Secretary of the Department of Health and Human Services, Rockville, Maryland 20852 (US); University of Utah Research Foundation, Salt Lake City, Utah 84108 (US)
(72) Inventor: ALLIKMETS, Rando, Frederick, MD 21703 (US); ANDERSON, Kent, L., Houston, TX 77254 (US); DEAN, Michael, Frederick, MD 21703 (US); LEPPART, Mark, Salt Lake City, UT 84105 (US); LEWIS, Richard, A., Houston, TX 77024 (US); LI, Yixin, Cupertino, CA 95014 (US); LUPSKI, James, R., Houston, TX 77096 (US); NATHANS, Jeremy, Baltimore, MD 21209 (US); RATTNER, Amir, Baltimore, MD 21209 (US); SHROYER, Noah, F., Houston, TX 77006 (US); SINGH, Nanda, Salt Lake City, UT 84106 (US); SMALLWOOD, Philip, M., Woodbine, MD 21797 (US); SUN, Hui, Baltimore, MD 21229 (US)
(74) Representative: Hallybone, Huw George
(86) International application number: PCT/US1998/003895
(87) International publication number: WO 1998/037764

(56) References cited:
- ALLIKMETS R ET AL: "CHARACTERIZATION OF THE HUMAN ABC SUPERFAMILY: ISOLATION AND MAPPING OF 21 NEW GENES USING THE EXPRESSED SEQUENCE TAGS DATABASE" HUMAN MOLECULAR GENETICS, OXFORD UNIVERSITY PRESS, SURREY, GB, vol. 5, no. 10, 1996, pages 1649-1655, XP002074412 ISSN: 0964-6906
- DATABASE EMBL [Online] Entry HS947245, Acc.no. H91947, 1 December 1995 (1995-12-01) HILLIER, L. ET AL.: "ys81h09.r1 Soares retina N2b4HR Homo sapiens cDNA clone IMAGE:221249 5' similar to SP:C48B4.5 CE00488 ATP-BINDING TRANSPORT PROTEIN ;, mRNA sequence." XP002191077
- ILLING MICHELLE ET AL: "The 220-kDa rim protein of retinal rod outer segments is a member of the ABC transporter superfamily." JOURNAL OF BIOLOGICAL CHEMISTRY, vol. 272, no. 15, 1997, pages 10303-10310, XP002191076 ISSN: 0021-9258
- ALLIKMETS R ET AL: "Mutation of the Stargardt Disease gene (ABCR) in Age-related Macular Degeneration" SCIENCE, AMERICAN ASSOCIATION FOR THE ADVANCEMENT OF SCIENCE,, US, vol. 277, 19 September 1997 (1997-09-19), pages 1805-1807, XP002099899 ISSN: 0036-8075
- ALLIKMETS R ET AL: "A PHOTORECEPTOR CELL-SPECIFIC ATP-BINDING TRANSPORTER GENE (ABCR) IS MUTATED IN RECESSIVE STARGARDT MACULAR DYSTROPHY" NATURE GENETICS, NEW YORK, NY, US, vol. 15, no. 3, March 1997 (1997-03), pages 236-246, XP000876817 ISSN: 1061-4036
- GERBER S ET AL: "COMPLETE EXON-INTRON STRUCTURE OF THE RETINA-SPECIFIC ATP BINDING TRANSPORTER GENE (ABCR) ALLOWS THE IDENTIFICATION OF NOVEL MUTATIONS UNDERLYING STARGARDT DISEASE" GENOMICS, ACADEMIC PRESS, SAN DIEGO, US, vol. 48, 1998, pages 139-142, XP002911846 ISSN: 0888-7543
- AZARIAN S M ET AL: "THE PHOTORECEPTOR RIM PROTEIN IS AN ABC TRANSPORTER ENCODED BY THE GENE FOR RECESSIVE STARGARDT'S DISEASE. (ABCR)" FEBS LETTERS, ELSEVIER SCIENCE PUBLISHERS, AMSTERDAM, NL, vol. 409, no. 2, 9 June 1997 (1997-06-09), pages 247-252, XP000876822 ISSN: 0014-5793
- NASONKIN IGOR ET AL: "Mapping of the rod photoreceptor ABC transporter (ABCR) to 1p21-p22.1 and identification of novel mutations in Stargardt's disease." HUMAN GENETICS, vol. 102, no. 1, January 1998 (1998-01), pages 21-26, XP002191075 ISSN: 0340-6717
- GERBER S. et al., "Complete Exon-Intron Structure of the Retina-Specific ATP Binding Transporter Gene (ABCR) Allows Identification of Novel Mutations Underlying Stargardt Disease", GENOMICS, 1998, Vol. 48, pages 139-142, XP002911846
- HOYNG C.B. et al., "Genetic Fine Mapping of the Gene for Recessive Stargardt Disease", HUMAN GENETICS, 1996, Vol. 98, pages 500-504, XP002911847
- SUN H. et al., "Stargardt's ABCR is Localized to the Disc Membrane of Retinal Rod Outer Segments", NATURE GENETICS, 17 September 1997, Vol. 17, pages 15-16, XP002911848
- ALLIKMETS R. et al., "A Photoreceptor Cell-Specific ATP-Binding Transporter Gene (ABCR) is Mutated in Recessive Stargardt Macular Dystrophy", NATURE GENETICS, 15 March 1997, Vol. 15, pages 236-245, XP002911849

### SEQUENCE LISTING

<110> Baylor College of Medicine
   Johns Hopkins University
   United States of America
   University of Utah
   Allikmets, Rando
   Anderson, Kent L.
   Dean, Michael
   Leppert, Mark
   Lewis, Richard A.
   Li, Yixin
   Lupski, James R.
   Nathans, Jeremy
   Rattner, Amir
   Shroyer, Noah F.
   Singh, Nanda
   Smallwood, Philip M.
   Sun, Hui
<120> NUCLEIC ACID SEQUENCES FOR ATP-BINDING CASSETTE TRANSPORTER
<130> BYLR-0079
<140> PCT/US98/03895
   <141> 1998-02-27
<150> US 60/039,388
   <151> 1997-02-27
<160> 120
<170> PatentIn version 3.3
<210> 1
   <211> 7782
   <212> DNA
   <213> Homo sapiens
<400> 1
   cccctacccc tctgctaagc tcagggataa cccaactagc tgaccataat gacttcagtc 60
   attacggagc aagatgaaag actaaaagag ggagggatca cttcagatct gccgagtgag 120
   tcgattggac ttaaagggcc agtcaaaccc tgactgccgg ctcatggcag gctcttgccg 180
   aggacaaatg cccagcctat atttatgcaa agagattttg ttccaaactt aaggtcaaag 240
   atacctaaag acatccccct caggaacccc tctcatggag gagagtgcct gagggtcttg 300
   gtttcccatt gcatccccca cctcaatttc cctggtgccc agccacttgt gtctttaggg 360
   ttctctttct ctccataaaa gggagccaac acagtgtcgg cctcctctcc ccaactaagg 420
   gcttatgtgt aattaaaagg gattatgctt tgaaggggaa aagtagcctt taatcaccag 480
   gagaaggaca cagcgtccgg agccagaggc gctcttaacg gcgtttatgt cctttgctgt 540
   ctgaggggcc tcagctctga ccaatctggt cttcgtgtgg tcattagcat gggcttcgtg 600
   agacagatac agcttttgct ctggaagaac tggaccctgc ggaaaaggca aaagattcgc 660
   tttgtggtgg aactcgtgtg gcctttatct ttatttctgg tcttgatctg gttaaggaat 720
   gccaacccgc tctacagcca tcatgaatgc catttcccca acaaggcgat gccctcagca 780
   ggaatgctgc cgtggctcca ggggatcttc tgcaatgtga acaatccctg ttttcaaagc 840
   cccaccccag gagaatctcc tggaattgtg tcaaactata acaactccat cttggcaagg 900
   gtatatcgag attttcaaga actcctcatg aatgcaccag agagccagca ccttggccgt 960
   atttggacag agctacacat cttgtcccaa ttcatggaca ccctccggac tcacccggag 1020
   agaattgcag gaagaggaat acgaataagg gatatcttga aagatgaaga aacactgaca 1080
   ctatttctca ttaaaaacat cggcctgtct gactcagtgg tctaccttct gatcaactct 1140
   caagtccgtc cagagcagtt cgctcatgga gtcccggacc tggcgctgaa ggacatcgcc 1200
   tgcagcgagg ccctcctgga gcgcttcatc atcttcagcc agagacgcgg ggcaaagacg 1260
   gtgcgctatg ccctgtgctc cctctcccag ggcaccctac agtggataga agacactctg 1320
   tatgccaacg tggacttctt caagctcttc cgtgtgcttc ccacactcct agacagccgt 1380
   tctcaaggta tcaatctgag atcttgggga ggaatattat ctgatatgtc accaagaatt 1440
   caagagttta tccatcggcc gagtatgcag gacttgctgt gggtgaccag gcccctcatg 1500
   cagaatggtg gtccagagac ctttacaaag ctgatgggca tcctgtctga cctcctgtgt 1560
   ggctaccccg agggaggtgg ctctcgggtg ctctccttca actggtatga agacaataac 1620
   tataaggcct ttctggggat tgactccaca aggaaggatc ctatctattc ttatgacaga 1680
   agaacaacat ccttttgtaa tgcattgatc cagagcctgg agtcaaatcc tttaaccaaa 1740
   atcgcttgga gggcggcaaa gcctttgctg atgggaaaaa tcctgtacac tcctgattca 1800
   cctgcagcac gaaggatact gaagaatgcc aactcaactt ttgaagaact ggaacacgtt 1860
   aggaagttgg tcaaagcctg ggaagaagta gggccccaga tctggtactt ctttgacaac 1920
   agcacacaga tgaacatgat cagagatacc ctggggaacc caacagtaaa agactttttg 1980
   aataggcagc ttggtgaaga aggtattact gctgaagcca tcctaaactt cctctacaag 2040
   ggccctcggg aaagccaggc tgacgacatg gccaacttcg actggaggga catatttaac 2100
   atcactgatc gcaccctccg cctggtcaat caatacctgg agtgcttggt cctggataag 2160
   tttgaaagct acaatgatga aactcagctc acccaacgtg ccctctctct actggaggaa 2220
   aacatgttct gggccggagt ggtattccct gacatgtatc cctggaccag ctctctacca 2280
   ccccacgtga agtataagat ccgaatggac atagacgtgg tggagaaaac caataagatt 2340
   aaagacaggt attgggattc tggtcccaga gctgatcccg tggaagattt ccggtacatc 2400
   tggggcgggt ttgcctatct gcaggacatg gttgaacagg ggatcacaag gagccaggtg 2460
   caggcggagg ctccagttgg aatctacctc cagcagatgc cctacccctg cttcgtggac 2520
   gattctttca tgatcatcct gaaccgctgt ttccctatct tcatggtgct ggcatggatc 2580
   tactctgtct ccatgactgt gaagagcatc gtcttggaga aggagttgcg actgaaggag 2640
   accttgaaaa atcagggtgt ctccaatgca gtgatttggt gtacctggtt cctggacagc 2700
   ttctccatca tgtcgatgag catcttcctc ctgacgatat tcatcatgca tggaagaatc 2760
   ctacattaca gcgacccatt catcctcttc ctgttcttgt tggctttctc cactgccacc 2820
   atcatgctgt gctttctgct cagcaccttc ttctccaagg ccagtctggc agcagcctgt 2880
   agtggtgtca tctatttcac cctctacctg ccacacatcc tgtgcttcgc ctggcaggac 2940
   cgcatgaccg ctgagctgaa gaaggctgtg agcttactgt ctccggtggc atttggattt 3000
   ggcactgagt acctggttcg ctttgaagag caaggcctgg ggctgcagtg gagcaacatc 3060
   gggaacagtc ccacggaagg ggacgaattc agcttcctgc tgtccatgca gatgatgctc 3120
   cttgatgctg cgtgctatgg cttactcgct tggtaccttg atcaggtgtt tccaggagac 3180
   tatggaaccc cacttccttg gtactttctt ctacaagagt cgtattggct tggcggtgaa 3240
   gggtgttcaa ccagagaaga aagagccctg gaaaagaccg agcccctaac agaggaaacg 3300
   gaggatccag agcacccaga aggaatacac gactccttct ttgaacgtga gcatccaggg 3360
   tgggttcctg gggtatgcgt gaagaatctg gtaaagattt ttgagccctg tggccggcca 3420
   gctgtggacc gtctgaacat caccttctac gagaaccaga tcaccgcatt cctgggccac 3480
   aatggagctg ggaaaaccac caccttgtcc atcctgacgg gtctgttgcc accaacctct 3540
   gggactgtgc tcgttggggg aagggacatt gaaaccagcc tggatgcagt ccggcagagc 3600
   cttggcatgt gtccacagca caacatcctg ttccaccacc tcacggtggc tgagcacatg 3660
   ctgttctatg cccagctgaa aggaaagtcc caggaggagg cccagctgga gatggaagcc 3720
   atgttggagg acacaggcct ccaccacaag cggaatgaag aggctcagga cctatcaggt 3780
   ggcatgcaga gaaagctgtc ggttgccatt gcctttgtgg gagatgccaa ggtggtgatt 3840
   ctggacgaac ccacctctgg ggtggaccct tactcgagac gctcaatctg ggatctgctc 3900
   ctgaagtatc gctcaggcag aaccatcatc atgtccactc accacatgga cgaggccgac 3960
   ctccttgggg accgcattgc catcattgcc cagggaaggc tctactgctc aggcacccca 4020
   ctcttcctga agaactgctt tggcacaggc ttgtacttaa ccttggtgcg caagatgaaa 4080
   aacatccaga gccaaaggaa aggcagtgag gggacctgca gctgctcgtc taagggtttc 4140
   tccaccacgt gtccagccca cgtcgatgac ctaactccag aacaagtcct ggatggggat 4200
   gtaaatgagc tgatggatgt agttctccac catgttccag aggcaaagct ggtggagtgc 4260
   attggtcaag aacttatctt ccttcttcca aataagaact tcaagcacag agcatatgcc 4320
   agccttttca gagagctgga ggagacgctg gctgaccttg gtctcagcag ttttggaatt 4380
   tctgacactc ccctggaaga gatttttctg aaggtcacgg aggattctga ttcaggacct 4440
   ctgtttgcgg gtggcgctca gcagaaaaga gaaaacgtca acccccgaca cccctgcttg 4500
   ggtcccagag agaaggctgg acagacaccc caggactcca atgtctgctc cccaggggcg 4560
   ccggctgctc acccagaggg ccagcctccc ccagagccag agtgcccagg cccgcagctc 4620
   aacacgggga cacagctggt cctccagcat gtgcaggcgc tgctggtcaa gagattccaa 4680
   cacaccatcc gcagccacaa ggacttcctg gcgcagatcg tgctcccggc tacctttgtg 4740
   tttttggctc tgatgctttc tattgttatc cttccttttg gcgaataccc cgctttgacc 4800
   cttcacccct ggatatatgg gcagcagtac accttcttca gcatggatga accaggcagt 4860
   gagcagttca cggtacttgc agacgtcctc ctgaataagc caggctttgg caaccgctgc 4920
   ctgaaggaag ggtggcttcc ggagtacccc tgtggcaact caacaccctg gaagactcct 4980
   tctgtgtccc caaacatcac ccagctgttc cagaagcaga aatggacaca ggtcaaccct 5040
   tcaccatcct gcaggtgcag caccagggag aagctcacca tgctgccaga gtgccccgag 5100
   ggtgccgggg gcctcccgcc cccccagaga acacagcgca gcacggaaat tctacaagac 5160
   ctgacggaca ggaacatctc cgacttcttg gtaaaaacgt atcctgctct tataagaagc 5220
   agcttaaaga gcaaattctg ggtcaatgaa cagaggtatg gaggaatttc cattggagga 5280
   aagctcccag tcgtccccat cacgggggaa gcacttgttg ggtttttaag cgaccttggc 5340
   cggatcatga atgtgagcgg gggccctatc actagagagg cctctaaaga aatacctgat 5400
   ttccttaaac atctagaaac tgaagacaac attaaggtgt ggtttaataa caaaggctgg 5460
   catgccctgg tcagctttct caatgtggcc cacaacgcca tcttacgggc cagcctgcct 5520
   aaggacagga gccccgagga gtatggaatc accgtcatta gccaacccct gaacctgacc 5580
   aaggagcagc tctcagagat tacagtgctg accacttcag tggatgctgt ggttgccatc 5640
   tgcgtgattt tctccatgtc cttcgtccca gccagctttg tcctttattt gatccaggag 5700
   cgggtgaaca aatccaagca cctccagttt atcagtggag tgagccccac cacctactgg 5760
   gtgaccaact tcctctggga catcatgaat tattccgtga gtgctgggct ggtggtgggc 5820
   atcttcatcg ggtttcagaa gaaagcctac acttctccag aaaaccttcc tgcccttgtg 5880
   gcactgctcc tgctgtatgg atgggcggtc attcccatga tgtacccagc atccttcctg 5940
   tttgatgtcc ccagcacagc ctatgtggct ttatcttgtg ctaatctgtt catcggcatc 6000
   aacagcagtg ctattacctt catcttggaa ttatttgata ataaccggac gctgctcagg 6060
   ttcaacgccg tgctgaggaa gctgctcatt gtcttccccc acttctgcct gggccggggc 6120
   ctcattgacc ttgcactgag ccaggctgtg acagatgtct atgcccggtt tggtgaggag 6180
   cactctgcaa atccgttcca ctgggacctg attgggaaga acctgtttgc catggtggtg 6240
   gaaggggtgg tgtacttcct cctgaccctg ctggtccagc gccacttctt cctctcccaa 6300
   tggattgccg agcccactaa ggagcccatt gttgatgaag atgatgatgt ggctgaagaa 6360
   agacaaagaa ttattactgg tggaaataaa actgacatct taaggctaca tgaactaacc 6420
   aagatttatc tgggcacctc cagcccagca gtggacaggc tgtgtgtcgg agttcgccct 6480
   ggagagtgct ttggcctcct gggagtgaat ggtgccggca aaacaaccac attcaagatg 6540
   ctcactgggg acaccacagt gacctcaggg gatgccaccg tagcaggcaa gagtatttta 6600
   accaatattt ctgaagtcca tcaaaatatg ggctactgtc ctcagtttga tgcaatcgat 6660
   gagctgctca caggacgaga acatctttac ctttatgccc ggcttcgagg tgtaccagca 6720
   gaagaaatcg aaaaggttgc aaactggagt attaagagcc tgggcctgac tgtctacgcc 6780
   gactgcctgg ctggcacgta cagtgggggc aacaagcgga aactctccac agccatcgca 6840
   ctcattggct gcccaccgct ggtgctgctg gatgagccca ccacagggat ggacccccag 6900
   gcacgccgca tgctgtggaa cgtcatcgtg agcatcatca gagaagggag ggctgtggtc 6960
   ctcacatccc acagcatgga agaatgtgag gcactgtgta cccggctggc catcatggta 7020
   aagggcgcct ttcgatgtat gggcaccatt cagcatctca agtccaaatt tggagatggc 7080
   tatatcgtca caatgaagat caaatccccg aaggacgacc tgcttcctga cctgaaccct 7140
   gtggagcagt tcttccaggg gaacttccca ggcagtgtgc agagggagag gcactacaac 7200
   atgctccagt tccaggtctc ctcctcctcc ctggcgagga tcttccagct cctcctctcc 7260
   cacaaggaca gcctgctcat cgaggagtac tcagtcacac agaccacact ggaccaggtg 7320
   tttgtaaatt ttgctaaaca gcagactgaa agtcatgacc tccctctgca ccctcgagct 7380
   gctggagcca gtcgacaagc ccaggactga tctttcacac cgctcgttcc tgcagccaga 7440
   aaggaactct gggcagctgg aggcgcagga gcctgtgccc atatggtcat ccaaatggac 7500
   tggcccagcg taaatgaccc cactgcagca gaaaacaaac acacgaggag catgcagcga 7560
   attcagaaag aggtctttca gaaggaaacc gaaactgact tgctacctgg aacacctgat 7620
   ggtgaaacca aacaaataca aaatccttct ccagacccca gaactagaaa ccccgggcca 7680
   tcccactagc agctttggcc tccatattgc tctcatttca agcagatctg cttttctgca 7740
   tgtttgtctg tgtgtctgcg ttgtgtgtga ttttcatgga aa 7782
<210> 2
   <211> 6819
   <212> DNA
   <213> Homo sapiens
<400> 2
   atgggcttcg tgagacagat acagcttttg ctctggaaga actggaccct gcggaaaagg 60
   caaaagattc gctttgtggt ggaactcgtg tggcctttat ctttatttct ggtcttgatc 120
   tggttaagga atgccaaccc gctctacagc catcatgaat gccatttccc caacaaggcg 180
   atgccctcag caggaatgct gccgtggctc caggggatct tctgcaatgt gaacaatccc 240
   tgttttcaaa gccccacccc aggagaatct cctggaattg tgtcaaacta taacaactcc 300
   atcttggcaa gggtatatcg agattttcaa gaactcctca tgaatgcacc agagagccag 360
   caccttggcc gtatttggac agagctacac atcttgtccc aattcatgga caccctccgg 420
   actcacccgg agagaattgc aggaagagga atacgaataa gggatatctt gaaagatgaa 480
   gaaacactga cactatttct cattaaaaac atcggcctgt ctgactcagt ggtctacctt 540
   ctgatcaact ctcaagtccg tccagagcag ttcgctcatg gagtcccgga cctggcgctg 600
   aaggacatcg cctgcagcga ggccctcctg gagcgcttca tcatcttcag ccagagacgc 660
   ggggcaaaga cggtgcgcta tgccctgtgc tccctctccc agggcaccct acagtggata 720
   gaagacactc tgtatgccaa cgtggacttc ttcaagctct tccgtgtgct tcccacactc 780
   ctagacagcc gttctcaagg tatcaatctg agatcttggg gaggaatatt atctgatatg 840
   tcaccaagaa ttcaagagtt tatccatcgg ccgagtatgc aggacttgct gtgggtgacc 900
   aggcccctca tgcagaatgg tggtccagag acctttacaa agctgatggg catcctgtct 960
   gacctcctgt gtggctaccc cgagggaggt ggctctcggg tgctctcctt caactggtat 1020
   gaagacaata actataaggc ctttctgggg attgactcca caaggaagga tcctatctat 1080
   tcttatgaca gaagaacaac atccttttgt aatgcattga tccagagcct ggagtcaaat 1140
   cctttaacca aaatcgcttg gagggcggca aagcctttgc tgatgggaaa aatcctgtac 1200
   actcctgatt cacctgcagc acgaaggata ctgaagaatg ccaactcaac ttttgaagaa 1260
   ctggaacacg ttaggaagtt ggtcaaagcc tgggaagaag tagggcccca gatctggtac 1320
   ttctttgaca acagcacaca gatgaacatg atcagagata ccctggggaa cccaacagta 1380
   aaagactttt tgaataggca gcttggtgaa gaaggtatta ctgctgaagc catcctaaac 1440
   ttcctctaca agggccctcg ggaaagccag gctgacgaca tggccaactt cgactggagg 1500
   gacatattta acatcactga tcgcaccctc cgcctggtca atcaatacct ggagtgcttg 1560
   gtcctggata agtttgaaag ctacaatgat gaaactcagc tcacccaacg tgccctctct 1620
   ctactggagg aaaacatgtt ctgggccgga gtggtattcc ctgacatgta tccctggacc 1680
   agctctctac caccccacgt gaagtataag atccgaatgg acatagacgt ggtggagaaa 1740
   accaataaga ttaaagacag gtattgggat tctggtccca gagctgatcc cgtggaagat 1800
   ttccggtaca tctggggcgg gtttgcctat ctgcaggaca tggttgaaca ggggatcaca 1860
   aggagccagg tgcaggcgga ggctccagtt ggaatctacc tccagcagat gccctacccc 1920
   tgcttcgtgg acgattcttt catgatcatc ctgaaccgct gtttccctat cttcatggtg 1980
   ctggcatgga tctactctgt ctccatgact gtgaagagca tcgtcttgga gaaggagttg 2040
   cgactgaagg agaccttgaa aaatcagggt gtctccaatg cagtgatttg gtgtacctgg 2100
   ttcctggaca gcttctccat catgtcgatg agcatcttcc tcctgacgat attcatcatg 2160
   catggaagaa tcctacatta cagcgaccca ttcatcctct tcctgttctt gttggctttc 2220
   tccactgcca ccatcatgct gtgctttctg ctcagcacct tcttctccaa ggccagtctg 2280
   gcagcagcct gtagtggtgt catctatttc accctctacc tgccacacat cctgtgcttc 2340
   gcctggcagg accgcatgac cgctgagctg aagaaggctg tgagcttact gtctccggtg 2400
   gcatttggat ttggcactga gtacctggtt cgctttgaag agcaaggcct ggggctgcag 2460
   tggagcaaca tcgggaacag tcccacggaa ggggacgaat tcagcttcct gctgtccatg 2520
   cagatgatgc tccttgatgc tgcgtgctat ggcttactcg cttggtacct tgatcaggtg 2580
   tttccaggag actatggaac cccacttcct tggtactttc ttctacaaga gtcgtattgg 2640
   cttggcggtg aagggtgttc aaccagagaa gaaagagccc tggaaaagac cgagccccta 2700
   acagaggaaa cggaggatcc agagcaccca gaaggaatac acgactcctt ctttgaacgt 2760
   gagcatccag ggtgggttcc tggggtatgc gtgaagaatc tggtaaagat ttttgagccc 2820
   tgtggccggc cagctgtgga ccgtctgaac atcaccttct acgagaacca gatcaccgca 2880
   ttcctgggcc acaatggagc tgggaaaacc accaccttgt ccatcctgac gggtctgttg 2940
   ccaccaacct ctgggactgt gctcgttggg ggaagggaca ttgaaaccag cctggatgca 3000
   gtccggcaga gccttggcat gtgtccacag cacaacatcc tgttccacca cctcacggtg 3060
   gctgagcaca tgctgttcta tgcccagctg aaaggaaagt cccaggagga ggcccagctg 3120
   gagatggaag ccatgttgga ggacacaggc ctccaccaca agcggaatga agaggctcag 3180
   gacctatcag gtggcatgca gagaaagctg tcggttgcca ttgcctttgt gggagatgcc 3240
   aaggtggtga ttctggacga acccacctct ggggtggacc cttactcgag acgctcaatc 3300
   tgggatctgc tcctgaagta tcgctcaggc agaaccatca tcatgtccac tcaccacatg 3360
   gacgaggccg acctccttgg ggaccgcatt gccatcattg cccagggaag gctctactgc 3420
   tcaggcaccc cactcttcct gaagaactgc tttggcacag gcttgtactt aaccttggtg 3480
   cgcaagatga aaaacatcca gagccaaagg aaaggcagtg aggggacctg cagctgctcg 3540
   tctaagggtt tctccaccac gtgtccagcc cacgtcgatg acctaactcc agaacaagtc 3600
   ctggatgggg atgtaaatga gctgatggat gtagttctcc accatgttcc agaggcaaag 3660
   ctggtggagt gcattggtca agaacttatc ttccttcttc caaataagaa cttcaagcac 3720
   agagcatatg ccagcctttt cagagagctg gaggagacgc tggctgacct tggtctcagc 3780
   agttttggaa tttctgacac tcccctggaa gagatttttc tgaaggtcac ggaggattct 3840
   gattcaggac ctctgtttgc gggtggcgct cagcagaaaa gagaaaacgt caacccccga 3900
   cacccctgct tgggtcccag agagaaggct ggacagacac cccaggactc caatgtctgc 3960
   tccccagggg cgccggctgc tcacccagag ggccagcctc ccccagagcc agagtgccca 4020
   ggcccgcagc tcaacacggg gacacagctg gtcctccagc atgtgcaggc gctgctggtc 4080
   aagagattcc aacacaccat ccgcagccac aaggacttcc tggcgcagat cgtgctcccg 4140
   gctacctttg tgtttttggc tctgatgctt tctattgtta tccttccttt tggcgaatac 4200
   cccgctttga cccttcaccc ctggatatat gggcagcagt acaccttctt cagcatggat 4260
   gaaccaggca gtgagcagtt cacggtactt gcagacgtcc tcctgaataa gccaggcttt 4320
   ggcaaccgct gcctgaagga agggtggctt ccggagtacc cctgtggcaa ctcaacaccc 4380
   tggaagactc cttctgtgtc cccaaacatc acccagctgt tccagaagca gaaatggaca 4440
   caggtcaacc cttcaccatc ctgcaggtgc agcaccaggg agaagctcac catgctgcca 4500
   gagtgccccg agggtgccgg gggcctcccg cccccccaga gaacacagcg cagcacggaa 4560
   attctacaag acctgacgga caggaacatc tccgacttct tggtaaaaac gtatcctgct 4620
   cttataagaa gcagcttaaa gagcaaattc tgggtcaatg aacagaggta tggaggaatt 4680
   tccattggag gaaagctccc agtcgtcccc atcacggggg aagcacttgt tgggttttta 4740
   agcgaccttg gccggatcat gaatgtgagc gggggcccta tcactagaga ggcctctaaa 4800
   gaaatacctg atttccttaa acatctagaa actgaagaca acattaaggt gtggtttaat 4860
   aacaaaggct ggcatgccct ggtcagcttt ctcaatgtgg cccacaacgc catcttacgg 4920
   gccagcctgc ctaaggacag gagccccgag gagtatggaa tcaccgtcat tagccaaccc 4980
   ctgaacctga ccaaggagca gctctcagag attacagtgc tgaccacttc agtggatgct 5040
   gtggttgcca tctgcgtgat tttctccatg tccttcgtcc cagccagctt tgtcctttat 5100
   ttgatccagg agcgggtgaa caaatccaag cacctccagt ttatcagtgg agtgagcccc 5160
   accacctact gggtgaccaa cttcctctgg gacatcatga attattccgt gagtgctggg 5220
   ctggtggtgg gcatcttcat cgggtttcag aagaaagcct acacttctcc agaaaacctt 5280
   cctgcccttg tggcactgct cctgctgtat ggatgggcgg tcattcccat gatgtaccca 5340
   gcatccttcc tgtttgatgt ccccagcaca gcctatgtgg ctttatcttg tgctaatctg 5400
   ttcatcggca tcaacagcag tgctattacc ttcatcttgg aattatttga taataaccgg 5460
   acgctgctca ggttcaacgc cgtgctgagg aagctgctca ttgtcttccc ccacttctgc 5520
   ctgggccggg gcctcattga ccttgcactg agccaggctg tgacagatgt ctatgcccgg 5580
   tttggtgagg agcactctgc aaatccgttc cactgggacc tgattgggaa gaacctgttt 5640
   gccatggtgg tggaaggggt ggtgtacttc ctcctgaccc tgctggtcca gcgccacttc 5700
   ttcctctccc aatggattgc cgagcccact aaggagccca ttgttgatga agatgatgat 5760
   gtggctgaag aaagacaaag aattattact ggtggaaata aaactgacat cttaaggcta 5820
   catgaactaa ccaagattta tctgggcacc tccagcccag cagtggacag gctgtgtgtc 5880
   ggagttcgcc ctggagagtg ctttggcctc ctgggagtga atggtgccgg caaaacaacc 5940
   acattcaaga tgctcactgg ggacaccaca gtgacctcag gggatgccac cgtagcaggc 6000
   aagagtattt taaccaatat ttctgaagtc catcaaaata tgggctactg tcctcagttt 6060
   gatgcaatcg atgagctgct cacaggacga gaacatcttt acctttatgc ccggcttcga 6120
   ggtgtaccag cagaagaaat cgaaaaggtt gcaaactgga gtattaagag cctgggcctg 6180
   actgtctacg ccgactgcct ggctggcacg tacagtgggg gcaacaagcg gaaactctcc     6240
   acagccatcg cactcattgg ctgcccaccg ctggtgctgc tggatgagcc caccacaggg     6300
   atggaccccc aggcacgccg catgctgtgg aacgtcatcg tgagcatcat cagagaaggg     6360
   agggctgtgg tcctcacatc ccacagcatg gaagaatgtg aggcactgtg tacccggctg     6420
   gccatcatgg taaagggcgc ctttcgatgt atgggcacca ttcagcatct caagtccaaa     6480
   tttggagatg gctatatcgt cacaatgaag atcaaatccc cgaaggacga cctgcttcct     6540
   gacctgaacc ctgtggagca gttcttccag gggaacttcc caggcagtgt gcagagggag     6600
   aggcactaca acatgctcca gttccaggtc tcctcctcct ccctggcgag gatcttccag     6660
   ctcctcctct cccacaagga cagcctgctc atcgaggagt actcagtcac acagaccaca     6720
   ctggaccagg tgtttgtaaa ttttgctaaa cagcagactg aaagtcatga cctccctctg     6780
   caccctcgag ctgctggagc cagtcgacaa gcccaggac                            6819
<210> 3
   <211> 2273
   <212> PRT
   <213> Homo sapiens
<400> 3
   Met Gly Phe Val Arg Gln Ile Gln Leu Leu Leu Trp Lys Asn Trp Thr
   1               5                   10                  15
   Leu Arg Lys Arg Gln Lys Ile Arg Phe Val Val Glu Leu Val Trp Pro
               20                  25                  30
   Leu Ser Leu Phe Leu Val Leu Ile Trp Leu Arg Asn Ala Asn Pro Leu
           35                  40                  45
   Tyr Ser His His Glu Cys His Phe Pro Asn Lys Ala Met Pro Ser Ala
       50                  55                  60
   Gly Met Leu Pro Trp Leu Gln Gly Ile Phe Cys Asn Val Asn Asn Pro
   65                  70                  75                  80
   Cys Phe Gln Ser Pro Thr Pro Gly Glu Ser Pro Gly Ile Val Ser Asn
                   85                  90                  95
   Tyr Asn Asn Ser Ile Leu Ala Arg Val Tyr Arg Asp Phe Gln Glu Leu
               100                 105                 110
   Leu Met Asn Ala Pro Glu Ser Gln His Leu Gly Arg Ile Trp Thr Glu
   115 120 125
   Leu His Ile Leu Ser Gln Phe Met Asp Thr Leu Arg Thr His Pro Glu
   130 135 140
   Arg Ile Ala Gly Arg Gly Ile Arg Ile Arg Asp Ile Leu Lys Asp Glu
   145 150 155 160
   Glu Thr Leu Thr Leu Phe Leu Ile Lys Asn Ile Gly Leu Ser Asp Ser
   165 170 175
   Val Val Tyr Leu Leu Ile Asn Ser Gln Val Arg Pro Glu Gln Phe Ala
   180 185 190
   His Gly Val Pro Asp Leu Ala Leu Lys Asp Ile Ala Cys Ser Glu Ala
   195 200 205
   Leu Leu Glu Arg Phe Ile Ile Phe Ser Gln Arg Arg Gly Ala Lys Thr
   210 215 220
   Val Arg Tyr Ala Leu Cys Ser Leu Ser Gln Gly Thr Leu Gln Trp Ile
   225 230 235 240
   Glu Asp Thr Leu Tyr Ala Asn Val Asp Phe Phe Lys Leu Phe Arg Val
   245 250 255
   Leu Pro Thr Leu Leu Asp Ser Arg Ser Gln Gly Ile Asn Leu Arg Ser
   260 265 270
   Trp Gly Gly Ile Leu Ser Asp Met Ser Pro Arg Ile Gln Glu Phe Ile
   275 280 285
   His Arg Pro Ser Met Gln Asp Leu Leu Trp Val Thr Arg Pro Leu Met
   290 295 300
   Gln Asn Gly Gly Pro Glu Thr Phe Thr Lys Leu Met Gly Ile Leu Ser
   305 310 315 320
   Asp Leu Leu Cys Gly Tyr Pro Glu Gly Gly Gly Ser Arg Val Leu Ser
   325 330 335
   Phe Asn Trp Tyr Glu Asp Asn Asn Tyr Lys Ala Phe Leu Gly Ile Asp
   340 345 350
   Ser Thr Arg Lys Asp Pro Ile Tyr Ser Tyr Asp Arg Arg Thr Thr Ser
   355 360 365
   Phe Cys Asn Ala Leu Ile Gln Ser Leu Glu Ser Asn Pro Leu Thr Lys
   370 375 380
   Ile Ala Trp Arg Ala Ala Lys Pro Leu Leu Met Gly Lys Ile Leu Tyr
   385 390 395 400
   Thr Pro Asp Ser Pro Ala Ala Arg Arg Ile Leu Lys Asn Ala Asn Ser
   405 410 415
   Thr Phe Glu Glu Leu Glu His Val Arg Lys Leu Val Lys Ala Trp Glu
   420 425 430
   Glu Val Gly Pro Gln Ile Trp Tyr Phe Phe Asp Asn Ser Thr Gln Met
   435 440 445
   Asn Met Ile Arg Asp Thr Leu Gly Asn Pro Thr Val Lys Asp Phe Leu
   450 455 460
   Asn Arg Gln Leu Gly Glu Glu Gly Ile Thr Ala Glu Ala Ile Leu Asn
   465 470 475 480
   Phe Leu Tyr Lys Gly Pro Arg Glu Ser Gln Ala Asp Asp Met Ala Asn
   485 490 495
   Phe Asp Trp Arg Asp Ile Phe Asn Ile Thr Asp Arg Thr Leu Arg Leu
   500 505 510
   Val Asn Gln Tyr Leu Glu Cys Leu Val Leu Asp Lys Phe Glu Ser Tyr
   515 520 525
   Asn Asp Glu Thr Gln Leu Thr Gln Arg Ala Leu Ser Leu Leu Glu Glu
   530 535 540
   Asn Met Phe Trp Ala Gly Val Val Phe Pro Asp Met Tyr Pro Trp Thr
   545 550 555 560
   Ser Ser Leu Pro Pro His Val Lys Tyr Lys Ile Arg Met Asp Ile Asp
   565 570 575
   Val Val Glu Lys Thr Asn Lys Ile Lys Asp Arg Tyr Trp Asp Ser Gly
   580 585 590
   Pro Arg Ala Asp Pro Val Glu Asp Phe Arg Tyr Ile Trp Gly Gly Phe
   595 600 605
   Ala Tyr Leu Gln Asp Met Val Glu Gln Gly Ile Thr Arg Ser Gln Val
   610 615 620
   Gln Ala Glu Ala Pro Val Gly Ile Tyr Leu Gln Gln Met Pro Tyr Pro
   625 630 635 640
   Cys Phe Val Asp Asp Ser Phe Met Ile Ile Leu Asn Arg Cys Phe Pro
   645 650 655
   Ile Phe Met Val Leu Ala Trp Ile Tyr Ser Val Ser Met Thr Val Lys
   660 665 670
   Ser Ile Val Leu Glu Lys Glu Leu Arg Leu Lys Glu Thr Leu Lys Asn
   675 680 685
   Gln Gly Val Ser Asn Ala Val Ile Trp Cys Thr Trp Phe Leu Asp Ser
   690 695 700
   Phe Ser Ile Met Ser Met Ser Ile Phe Leu Leu Thr Ile Phe Ile Met
   705 710 715 720
   His Gly Arg Ile Leu His Tyr Ser Asp Pro Phe Ile Leu Phe Leu Phe
   725 730 735
   Leu Leu Ala Phe Ser Thr Ala Thr Ile Met Leu Cys Phe Leu Leu Ser
   740 745 750
   Thr Phe Phe Ser Lys Ala Ser Leu Ala Ala Ala Cys Ser Gly Val Ile
   755 760 765
   Tyr Phe Thr Leu Tyr Leu Pro His Ile Leu Cys Phe Ala Trp Gln Asp
   770 775 780
   Arg Met Thr Ala Glu Leu Lys Lys Ala Val Ser Leu Leu Ser Pro Val
   785 790 795 800
   Ala Phe Gly Phe Gly Thr Glu Tyr Leu Val Arg Phe Glu Glu Gln Gly
                   805                 810                 815
   Leu Gly Leu Gln Trp Ser Asn Ile Gly Asn Ser Pro Thr Glu Gly Asp
               820                 825                 830
   Glu Phe Ser Phe Leu Leu Ser Met Gln Met Met Leu Leu Asp Ala Ala
           835                 840                 845
   Cys Tyr Gly Leu Leu Ala Trp Tyr Leu Asp Gln Val Phe Pro Gly Asp
       850                 855                 860
   Tyr Gly Thr Pro Leu Pro Trp Tyr Phe Leu Leu Gln Glu Ser Tyr Trp
   865                 870                 875                 880
   Leu Gly Gly Glu Gly Cys Ser Thr Arg Glu Glu Arg Ala Leu Glu Lys
                   885                 890                 895
   Thr Glu Pro Leu Thr Glu Glu Thr Glu Asp Pro Glu His Pro Glu Gly
               900                 905                 910
   Ile His Asp Ser Phe Phe Glu Arg Glu His Pro Gly Trp Val Pro Gly
           915                 920                 925
   Val Cys Val Lys Asn Leu Val Lys Ile Phe Glu Pro Cys Gly Arg Pro
       930                 935                 940
   Ala Val Asp Arg Leu Asn Ile Thr Phe Tyr Glu Asn Gln Ile Thr Ala
   945                 950                 955                 960
   Phe Leu Gly His Asn Gly Ala Gly Lys Thr Thr Thr Leu Ser Ile Leu
                   965                 970                 975
   Thr Gly Leu Leu Pro Pro Thr Ser Gly Thr Val Leu Val Gly Gly Arg
               980                 985                 990
   Asp Ile Glu Thr Ser Leu Asp Ala  Val Arg Gln Ser Leu  Gly Met Cys
           995                 1000                1005
   Pro Gln  His Asn Ile Leu Phe  His His Leu Thr Val  Ala Glu His
       1010                1015                1020
   Met Leu Phe Tyr Ala Gln Leu Lys Gly Lys Ser Gln Glu Glu Ala
       1025                1030                1035
   Gln Leu Glu Met Glu Ala Met Leu Glu Asp Thr Gly Leu His His
       1040                1045                1050
   Lys Arg Asn Glu Glu Ala Gln Asp Leu Ser Gly Gly Met Gln Arg
       1055                1060                1065
   Lys Leu Ser Val Ala Ile Ala Phe Val Gly Asp Ala Lys Val Val
       1070                1075                1080
   Ile Leu Asp Glu Pro Thr Ser Gly Val Asp Pro Tyr Ser Arg Arg
       1085                1090                1095
   Ser Ile Trp Asp Leu Leu Leu Lys Tyr Arg Ser Gly Arg Thr Ile
       1100                1105                1110
   Ile Met Ser Thr His His Met Asp Glu Ala Asp Leu Leu Gly Asp
       1115                1120                1125
   Arg Ile Ala Ile Ile Ala Gln Gly Arg Leu Tyr Cys Ser Gly Thr
       1130                1135                1140
   Pro Leu Phe Leu Lys Asn Cys Phe Gly Thr Gly Leu Tyr Leu Thr
       1145                1150                1155
   Leu Val Arg Lys Met Lys Asn Ile Gln Ser Gln Arg Lys Gly Ser
       1160                1165                1170
   Glu Gly Thr Cys Ser Cys Ser Ser Lys Gly Phe Ser Thr Thr Cys
       1175                1180                1185
   Pro Ala His Val Asp Asp Leu Thr Pro Glu Gln Val Leu Asp Gly
       1190                1195                1200
   Asp Val Asn Glu Leu Met Asp Val Val Leu His His Val Pro Glu
       1205                1210                1215
   Ala Lys Leu Val Glu Cys Ile Gly Gln Glu Leu Ile Phe Leu Leu
       1220                1225                1230
   Pro Asn Lys Asn Phe Lys His Arg Ala Tyr Ala Ser Leu Phe Arg
   1235 1240 1245
   Glu Leu Glu Glu Thr Leu Ala Asp Leu Gly Leu Ser Ser Phe Gly
   1250 1255 1260
   Ile Ser Asp Thr Pro Leu Glu Glu Ile Phe Leu Lys Val Thr Glu
   1265 1270 1275
   Asp Ser Asp Ser Gly Pro Leu Phe Ala Gly Gly Ala Gln Gln Lys
   1280 1285 1290
   Arg Glu Asn Val Asn Pro Arg His Pro Cys Leu Gly Pro Arg Glu
   1295 1300 1305
   Lys Ala Gly Gln Thr Pro Gln Asp Ser Asn Val Cys Ser Pro Gly
   1310 1315 1320
   Ala Pro Ala Ala His Pro Glu Gly Gln Pro Pro Pro Glu Pro Glu
   1325 1330 1335
   Cys Pro Gly Pro Gln Leu Asn Thr Gly Thr Gln Leu Val Leu Gln
   1340 1345 1350
   His Val Gln Ala Leu Leu Val Lys Arg Phe Gln His Thr Ile Arg
   1355 1360 1365
   Ser His Lys Asp Phe Leu Ala Gln Ile Val Leu Pro Ala Thr Phe
   1370 1375 1380
   Val Phe Leu Ala Leu Met Leu Ser Ile Val Ile Leu Pro Phe Gly
   1385 1390 1395
   Glu Tyr Pro Ala Leu Thr Leu His Pro Trp Ile Tyr Gly Gln Gln
   1400 1405 1410
   Tyr Thr Phe Phe Ser Met Asp Glu Pro Gly Ser Glu Gln Phe Thr
   1415 1420 1425
   Val Leu Ala Asp Val Leu Leu Asn Lys Pro Gly Phe Gly Asn Arg
   1430 1435 1440
   Cys Leu Lys Glu Gly Trp Leu Pro Glu Tyr Pro Cys Gly Asn Ser
   1445 1450 1455
   Thr Pro Trp Lys Thr Pro Ser Val Ser Pro Asn Ile Thr Gln Leu
   1460 1465 1470
   Phe Gln Lys Gln Lys Trp Thr Gln Val Asn Pro Ser Pro Ser Cys
   1475 1480 1485
   Arg Cys Ser Thr Arg Glu Lys Leu Thr Met Leu Pro Glu Cys Pro
   1490 1495 1500
   Glu Gly Ala Gly Gly Leu Pro Pro Pro Gln Arg Thr Gln Arg Ser
   1505 1510 1515
   Thr Glu Ile Leu Gln Asp Leu Thr Asp Arg Asn Ile Ser Asp Phe
   1520 1525 1530
   Leu Val Lys Thr Tyr Pro Ala Leu Ile Arg Ser Ser Leu Lys Ser
   1535 1540 1545
   Lys Phe Trp Val Asn Glu Gln Arg Tyr Gly Gly Ile Ser Ile Gly
   1550 1555 1560
   Gly Lys Leu Pro Val Val Pro Ile Thr Gly Glu Ala Leu Val Gly
   1565 1570 1575
   Phe Leu Ser Asp Leu Gly Arg Ile Met Asn Val Ser Gly Gly Pro
   1580 1585 1590
   Ile Thr Arg Glu Ala Ser Lys Glu Ile Pro Asp Phe Leu Lys His
   1595 1600 1605
   Leu Glu Thr Glu Asp Asn Ile Lys Val Trp Phe Asn Asn Lys Gly
   1610 1615 1620
   Trp His Ala Leu Val Ser Phe Leu Asn Val Ala His Asn Ala Ile
   1625 1630 1635
   Leu Arg Ala Ser Leu Pro Lys Asp Arg Ser Pro Glu Glu Tyr Gly
   1640 1645 1650
   Ile Thr Val Ile Ser Gln Pro Leu Asn Leu Thr Lys Glu Gln Leu
   1655 1660 1665
   Ser Glu Ile Thr Val Leu Thr Thr Ser Val Asp Ala Val Val Ala
   1670 1675 1680
   Ile Cys Val Ile Phe Ser Met Ser Phe Val Pro Ala Ser Phe Val
   1685 1690 1695
   Leu Tyr Leu Ile Gln Glu Arg Val Asn Lys Ser Lys His Leu Gln
   1700 1705 1710
   Phe Ile Ser Gly Val Ser Pro Thr Thr Tyr Trp Val Thr Asn Phe
   1715 1720 1725
   Leu Trp Asp Ile Met Asn Tyr Ser Val Ser Ala Gly Leu Val Val
   1730 1735 1740
   Gly Ile Phe Ile Gly Phe Gln Lys Lys Ala Tyr Thr Ser Pro Glu
   1745 1750 1755
   Asn Leu Pro Ala Leu Val Ala Leu Leu Leu Leu Tyr Gly Trp Ala
   1760 1765 1770
   Val Ile Pro Met Met Tyr Pro Ala Ser Phe Leu Phe Asp Val Pro
   1775 1780 1785
   Ser Thr Ala Tyr Val Ala Leu Ser Cys Ala Asn Leu Phe Ile Gly
   1790 1795 1800
   Ile Asn Ser Ser Ala Ile Thr Phe Ile Leu Glu Leu Phe Asp Asn
   1805 1810 1815
   Asn Arg Thr Leu Leu Arg Phe Asn Ala Val Leu Arg Lys Leu Leu
   1820 1825 1830
   Ile Val Phe Pro His Phe Cys Leu Gly Arg Gly Leu Ile Asp Leu
   1835 1840 1845
   Ala Leu Ser Gln Ala Val Thr Asp Val Tyr Ala Arg Phe Gly Glu
   1850 1855 1860
   Glu His Ser Ala Asn Pro Phe His Trp Asp Leu Ile Gly Lys Asn
   1865 1870 1875
   Leu Phe Ala Met Val Val Glu Gly Val Val Tyr Phe Leu Leu Thr
   1880 1885 1890
   Leu Leu Val Gln Arg His Phe Phe Leu Ser Gln Trp Ile Ala Glu
   1895 1900 1905
   Pro Thr Lys Glu Pro Ile Val Asp Glu Asp Asp Asp Val Ala Glu
   1910 1915 1920
   Glu Arg Gln Arg Ile Ile Thr Gly Gly Asn Lys Thr Asp Ile Leu
   1925 1930 1935
   Arg Leu His Glu Leu Thr Lys Ile Tyr Leu Gly Thr Ser Ser Pro
   1940 1945 1950
   Ala Val Asp Arg Leu Cys Val Gly Val Arg Pro Gly Glu Cys Phe
   1955 1960 1965
   Gly Leu Leu Gly Val Asn Gly Ala Gly Lys Thr Thr Thr Phe Lys
   1970 1975 1980
   Met Leu Thr Gly Asp Thr Thr Val Thr Ser Gly Asp Ala Thr Val
   1985 1990 1995
   Ala Gly Lys Ser Ile Leu Thr Asn Ile Ser Glu Val His Gln Asn
   2000 2005 2010
   Met Gly Tyr Cys Pro Gln Phe Asp Ala Ile Asp Glu Leu Leu Thr
   2015 2020 2025
   Gly Arg Glu His Leu Tyr Leu Tyr Ala Arg Leu Arg Gly Val Pro
   2030 2035 2040
   Ala Glu Glu Ile Glu Lys Val Ala Asn Trp Ser Ile Lys Ser Leu
   2045 2050 2055
   Gly Leu Thr Val Tyr Ala Asp Cys Leu Ala Gly Thr Tyr Ser Gly
   2060 2065 2070
   Gly Asn Lys Arg Lys Leu Ser Thr Ala Ile Ala Leu Ile Gly Cys
   2075 2080 2085
   Pro Pro Leu Val Leu Leu Asp Glu Pro Thr Thr Gly Met Asp Pro
   2090 2095 2100
   Gln Ala Arg Arg Met Leu Trp Asn Val Ile Val Ser Ile Ile Arg
   2105 2110 2115
   Glu Gly Arg Ala Val Val Leu Thr Ser His Ser Met Glu Glu Cys
   2120 2125 2130
   Glu Ala Leu Cys Thr Arg Leu Ala Ile Met Val Lys Gly Ala Phe
   2135 2140 2145
   Arg Cys Met Gly Thr Ile Gln His Leu Lys Ser Lys Phe Gly Asp
   2150 2155 2160
   Gly Tyr Ile Val Thr Met Lys Ile Lys Ser Pro Lys Asp Asp Leu
   2165 2170 2175
   Leu Pro Asp Leu Asn Pro Val Glu Gln Phe Phe Gln Gly Asn Phe
   2180 2185 2190
   Pro Gly Ser Val Gln Arg Glu Arg His Tyr Asn Met Leu Gln Phe
   2195 2200 2205
   Gln Val Ser Ser Ser Ser Leu Ala Arg Ile Phe Gln Leu Leu Leu
   2210 2215 2220
   Ser His Lys Asp Ser Leu Leu Ile Glu Glu Tyr Ser Val Thr Gln
   2225 2230 2235
   Thr Thr Leu Asp Gln Val Phe Val Asn Phe Ala Lys Gln Gln Thr
   2240 2245 2250
   Glu Ser His Asp Leu Pro Leu His Pro Arg Ala Ala Gly Ala Ser
   2255 2260 2265
   Arg Gln Ala Gln Asp
   2270
<210> 4
   <211> 114
   <212> DNA
   <213> Homo sapiens
<400> 4
   ggagtacccc tgtggcaact caacaccctg gaagactcct tctgtgtccc caaacatcac 60
   ccagctgttc cagaagcaga aatggacaca ggtcaaccct tcaccatcct gcag 114
<210> 5
   <211> 6705
   <212> DNA
   <213> Homo sapiens
<400> 5
   atgggcttcg tgagacagat acagcttttg ctctggaaga actggaccct gcggaaaagg 60
   caaaagattc gctttgtggt ggaactcgtg tggcctttat ctttatttct ggtcttgatc 120
   tggttaagga atgccaaccc gctctacagc catcatgaat gccatttccc caacaaggcg 180
   atgccctcag caggaatgct gccgtggctc caggggatct tctgcaatgt gaacaatccc 240
   tgttttcaaa gccccacccc aggagaatct cctggaattg tgtcaaacta taacaactcc 300
   atcttggcaa gggtatatcg agattttcaa gaactcctca tgaatgcacc agagagccag 360
   caccttggcc gtatttggac agagctacac atcttgtccc aattcatgga caccctccgg 420
   actcacccgg agagaattgc aggaagagga atacgaataa gggatatctt gaaagatgaa 480
   gaaacactga cactatttct cattaaaaac atcggcctgt ctgactcagt ggtctacctt 540
   ctgatcaact ctcaagtccg tccagagcag ttcgctcatg gagtcccgga cctggcgctg 600
   aaggacatcg cctgcagcga ggccctcctg gagcgcttca tcatcttcag ccagagacgc 660
   ggggcaaaga cggtgcgcta tgccctgtgc tccctctccc agggcaccct acagtggata 720
   gaagacactc tgtatgccaa cgtggacttc ttcaagctct tccgtgtgct tcccacactc 780
   ctagacagcc gttctcaagg tatcaatctg agatcttggg gaggaatatt atctgatatg 840
   tcaccaagaa ttcaagagtt tatccatcgg ccgagtatgc aggacttgct gtgggtgacc 900
   aggcccctca tgcagaatgg tggtccagag acctttacaa agctgatggg catcctgtct 960
   gacctcctgt gtggctaccc cgagggaggt ggctctcggg tgctctcctt caactggtat 1020
   gaagacaata actataaggc ctttctgggg attgactcca caaggaagga tcctatctat 1080
   tcttatgaca gaagaacaac atccttttgt aatgcattga tccagagcct ggagtcaaat 1140
   cctttaacca aaatcgcttg gagggcggca aagcctttgc tgatgggaaa aatcctgtac 1200
   actcctgatt cacctgcagc acgaaggata ctgaagaatg ccaactcaac ttttgaagaa 1260
   ctggaacacg ttaggaagtt ggtcaaagcc tgggaagaag tagggcccca gatctggtac 1320
   ttctttgaca acagcacaca gatgaacatg atcagagata ccctggggaa cccaacagta 1380
   aaagactttt tgaataggca gcttggtgaa gaaggtatta ctgctgaagc catcctaaac 1440
   ttcctctaca agggccctcg ggaaagccag gctgacgaca tggccaactt cgactggagg 1500
   gacatattta acatcactga tcgcaccctc cgcctggtca atcaatacct ggagtgcttg 1560
   gtcctggata agtttgaaag ctacaatgat gaaactcagc tcacccaacg tgccctctct 1620
   ctactggagg aaaacatgtt ctgggccgga gtggtattcc ctgacatgta tccctggacc 1680
   agctctctac caccccacgt gaagtataag atccgaatgg acatagacgt ggtggagaaa 1740
   accaataaga ttaaagacag gtattgggat tctggtccca gagctgatcc cgtggaagat 1800
   ttccggtaca tctggggcgg gtttgcctat ctgcaggaca tggttgaaca ggggatcaca 1860
   aggagccagg tgcaggcgga ggctccagtt ggaatctacc tccagcagat gccctacccc 1920
   tgcttcgtgg acgattcttt catgatcatc ctgaaccgct gtttccctat cttcatggtg 1980
   ctggcatgga tctactctgt ctccatgact gtgaagagca tcgtcttgga gaaggagttg 2040
   cgactgaagg agaccttgaa aaatcagggt gtctccaatg cagtgatttg gtgtacctgg 2100
   ttcctggaca gcttctccat catgtcgatg agcatcttcc tcctgacgat attcatcatg 2160
   catggaagaa tcctacatta cagcgaccca ttcatcctct tcctgttctt gttggctttc 2220
   tccactgcca ccatcatgct gtgctttctg ctcagcacct tcttctccaa ggccagtctg 2280
   gcagcagcct gtagtggtgt catctatttc accctctacc tgccacacat cctgtgcttc 2340
   gcctggcagg accgcatgac gcgtgagctg aagaaggctg tgagcttact gtctccggtg 2400
   gcatttggat ttggcactga gtacctggtt cgctttgaag agcaaggcct ggggctgcag 2460
   tggagcaaca tcgggaacag tcccacggaa ggggacgaat tcagcttcct gctgtccatg 2520
   cagatgatgc tccttgatgc tgcgtgctat ggcttactcg cttggtacct tgatcaggtg 2580
   tttccaggag actatggaac cccacttcct tggtactttc ttctacaaga gtcgtattgg 2640
   cttggcggtg aagggtgttc aaccagagaa gaaagagccc tggaaaagac cgagccccta 2700
   acagaggaaa cggaggatcc agagcaccca gaaggaatac acgactcctt ctttgaacgt 2760
   gagcatccag ggtgggttcc tggggtatgc gtgaagaatc tggtaaagat ttttgagccc 2820
   tgtggccggc cagctgtgga ccgtctgaac atcaccttct acgagaacca gatcaccgca 2880
   ttcctgggcc acaatggagc tgggaaaacc accaccttgt ccatcctgac gggtctgttg 2940
   ccaccaacct ctgggactgt gctcgttggg ggaagggaca ttgaaaccag cctggatgca 3000
   gtccggcaga gccttggcat gtgtccacag cacaacatcc tgttccacca cctcacggtg 3060
   gctgagcaca tgctgttcta tgcccagctg aaaggaaagt cccaggagga ggcccagctg 3120
   gagatggaag ccatgttgga ggacacaggc ctccaccaca agcggaatga agaggctcag 3180
   gacctatcag gtggcatgca gagaaagctg tcggttgcca ttgcctttgt gggagatgcc 3240
   aaggtggtga ttctggacga acccacctct ggggtggacc cttactcgag acgctcaatc 3300
   tgggatctgc tcctgaagta tcgctcaggc agaaccatca tcatgtccac tcaccacatg 3360
   gacgaggccg acctccttgg ggaccgcatt gccatcattg cccagggaag gctctactgc 3420
   tcaggcaccc cactcttcct gaagaactgc tttggcacag gcttgtactt aaccttggtg 3480
   cgcaagatga aaaacatcca gagccaaagg aaaggcagtg aggggacctg cagctgctcg 3540
   tctaagggtt tctccaccac gtgtccagcc cacgtcgatg acctaactcc agaacaagtc 3600
   ctggatgggg atgtaaatga gctgatggat gtagttctcc accatgttcc agaggcaaag 3660
   ctggtggagt gcattggtca agaacttatc ttccttcttc caaataagaa cttcaagcac 3720
   agagcatatg ccagcctttt cagagagctg gaggagacgc tggctgacct tggtctcagc 3780
   agttttggaa tttctgacac tcccctggaa gagatttttc tgaaggtcac ggaggattct 3840
   gattcaggac ctctgtttgc gggtggcgct cagcagaaaa gagaaaacgt caacccccga 3900
   cacccctgct tgggtcccag agagaaggct ggacagacac cccaggactc caatgtctgc 3960
   tccccagggg cgccggctgc tcacccagag ggccagcctc ccccagagcc agagtgccca 4020
   ggcccgcagc tcaacacggg gacacagctg gtcctccagc atgtgcaggc gctgctggtc 4080
   aagagattcc aacacaccat ccgcagccac aaggacttcc tggcgcagat cgtgctcccg 4140
   gctacctttg tgtttttggc tctgatgctt tctattgtta tccttccttt tggcgaatac 4200
   cccgctttga cccttcaccc ctggatatat gggcagcagt acaccttctt cagcatggat 4260
   gaaccaggca gtgagcagtt cacggtactt gcagacgtcc tcctgaataa gccaggcttt 4320
   ggcaaccgct gcctgaagga agggtggctt ccgtgcagca ccagggagaa gctcaccatg 4380
   ctgccagagt gccccgaggg tgccgggggc ctcccgcccc cccagagaac acagcgcagc 4440
   acggaaattc tacaagacct gacggacagg aacatctccg acttcttggt aaaaacgtat 4500
   cctgctctta taagaagcag cttaaagagc aaattctggg tcaatgaaca gaggtatgga 4560
   ggaatttcca ttggaggaaa gctcccagtc gtccccatca cgggggaagc acttgttggg 4620
   tttttaagcg accttggccg gatcatgaat gtgagcgggg gccctatcac tagagaggcc 4680
   tctaaagaaa tacctgattt ccttaaacat ctagaaactg aagacaacat taaggtgtgg 4740
   tttaataaca aaggctggca tgccctggtc agctttctca atgtggccca caacgccatc 4800
   ttacgggcca gcctgcctaa ggacaggagc cccgaggagt atggaatcac cgtcattagc 4860
   caacccctga acctgaccaa ggagcagctc tcagagatta cagtgctgac cacttcagtg 4920
   gatgctgtgg ttgccatctg cgtgattttc tccatgtcct tcgtcccagc cagctttgtc 4980
   ctttatttga tccaggagcg ggtgaacaaa tccaagcacc tccagtttat cagtggagtg 5040
   agccccacca cctactgggt gaccaacttc ctctgggaca tcatgaatta ttccgtgagt 5100
   gctgggctgg tggtgggcat cttcatcggg tttcagaaga aagcctacac ttctccagaa 5160
   aaccttcctg cccttgtggc actgctcctg ctgtatggat gggcggtcat tcccatgatg 5220
   tacccagcat ccttcctgtt tgatgtcccc agcacagcct atgtggcttt atcttgtgct 5280
   aatctgttca tcggcatcaa cagcagtgct attaccttca tcttggaatt atttgataat 5340
   aaccggacgc tgctcaggtt caacgccgtg ctgaggaagc tgctcattgt cttcccccac 5400
   ttctgcctgg gccggggcct cattgacctt gcactgagcc aggctgtgac agatgtctat 5460
   gcccggtttg gtgaggagca ctctgcaaat ccgttccact gggacctgat tgggaagaac 5520
   ctgtttgcca tggtggtgga aggggtggtg tacttcctcc tgaccctgct ggtccagcgc 5580
   cacttcttcc tctcccaatg gattgccgag cccactaagg agcccattgt tgatgaagat 5640
   gatgatgtgg ctgaagaaag acaaagaatt attactggtg gaaataaaac tgacatctta 5700
   aggctacatg aactaaccaa gatttatcca ggcacctcca gcccagcagt ggacaggctg 5760
   tgtgtcggag ttcgccctgg agagtgcttt ggcctcctgg gagtgaatgg tgccggcaaa 5820
   acaaccacat tcaagatgct cactggggac accacagtga cctcagggga tgccaccgta 5880
   gcaggcaaga gtattttaac caatatttct gaagtccatc aaaatatggg ctactgtcct 5940
   cagtttgatg caatcgatga gctgctcaca ggacgagaac atctttacct ttatgcccgg 6000
   cttcgaggtg taccagcaga agaaatcgaa aaggttgcaa actggagtat taagagcctg 6060
   ggcctgactg tctacgccga ctgcctggct ggcacgtaca gtgggggcaa caagcggaaa 6120
   ctctccacag ccatcgcact cattggctgc ccaccgctgg tgctgctgga tgagcccacc 6180
   acagggatgg acccccaggc acgccgcatg ctgtggaacg tcatcgtgag catcatcaga 6240
   gaagggaggg ctgtggtcct cacatcccac agcatggaag aatgtgaggc actgtgtacc 6300
   cggctggcca tcatggtaaa gggcgccttt cgatgtatgg gcaccattca gcatctcaag 6360
   tccaaatttg gagatggcta tatcgtcaca atgaagatca aatccccgaa ggacgacctg 6420
   cttcctgacc tgaaccctgt ggagcagttc ttccagggga acttcccagg cagtgtgcag 6480
   agggagaggc actacaacat gctccagttc caggtctcct cctcctccct ggcgaggatc 6540
   ttccagctcc tcctctccca caaggacagc ctgctcatcg aggagtactc agtcacacag 6600
   accacactgg accaggtgtt tgtaaatttt gctaaacagc agactgaaag tcatgacctc 6660
   cctctgcacc ctcgagctgc tggagccagt cgacaagccc aggac 6705
<210> 6
   <211> 2235
   <212> PRT
   <213> Homo sapiens
<400> 6
   Met Gly Phe Val Arg Gln Ile Gln Leu Leu Leu Trp Lys Asn Trp Thr
   1               5                   10                  15
   Leu Arg Lys Arg Gln Lys Ile Arg Phe Val Val Glu Leu Val Trp Pro
               20                  25                  30
   Leu Ser Leu Phe Leu Val Leu Ile Trp Leu Arg Asn Ala Asn Pro Leu
           35                  40                  45
   Tyr Ser His His Glu Cys His Phe Pro Asn Lys Ala Met Pro Ser Ala
       50                  55                  60
   Gly Met Leu Pro Trp Leu Gln Gly Ile Phe Cys Asn Val Asn Asn Pro
   65                  70                  75                  80
   Cys Phe Gln Ser Pro Thr Pro Gly Glu Ser Pro Gly Ile Val Ser Asn
                   85                  90                  95
   Tyr Asn Asn Ser Ile Leu Ala Arg Val Tyr Arg Asp Phe Gln Glu Leu
               100                 105                 110
   Leu Met Asn Ala Pro Glu Ser Gln His Leu Gly Arg Ile Trp Thr Glu
           115                 120                 125
   Leu His Ile Leu Ser Gln Phe Met Asp Thr Leu Arg Thr His Pro Glu
       130                 135                 140
   Arg Ile Ala Gly Arg Gly Ile Arg Ile Arg Asp Ile Leu Lys Asp Glu
   145                 150                 155                 160
   Glu Thr Leu Thr Lys Phe Leu Ile Lys Asn Ile Gly Leu Ser Asp Ser
   165 170 175
   Val Val Tyr Leu Leu Ile Asn Ser Gln Val Arg Pro Glu Gln Phe Ala
   180 185 190
   His Gly Val Pro Asp Leu Ala Leu Lys Asp Ile Ala Cys Ser Glu Ala
   195 200 205
   Leu Leu Glu Arg Phe Ile Ile Phe Ser Gln Arg Arg Gly Ala Lys Thr
   210 215 220
   Val Arg Tyr Ala Lys Cys Ser Leu Ser Gln Gly Thr Leu Gln Trp Ile
   225 230 235 240
   Glu Asp Thr Leu Tyr Ala Asn Val Asp Phe Phe Lys Leu Phe Arg Val
   245 250 255
   Leu Pro Thr Leu Leu Asp Ser Arg Ser Gln Gly Ile Asn Leu Arg Ser
   260 265 270
   Trp Gly Gly Ile Leu Ser Asp Met Ser Pro Arg Ile Gln Glu Phe Ile
   275 280 285
   His Arg Pro Ser Met Gln Asp Leu Leu Trp Val Thr Arg Pro Leu Met
   290 295 300
   Gln Asn Gly Gly Pro Glu Thr Phe Thr Lys Leu Met Gly Ile Leu Ser
   305 310 315 320
   Asp Leu Leu Cys Gly Tyr Pro Glu Gly Gly Gly Ser Arg Val Leu Ser
   325 330 335
   Phe Asn Trp Tyr Glu Asp Asn Asn Tyr Lys Ala Phe Leu Gly Ile Asp
   340 345 350
   Ser Thr Arg Lys Asp Pro Ile Tyr Ser Tyr Asp Arg Arg Thr Thr Ser
   355 360 365
   Phe Cys Asn Ala Leu Ile Gln Ser Leu Glu Ser Asn Pro Leu Thr Lys
   370 375 380
   Ile Ala Trp Arg Ala Ala Lys Pro Leu Leu Met Gly Lys Ile Leu Tyr
   385 390 395 400
   Thr Pro Asp Ser Pro Ala Ala Arg Arg Ile Leu Lys Asn Ala Asn Ser
   405 410 415
   Thr Phe Glu Glu Leu Glu His Val Arg Lys Leu Val Lys Ala Trp Glu
   420 425 430
   Glu Val Gly Pro Gln Ile Trp Tyr Phe Phe Asp Asn Ser Thr Gln Met
   435 440 445
   Asn Met Ile Arg Asp Thr Leu Gly Asn Pro Thr Val Lys Asp Phe Leu
   450 455 460
   Asn Arg Gln Leu Gly Glu Glu Gly Ile Thr Ala Glu Ala Ile Leu Asn
   465 470 475 480
   Phe Leu Tyr Lys Gly Pro Arg Glu Ser Gln Ala Asp Asp Met Ala Asn
   485 490 495
   Phe Asp Trp Arg Asp Ile Phe Asn Ile Thr Asp Arg Thr Leu Arg Leu
   500 505 510
   Val Asn Gln Tyr Leu Glu Cys Leu Val Leu Asp Lys Phe Glu Ser Tyr
   515 520 525
   Asn Asp Glu Thr Gln Leu Thr Gln Arg Ala Leu Ser Leu Leu Glu Glu
   530 535 540
   Asn Met Phe Trp Ala Gly Val Val Phe Pro Asp Met Tyr Pro Trp Thr
   545 550 555 560
   Ser Ser Leu Pro Pro His Val Lys Tyr Lys Ile Arg Met Asp Ile Asp
   565 570 575
   Val Val Glu Lys Thr Asn Lys Ile Lys Asp Arg Tyr Trp Asp Ser Gly
   580 585 590
   Pro Arg Ala Asp Pro Val Glu Asp Phe Arg Tyr Ile Trp Gly Gly Phe
   595 600 605
   Ala Tyr Leu Gln Asp Met Val Glu Gln Gly Ile Thr Arg Ser Gln Val
   610 615 620
   Gln Ala Glu Ala Pro Val Gly Ile Tyr Leu Gln Gln Met Pro Tyr Pro
   625                 630                 635                 640
   Cys Phe Val Asp Asp Ser Phe Met Ile Ile Leu Asn Arg Cys Phe Pro
                   645                 650                 655
   Ile Phe Met Val Leu Ala Trp Ile Tyr Ser Val Ser Met Thr Val Lys
               660                 665                 670
   Ser Ile Val Leu Glu Lys Glu Leu Arg Leu Lys Glu Thr Leu Lys Asn
           675                 680                 685
   Gln Gly Val Ser Asn Ala Val Ile Trp Cys Thr Trp Phe Leu Asp Ser
       690                 695                 700
   Phe Ser Ile Met Ser Met Ser Ile Phe Leu Leu Thr Ile Phe Ile Met
   705                 710                 715                 720
   His Gly Arg Ile Leu His Tyr Ser Asp Pro Phe Ile Leu Phe Leu Phe
                   725                 730                 735
   Leu Leu Ala Phe Ser Thr Ala Thr Ile Met Leu Cys Phe Leu Leu Ser
               740                 745                 750
   Thr Phe Phe Ser Lys Ala Ser Leu Ala Ala Ala Cys Ser Gly Val Ile
           755                 760                 765
   Tyr Phe Thr Leu Tyr Leu Pro His Ile Leu Cys Phe Ala Trp Gln Asp
       770                 775                 780
   Arg Met Thr Ala Glu Leu Lys Lys Ala Val Ser Leu Leu Ser Pro Val
   785                 790                 795                 800
   Ala Phe Gly Phe Gly Thr Glu Tyr Leu Val Arg Phe Glu Glu Gln Gly
                   805                 810                 815
   Leu Gly Leu Gln Trp Ser Asn Ile Gly Asn Ser Pro Thr Glu Gly Asp
               820                 825                 830
   Glu Phe Ser Phe Leu Leu Ser Met Gln Met Met Leu Leu Asp Ala Ala
           835                 840                 845
   Cys Tyr Gly Leu Leu Ala Trp Tyr Leu Asp Gln Val Phe Pro Gly Asp
   850 855 860
   Tyr Gly Thr Pro Leu Pro Trp Tyr Phe Leu Leu Gln Glu Ser Tyr Trp
   865 870 875 880
   Leu Gly Gly Glu Gly Cys Ser Thr Arg Glu Glu Arg Ala Leu Glu Lys
   885 890 895
   Thr Glu Pro Leu Thr Glu Glu Thr Glu Asp Pro Glu His Pro Glu Gly
   900 905 910
   Ile His Asp Ser Phe Phe Glu Arg Glu His Pro Gly Trp Val Pro Gly
   915 920 925
   Val Cys Val Lys Asn Leu Val Lys Ile Phe Glu Pro Cys Gly Arg Pro
   930 935 940
   Ala Val Asp Arg Leu Asn Ile Thr Phe Tyr Glu Asn Gln Ile Thr Ala
   945 950 955 960
   Phe Leu Gly His Asn Gly Ala Gly Lys Thr Thr Thr Leu Ser Ile Leu
   965 970 975
   Thr Gly Leu Leu Pro Pro Thr Ser Gly Thr Val Leu Val Gly Gly Arg
   980 985 990
   Asp Ile Glu Thr Ser Leu Asp Ala Val Arg Gln Ser Leu Gly Met Cys
   995 1000 1005
   Pro Gln His Asn Ile Leu Phe His His Leu Thr Val Ala Glu His
   1010 1015 1020
   Met Leu Phe Tyr Ala Gln Leu Lys Gly Lys Ser Gln Glu Glu Ala
   1025 1030 1035
   Gln Leu Glu Met Glu Ala Met Leu Glu Asp Thr Gly Leu His His
   1040 1045 1050
   Lys Arg Asn Glu Glu Ala Gln Asp Leu Ser Gly Gly Met Gln Arg
   1055 1060 1065
   Lys Leu Ser Val Ala Ile Ala Phe Val Gly Asp Ala Lys Val Val
   1070 1075 1080
   Ile Leu Asp Glu Pro Thr Ser Gly Val Asp Pro Tyr Ser Arg Arg
   1085 1090 1095
   Ser Ile Trp Asp Leu Leu Leu Lys Tyr Arg Ser Gly Arg Thr Ile
   1100 1105 1110
   Ile Met Ser Thr His His Met Asp Glu Ala Asp Leu Leu Gly Asp
   1115 1120 1125
   Arg Ile Ala Ile Ile Ala Gln Gly Arg Leu Tyr Cys Ser Gly Thr
   1130 1135 1140
   Pro Leu Phe Leu Lys Asn Cys Phe Gly Thr Gly Leu Tyr Leu Thr
   1145 1150 1155
   Leu Val Arg Lys Met Lys Asn Ile Gln Ser Gln Arg Lys Gly Ser
   1160 1165 1170
   Glu Gly Thr Cys Ser Cys Ser Ser Lys Gly Phe Ser Thr Thr Cys
   1175 1180 1185
   Pro Ala His Val Asp Asp Leu Thr Pro Glu Gln Val Leu Asp Gly
   1190 1195 1200
   Asp Val Asn Glu Leu Met Asp Val Val Leu His His Val Pro Glu
   1205 1210 1215
   Ala Lys Leu Val Glu Cys Ile Gly Gln Glu Leu Ile Phe Leu Leu
   1220 1225 1230
   Pro Asn Lys Asn Phe Lys His Arg Ala Tyr Ala Ser Leu Phe Arg
   1235 1240 1245
   Glu Leu Glu Glu Thr Leu Ala Asp Leu Gly Leu Ser Ser Phe Gly
   1250 1255 1260
   Ile Ser Asp Thr Pro Leu Glu Glu Ile Phe Leu Lys Val Thr Glu
   1265 1270 1275
   Asp Ser Asp Ser Gly Pro Leu Phe Ala Gly Gly Ala Gln Gln Lys
   1280 1285 1290
   Arg Glu Asn Val Asn Pro Arg His Pro Cys Leu Gly Pro Arg Glu
   1295 1300 1305
   Lys Ala Gly Gln Thr Pro Gln Asp Ser Asn Val Cys Ser Pro Gly
   1310 1315 1320
   Ala Pro Ala Ala His Pro Glu Gly Gln Pro Pro Pro Glu Pro Glu
   1325 1330 1335
   Cys Pro Gly Pro Gln Leu Asn Thr Gly Thr Gln Leu Val Leu Gln
   1340 1345 1350
   His Val Gln Ala Leu Leu Val Lys Arg Phe Gln His Thr Ile Arg
   1355 1360 1365
   Ser His Lys Asp Phe Leu Ala Gln Ile Val Leu Pro Ala Thr Phe
   1370 1375 1380
   Val Phe Leu Ala Leu Met Leu Ser Ile Val Ile Leu Pro Phe Gly
   1385 1390 1395
   Glu Tyr Pro Ala Leu Thr Leu His Pro Trp Ile Tyr Gly Gln Gln
   1400 1405 1410
   Tyr Thr Phe Phe Ser Met Asp Glu Pro Gly Ser Glu Gln Phe Thr
   1415 1420 1425
   Val Leu Ala Asp Val Leu Leu Asn Lys Pro Gly Phe Gly Asn Arg
   1430 1435 1440
   Cys Leu Lys Glu Gly Trp Leu Pro Cys Ser Thr Arg Glu Lys Leu
   1445 1450 1455
   Thr Met Leu Pro Glu Cys Pro Glu Gly Ala Gly Gly Leu Pro Pro
   1460 1465 1470
   Pro Gln Arg Thr Gln Arg Ser Thr Glu Ile Leu Gln Asp Leu Thr
   1475 1480 1485
   Asp Arg Asn Ile Ser Asp Phe Leu Val Lys Thr Tyr Pro Ala Leu
   1490 1495 1500
   Ile Arg Ser Ser Leu Lys Ser Lys Phe Trp Val Asn Glu Gln Arg
   1505 1510 1515
   Thr Gly Gly Ile Ser Ile Gly Gly Lys Leu Pro Val Val Pro Ile
   1520 1525 1530
   Thr Gly Glu Ala Leu Val Gly Phe Leu Ser Asp Leu Gly Arg Ile
   1535 1540 1545
   Met Asn Val Ser Gly Gly Pro Ile Thr Arg Glu Ala Ser Lys Glu
   1550 1555 1560
   Ile Pro Asp Phe Leu Lys His Leu Glu Thr Glu Asp Asn Ile Lys
   1565 1570 1575
   Val Trp Phe Asn Asn Lys Gly Trp His Ala Leu Val Ser Phe Leu
   1580 1585 1590
   Asn Val Ala His Asn Ala Ile Leu Arg Ala Ser Leu Pro Lys Asp
   1595 1600 1605
   Arg Ser Pro Glu Glu Tyr Gly Ile Thr Val Ile Ser Gln Pro Leu
   1610 1615 1620
   Asn Leu Thr Lys Glu Gln Leu Ser Glu Ile Thr Val Leu Thr Thr
   1625 1630 1635
   Ser Val Asp Ala Val Val Ala Ile Cys Val Ile Phe Ser Met Ser
   1640 1645 1650
   Phe Val Pro Ala Ser Phe Val Leu Tyr Leu Ile Gln Glu Arg Val
   1655 1660 1665
   Asn Lys Ser Lys His Leu Gln Phe Ile Ser Gly Val Ser Pro Thr
   1670 1675 1680
   Thr Tyr Trp Val Thr Asn Phe Leu Trp Ser Ile Met Asn Tyr Ser
   1685 1690 1695
   Val Ser Ala Gly Leu Val Val Gly Ile Phe Ile Gly Phe Gln Lys
   1700 1705 1710
   Lys Ala Tyr Thr Ser Pro Glu Asn Leu Pro Ala Leu Val Ala Leu
   1715 1720 1725
   Leu Leu Leu Tyr Gly Trp Ala Val Ile Pro Met Met Tyr Pro Ala
   1730 1735 1740
   Ser Phe Leu Phe Asp Val Pro Ser Thr Ala Tyr Val Ala Leu Ser
   1745 1750 1755
   Cys Ala Asn Leu Phe Ile Gly Ile Asn Ser Ser Ala Ile Thr Phe
   1760 1765 1770
   Ile Leu Glu Leu Phe Asp Asn Asn Arg Thr Leu Leu Arg Phe Asn
   1775 1780 1785
   Ala Val Leu Arg Lys Leu Leu Ile Val Phe Pro His Phe Cys Leu
   1790 1795 1800
   Gly Arg Gly Leu Ile Asp Leu Ala Leu Ser Gln Ala Val Thr Asp
   1805 1810 1815
   Val Tyr Ala Arg Phe Gly Glu Glu His Ser Ala Asn Pro Phe His
   1820 1825 1830
   Trp Asp Leu Ile Gly Lys Asn Leu Phe Ala Met Val Val Glu Gly
   1835 1840 1845
   Val Val Tyr Phe Leu Leu Thr Leu Leu Val Gln Arg His Phe Phe
   1850 1855 1860
   Leu Ser Gln Trp Ile Ala Glu Pro Thr Lys Glu Pro Ile Val Asp
   1865 1870 1875
   Glu Asp Asp Asp Val Ala Glu Glu Arg Gln Arg Ile Ile Thr Gly
   1880 1885 1890
   Gly Asn Lys Thr Asp Ile Leu Arg Leu His Glu Leu Thr Lys Ile
   1895 1900 1905
   Tyr Pro Gly Thr Ser Ser Pro Ala Val Asp Arg Leu Cys Val Gly
   1910 1915 1920
   Val Arg Pro Gly Glu Cys Phe Gly Leu Leu Gly Val Asn Gly Ala
   1925 1930 1935
   Gly Lys Thr Thr Thr Phe Lys Met Leu Thr Gly Asp Thr Thr Val
   1940 1945 1950
   Thr Ser Gly Asp Ala Thr Val Ala Gly Lys Ser Ile Leu Thr Asn
   1955 1960 1965
   Ile Ser Glu Val His Gln Asn Met Gly Tyr Cys Pro Gln Phe Asp
   1970 1975 1980
   Ala Ile Asp Glu Leu Leu Thr Gly Arg Glu His Leu Tyr Leu Tyr
   1985 1990 1995
   Ala Arg Leu Arg Gly Val Pro Ala Glu Glu Ile Glu Lys Leu Ala
   2000 2005 2010
   Asn Trp Ser Ile Lys Ser Leu Gly Leu Thr Val Tyr Ala Asp Cys
   2015 2020 2025
   Leu Ala Gly Thr Tyr Ser Gly Gly Asn Lys Arg Lys Leu Ser Thr
   2030 2035 2040
   Ala Ile Ala Leu Ile Gly Cys Pro Pro Leu Val Leu Leu Asp Glu
   2045 2050 2055
   Pro Thr Thr Gly Met Asp Pro Gln Ala Arg Arg Met Leu Trp Asn
   2060 2065 2070
   Val Ile Val Ser Ile Ile Arg Glu Gly Arg Ala Val Val Leu Thr
   2075 2080 2085
   Ser His Ser Met Glu Glu Cys Glu Ala Leu Cys Thr Arg Leu Ala
   2090 2095 2100
   Ile Met Val Lys Gly Ala Phe Arg Cys Met Gly Thr Ile Gln His
   2105 2110 2115
   Leu Lys Ser Lys Phe Gly Asp Gly Tyr Ile Val Thr Met Lys Ile
   2120 2125 2130
   Lys Ser Pro Lys Asp Asp Leu Leu Pro Asp Leu Asn Pro Val Glu
   2135 2140 2145
   Gln Phe Phe Gln Gly Asn Phe Pro Gly Ser Val Gln Arg Glu Arg
   2150 2155 2160
   His Tyr Asn Met Leu Gln Phe Gln Val Ser Ser Ser Ser Leu Ala
   2165 2170 2175
   Arg Ile Phe Gln Leu Leu Leu Ser His Lys Asp Ser Leu Leu Ile
   2180 2185 2190
   Glu Glu Tyr Ser Val Thr Gln Thr Thr Leu Asp Gln Val Phe Val
   2195 2200 2205
   Asn Phe Ala Lys Gln Gln Thr Glu Ser His Asp Leu Pro Leu His
   2210 2215 2220
   Pro Arg Ala Ala Gly Ala Ser Arg Gln Ala Gln Asp
   2225 2230 2235
<210> 7
   <211> 22
   <212> DNA
   <213> Artificial Sequence
<220>
   <223> Oligonucleotide primer
<400> 7
   atcctctgac tcagcaatca ca 22
<210> 8
   <211> 21
   <212> DNA
   <213> Artificial Sequence
<220>
   <223> Oligonucleotide primer
<400> 8
   ttgcaattac aaatgcaatg g 21
<210> 9
   <211> 20
   <212> DNA
   <213> Artificial Sequence
<220>
   <223> Oligonucleotide primer
<400> 9
   atccataccc ttcccactcc 20
<210> 10
   <211> 21
   <212> DNA
   <213> Artificial Sequence
<220>
   <223> Oligonucleotide primer
<400> 10
   gcagcagaag ataagcacac c 21
<210> 11
   <211> 38
   <212> PRT
   <213> Homo sapiens
<400> 11
   Glu Tyr Pro Cys Gly Asn Ser Thr Pro Trp Lys Thr Pro Ser Val Ser
   1               5                   10                  15
   Pro Asn Ile Thr Gln Leu Phe Gln Lys Gln Lys Trp Thr Gln Val Asn
               20                  25                  30
   Pro Ser Pro Ser Cys Arg
           35
<210> 12
   <211> 20
   <212> DNA
   <213> Artificial Sequence
<220>
   <223> Oligonucleotide primer
<400> 12
   accctctgct aagctcagag 20
<210> 13
   <211> 19
   <212> DNA
   <213> Artificial Sequence
<220>
   <223> Oligonucleotide primer
<400> 13
   accccacact tccaacctg 19
<210> 14
   <211> 20
   <212> DNA
   <213> Artificial Sequence
<220>
   <223> Oligonucleotide primer
<400> 14
   aagtcctact gcacacatgg 20
<210> 15
   <211> 19
   <212> DNA
   <213> Artificial Sequence
<220>
   <223> Oligonucleotide primer
<400> 15
   acactcccac cccaagatc 19
<210> 16
   <211> 19
   <212> DNA
   <213> Artificial Sequence
<220>
   <223> Oligonucleotide primer
<400> 16
   ttcccaaaaa ggccaactc 19
<210> 17
   <211> 19
   <212> DNA
   <213> Artificial Sequence
<220>
   <223> Oligonucleotide primer
<400> 17
   cacgcacgtg tgcattcag 19
<210> 18
   <211> 22
   <212> DNA
   <213> Artificial Sequence
<220>
   <223> Oligonucleotide primer
<400> 18
   gctatttcct tattaatgag gc 22
<210> 19
   <211> 20
   <212> DNA
   <213> Artificial Sequence
<220>
   <223> Oligonucleotide primer
<400> 19
   ccaactctcc ctgttctttc 20
<210> 20
   <211> 20
   <212> DNA
   <213> Artificial Sequence
<220>
   <223> Oligonucleotide primer
<400> 20
   tgtttccaat cgactctggc 20
<210> 21
   <211> 21
   <212> DNA
   <213> Artificial Sequence
<220>
   <223> Oligonucleotide primer
<400> 21
   ttcttgcctt tctcaggctg g 21
<210> 22
   <211> 19
   <212> DNA
   <213> Artificial Sequence
<220>
   <223> Oligonucleotide primer
<400> 22
   gtattcccag gttctgtgg 19
<210> 23
   <211> 19
   <212> DNA
   <213> Artificial Sequence
<220>
   <223> Oligonucleotide primer
<400> 23
   taccccagga atcaccttg 19
<210> 24
   <211> 21
   <212> DNA
   <213> Artificial Sequence
<220>
   <223> Oligonucleotide primer
<400> 24
   agcatatagg agatcagact g 21
<210> 25
   <211> 21
   <212> DNA
   <213> Artificial Sequence
<220>
   <223> Oligonucleotide primer
<400> 25
   tgacataagt ggggtaaatg g 21
<210> 26
   <211> 20
   <212> DNA
   <213> Artificial Sequence
<220>
   <223> Oligonucleotide primer
<400> 26
   gagcattggc ctcacagcag 20
<210> 27
   <211> 18
   <212> DNA
   <213> Artificial Sequence
<220>
   <223> Oligonucleotide primer
<400> 27
   ccccaggttt gtttcacc 18
<210> 28
   <211> 21
   <212> DNA
   <213> Artificial Sequence
<220>
   <223> Oligonucleotide primer
<400> 28
   agacatgtga tgtggataca c 21
<210> 29
   <211> 20
   <212> DNA
   <213> Artificial Sequence
<220>
   <223> Oligonucleotide primer
<400> 29
   gtgggaggtc cagggtacac 20
<210> 30
   <211> 19
   <212> DNA
   <213> Artificial Sequence
<220>
   <223> Oligonucleotide primer
<400> 30
   aggggcagaa aagacacac 19
<210> 31
   <211> 21
   <212> DNA
   <213> Artificial Sequence
<220>
   <223> Oligonucleotide primer
<400> 31
   tagcgattaa ctctttcctg g 21
<210> 32
   <211> 19
   <212> DNA
   <213> Artificial Sequence
<220>
   <223> Oligonucleotide primer
<400> 32
   ctcttcaggg agccttagc 19
<210> 33
   <211> 20
   <212> DNA
   <213> Artificial Sequence
<220>
   <223> Oligonucleotide primer
<400> 33
   ttcaagacca cttgacttgc 20
<210> 34
   <211> 18
   <212> DNA
   <213> Artificial Sequence
<220>
   <223> Oligonucleotide primer
<400> 34
   tgggacagca gccttatc 18
<210> 35
   <211> 22
   <212> DNA
   <213> Artificial Sequence
<220>
   <223> Oligonucleotide primer
<400> 35
   ccaaatgtaa tttcccactg ac 22
<210> 36
   <211> 21
   <212> DNA
   <213> Artificial Sequence
<220>
   <223> Oligonucleotide primer
<400> 36
   aatgagttcc gagtcaccct g 21
<210> 37
   <211> 18
   <212> DNA
   <213> Artificial Sequence
<220>
   <223> Oligonucleotide primer
<400> 37
   cccattcgcg tgtcatgg 18
<210> 38
   <211> 20
   <212> DNA
   <213> Artificial Sequence
<220>
   <223> Oligonucleotide primer
<400> 38
   tccatctggg ctttgttctc 20
<210> 39
   <211> 20
   <212> DNA
   <213> Artificial Sequence
<220>
   <223> Oligonucleotide primer
<400> 39
   aatccaggca catgaacagg 20
<210> 40
   <211> 18
   <212> DNA
   <213> Artificial Sequence
<220>
   <223> Oligonucleotide primer
<400> 40
   aggctggtgg gagagagc 18
<210> 41
   <211> 18
   <212> DNA
   <213> Artificial Sequence
<220>
   <223> Oligonucleotide primer
<400> 41
   agtggacccc ctcagagg 18
<210> 42
   <211> 21
   <212> DNA
   <213> Artificial Sequence
<220>
   <223> Oligonucleotide primer
<400> 42
   ctgttgcatt ggataaaagg c 21
<210> 43
   <211> 19
   <212> DNA
   <213> Artificial Sequence
<220>
   <223> Oligonucleotide primer
<400> 43
   gatgaatgga gagggctgg 19
<210> 44
   <211> 21
   <212> DNA
   <213> Artificial Sequence
<220>
   <223> Oligonucleotide primer
<400> 44
   ctgcggtaag gtaggatagg g 21
<210> 45
   <211> 21
   <212> DNA
   <213> Artificial Sequence
<220>
   <223> Oligonucleotide primer
<400> 45
   cacaccgttt acatagaggg c 21
<210> 46
   <211> 19
   <212> DNA
   <213> Artificial Sequence
<220>
   <223> Oligonucleotide primer
<400> 46
   cctctcccct cctttcctg 19
<210> 47
   <211> 19
   <212> DNA
   <213> Artificial Sequence
<220>
   <223> Oligonucleotide primer
<400> 47
   gtcagtttcc gtaggcttc 19
<210> 48
   <211> 19
   <212> DNA
   <213> Artificial Sequence
<220>
   <223> Oligonucleotide primer
<400> 48
   tggggccatg taattaggc 19
<210> 49
   <211> 20
   <212> DNA
   <213> Artificial Sequence
<220>
   <223> Oligonucleotide primer
<400> 49
   tgggaaagag tagacagccg 20
<210> 50
   <211> 18
   <212> DNA
   <213> Artificial Sequence
<220>
   <223> Oligonucleotide primer
<400> 50
   actgaacctg gtgtgggg 18
<210> 51
   <211> 19
   <212> DNA
   <213> Artificial Sequence
<220>
   <223> Oligonucleotide primer
<400> 51
   tatctctgcc tgtgcccag 19
<210> 52
   <211> 20
   <212> DNA
   <213> Artificial Sequence
<220>
   <223> Oligonucleotide primer
<400> 52
   gtaagatcag ctgctggaag 20
<210> 53
   <211> 20
   <212> DNA
   <213> Artificial Sequence
<220>
   <223> Oligonucleotide primer
<400> 53
   gaagctctcc tgcaccaagc 20
<210> 54
   <211> 18
   <212> DNA
   <213> Artificial Sequence
<220>
   <223> Oligonucleotide primer
<400> 54
   aggtaccccc acaatgcc 18
<210> 55
   <211> 22
   <212> DNA
   <213> Artificial Sequence
<220>
   <223> Oligonucleotide primer
<400> 55
   tcattgtggt tccagtactc ag 22
<210> 56
   <211> 22
   <212> DNA
   <213> Artificial Sequence
<220>
   <223> Oligonucleotide primer
<400> 56
   tttttgcaac tatatagcca gg 22
<210> 57
   <211> 20
   <212> DNA
   <213> Artificial Sequence
<220>
   <223> Oligonucleotide primer
<400> 57
   agcctgtgtg agtagccatg 20
<210> 58
   <211> 19
   <212> DNA
   <213> Artificial Sequence
<220>
   <223> Oligonucleotide primer
<400> 58
   gcatcagggc gaggctgtc 19
<210> 59
   <211> 20
   <212> DNA
   <213> Artificial Sequence
<220>
   <223> Oligonucleotide primer
<400> 59
   cccagcaata ctgggagatg 20
<210> 60
   <211> 19
   <212> DNA
   <213> Artificial Sequence
<220>
   <223> Oligonucleotide primer
<400> 60
   ggtaacctca cagtcttcc 19
<210> 61
   <211> 19
   <212> DNA
   <213> Artificial Sequence
<220>
   <223> Oligonucleotide primer
<400> 61
   gggaacgatg gctttttgc 19
<210> 62
   <211> 20
   <212> DNA
   <213> Artificial Sequence
<220>
   <223> Oligonucleotide primer
<400> 62
   tcccattatg aagcaatacc 20
<210> 63
   <211> 19
   <212> DNA
   <213> Artificial Sequence
<220>
   <223> Oligonucleotide primer
<400> 63
   ccttagactt tcgagatgg 19
<210> 64
   <211> 23
   <212> DNA
   <213> Artificial Sequence
<220>
   <223> Oligonucleotide primer
<400> 64
   gctaccagcc tggtatttca ttg 23
<210> 65
   <211> 20
   <212> DNA
   <213> Artificial Sequence
<220>
   <223> Oligonucleotide primer
<400> 65
   gttataaccc atgcctgaag 20
<210> 66
   <211> 18
   <212> DNA
   <213> Artificial Sequence
<220>
   <223> Oligonucleotide primer
<400> 66
   tgcacgcgca cgtgtgac 18
<210> 67
   <211> 21
   <212> DNA
   <213> Artificial Sequence
<220>
   <223> Oligonucleotide primer
<400> 67
   tgaaggtccc agtgaagtgg g 21
<210> 68
   <211> 20
   <212> DNA
   <213> Artificial Sequence
<220>
   <223> Oligonucleotide primer
<400> 68
   cagcagctat ccagtaaagg 20
<210> 69
   <211> 19
   <212> DNA
   <213> Artificial Sequence
<220>
   <223> Oligonucleotide primer
<400> 69
   aacgcctgcc atcttgaac 19
<210> 70
   <211> 20
   <212> DNA
   <213> Artificial Sequence
<220>
   <223> Oligonucleotide primer
<400> 70
   gttgggcaca attcttatgc 20
<210> 71
   <211> 20
   <212> DNA
   <213> Artificial Sequence
<220>
   <223> Oligonucleotide primer
<400> 71
   gttgtttgga ggtcaggtac 20
<210> 72
   <211> 21
   <212> DNA
   <213> Artificial Sequence
<220>
   <223> Oligonucleotide primer
<400> 72
   aacatcaccc agctgttcca g 21
<210> 73
   <211> 20
   <212> DNA
   <213> Artificial Sequence
<220>
   <223> Oligonucleotide primer
<400> 73
   actcaggaga taccagggac 20
<210> 74
   <211> 22
   <212> DNA
   <213> Artificial Sequence
<220>
   <223> Oligonucleotide primer
<400> 74
   ggaagacaac aagcagtttc ac 22
<210> 75
   <211> 20
   <212> DNA
   <213> Artificial Sequence
<220>
   <223> Oligonucleotide primer
<400> 75
   atctactgcc ctgatcatac 20
<210> 76
   <211> 21
   <212> DNA
   <213> Artificial Sequence
<220>
   <223> Oligonucleotide primer
<400> 76
   aagactgaga cttcagtctt c 21
<210> 77
   <211> 22
   <212> DNA
   <213> Artificial Sequence
<220>
   <223> Oligonucleotide primer
<400> 77
   ggtgtgcctt ttaaaagtgt gc 22
<210> 78
   <211> 22
   <212> DNA
   <213> Artificial Sequence
<220>
   <223> Oligonucleotide primer
<400> 78
   ttcatgtttc cctacaaaac cc 22
<210> 79
   <211> 22
   <212> DNA
   <213> Artificial Sequence
<220>
   <223> Oligonucleotide primer
<400> 79
   catgagagtt tctcattcat gg 22
<210> 80
   <211> 22
   <212> DNA
   <213> Artificial Sequence
<220>
   <223> Oligonucleotide primer
<400> 80
   tgtttacatg gtttttaggg cc 22
<210> 81
   <211> 19
   <212> DNA
   <213> Artificial Sequence
<220>
   <223> Oligonucleotide primer
<400> 81
   ttcagcagga ggagggatg 19
<210> 82
   <211> 22
   <212> DNA
   <213> Artificial Sequence
<220>
   <223> Oligonucleotide primer
<400> 82
   cctttccttc actgatttct gc 22
<210> 83
   <211> 18
   <212> DNA
   <213> Artificial Sequence
<220>
   <223> Oligonucleotide primer
<400> 83
   aatcagcact tcgcggtg 18
<210> 84
   <211> 19
   <212> DNA
   <213> Artificial Sequence
<220>
   <223> Oligonucleotide primer
<400> 84
   tgtaaggcct tcccaaagc 19
<210> 85
   <211> 20
   <212> DNA
   <213> Artificial Sequence
<220>
   <223> Oligonucleotide primer
<400> 85
   tggtccttca gcgcacacac 20
<210> 86
   <211> 20
   <212> DNA
   <213> Artificial Sequence
<220>
   <223> Oligonucleotide primer
<400> 86
   cattttgcag agctggcagc 20
<210> 87
   <211> 20
   <212> DNA
   <213> Artificial Sequence
<220>
   <223> Oligonucleotide primer
<400> 87
   cttctgtcag gagatgatcc 20
<210> 88
   <211> 21
   <212> DNA
   <213> Artificial Sequence
<220>
   <223> Oligonucleotide primer
<400> 88
   ggagtgcatt atatccagac g 21
<210> 89
   <211> 20
   <212> DNA
   <213> Artificial Sequence
<220>
   <223> Oligonucleotide primer
<400> 89
   cctggctctg cttgaccaac 20
<210> 90
   <211> 20
   <212> DNA
   <213> Artificial Sequence
<220>
   <223> Oligonucleotide primer
<400> 90
   tgctgtcctg tgagagcatc 20
<210> 91
   <211> 19
   <212> DNA
   <213> Artificial Sequence
<220>
   <223> Oligonucleotide primer
<400> 91
   gtaaccctcc cagctttgg 19
<210> 92
   <211> 20
   <212> DNA
   <213> Artificial Sequence
<220>
   <223> Oligonucleotide primer
<400> 92
   cagttcccac ataaggcctg 20
<210> 93
   <211> 19
   <212> DNA
   <213> Artificial Sequence
<220>
   <223> Oligonucleotide primer
<400> 93
   cagttctgga tgccctgag 19
<210> 94
   <211> 22
   <212> DNA
   <213> Artificial Sequence
<220>
   <223> Oligonucleotide primer
<400> 94
   gaagagaggt cccatggaaa gg 22
<210> 95
   <211> 22
   <212> DNA
   <213> Artificial Sequence
<220>
   <223> Oligonucleotide primer
<400> 95
   gcttgcataa gcatatcaat tg 22
<210> 96
   <211> 21
   <212> DNA
   <213> Artificial Sequence
<220>
   <223> Oligonucleotide primer
<400> 96
   ctcctaaacc atcctttgct c 21
<210> 97
   <211> 18
   <212> DNA
   <213> Artificial Sequence
<220>
   <223> Oligonucleotide primer
<400> 97
   aggcaggcac aagagctg 18
<210> 98
   <211> 18
   <212> DNA
   <213> Artificial Sequence
<220>
   <223> Oligonucleotide primer
<400> 98
   cttaccctgg ggcctgac 18
<210> 99
   <211> 21
   <212> DNA
   <213> Artificial Sequence
<220>
   <223> Oligonucleotide primer
<400> 99
   ctcagagcca ccctactata g 21
<210> 100
   <211> 20
   <212> DNA
   <213> Artificial Sequence
<220>
   <223> Oligonucleotide primer
<400> 100
   gaagcttctc cagccctagc 20
<210> 101
   <211> 20
   <212> DNA
   <213> Artificial Sequence
<220>
   <223> Oligonucleotide primer
<400> 101
   tgcactctca tgaaacaggc 20
<210> 102
   <211> 20
   <212> DNA
   <213> Artificial Sequence
<220>
   <223> Oligonucleotide primer
<400> 102
   gtttggggtg tttgcttgtc 20
<210> 103
   <211> 21
   <212> DNA
   <213> Artificial Sequence
<220>
   <223> Oligonucleotide primer
<400> 103
   acctctttcc ccaacccaga g 21
<210> 104
   <211> 20
   <212> DNA
   <213> Artificial Sequence
<220>
   <223> Oligonucleotide primer
<400> 104
   gaagcagtaa tcagaagggc 20
<210> 105
   <211> 21
   <212> DNA
   <213> Artificial Sequence
<220>
   <223> Oligonucleotide primer
<400> 105
   gcctcacatt cttccatgct g 21
<210> 106
   <211> 20
   <212> DNA
   <213> Artificial Sequence
<220>
   <223> Oligonucleotide primer
<400> 106
   tcacatccca caggcaagag 20
<210> 107
   <211> 21
   <212> DNA
   <213> Artificial Sequence
<220>
   <223> Oligonucleotide primer
<400> 107
   ttccaagtgt caatggagaa c 21
<210> 108
   <211> 20
   <212> DNA
   <213> Artificial Sequence
<220>
   <223> Oligonucleotide primer
<400> 108
   attaccttag gcccaaccac 20
<210> 109
   <211> 19
   <212> DNA
   <213> Artificial Sequence
<220>
   <223> Oligonucleotide primer
<400> 109
   acactgggtg ttctggacc 19
<210> 110
   <211> 19
   <212> DNA
   <213> Artificial Sequence
<220>
   <223> Oligonucleotide primer
<400> 110
   gtgtagggtg gtgttttcc 19
<210> 111
   <211> 20
   <212> DNA
   <213> Artificial Sequence
<220>
   <223> Oligonucleotide primer
<400> 111
   aagcccagtg aaccagctgg 20
<210> 112
   <211> 19
   <212> DNA
   <213> Artificial Sequence
<220>
   <223> Oligonucleotide primer
<400> 112
   tcagctgagt gcccttcag 19
<210> 113
   <211> 21
   <212> DNA
   <213> Artificial Sequence
<220>
   <223> Oligonucleotide primer
<400> 113
   aggtgagcaa gtcagtttcg g 21
<210> 114
   <211> 20
   <212> DNA
   <213> Artificial Sequence
<220>
   <223> Oligonucleotide primer
<400> 114
   ggtcttcgtg tgtggtcatt 20
<210> 115
   <211> 18
   <212> DNA
   <213> Artificial Sequence
<220>
   <223> Oligonucleotide primer
<400> 115
   ggtccagttc ttccagag 18
<210> 116
   <211> 22
   <212> DNA
   <213> Artificial Sequence
<220>
   <223> Oligonucleotide primer
<400> 116
   atcctctgac tcagcaatca ca 22
<210> 117
   <211> 21
   <212> DNA
   <213> Artificial Sequence
<220>
   <223> Oligonucleotide primer
<400> 117
   ttgcaattac aaatgcaatg g 21
<210> 118
   <211> 2261
   <212> PRT
   <213> Mouse
<400> 118
   Met Ala Cys Lys Pro Gln Leu Arg Leu Leu Leu Trp Lys Asn Leu Thr
   1 5 10 15
   Phe Arg Arg Arg Gln Thr Cys Gln Leu Leu Leu Glu Val Ala Trp Pro
   20 25 30
   Leu Phe Ile Phe Leu Ile Leu Ile Ser Val Arg Leu Ser Tyr Pro Pro
   35 40 45
   Tyr Glu Gln His Glu Cys His Phe Pro Asn Lys Ala Met Pro Ser Ala
   50 55 60
   Gly Thr Leu Pro Trp Val Gln Gly Ile Ile Cys Asn Ala Asn Asn Pro
   65 70 75 80
   Cys Phe Arg Tyr Pro Thr Pro Gly Glu Ala Pro Gly Val Val Gly Asn
   85 90 95
   Phe Asn Lys Ser Ile Val Ser Arg Leu Phe Ser Asp Ala Gln Arg Leu
   100 105 110
   Leu Leu Tyr Ser Gln Arg Asp Thr Ser Ile Lys Asp Met His Lys Val
   115 120 125
   Leu Arg Met Leu Arg Gln Ile Lys His Pro Asn Ser Asn Leu Lys Leu
   130 135 140
   Gln Asp Phe Leu Val Asp Asn Glu Thr Phe Ser Gly Phe Leu Gln His
   145 150 155 160
   Asn Leu Ser Leu Pro Arg Ser Thr Val Asp Ser Leu Leu Gln Lys Asn
   165 170 175
   Val Gly Leu Gln Lys Val Phe Leu Gln Gly Tyr Gln Leu His Leu Ala
   180 185 190
   Ser Leu Cys Asn Gly Ser Lys Leu Glu Glu Ile Ile Gln Leu Gly Asp
   195 200 205
   Ala Glu Val Ser Ala Leu Cys Gly Leu Pro Arg Lys Lys Leu Asp Ala
   210 215 220
   Ala Glu Arg Val Leu Arg Tyr Asn Met Asp Ile Leu Lys Pro Val Val
   225 230 235 240
   Thr Lys Leu Asn Ser Thr Ser His Leu Pro Thr Gln His Leu Ala Glu
   245 250 255
   Ala Thr Thr Val Leu Leu Asp Ser Leu Gly Gly Leu Ala Gln Glu Leu
   260 265 270
   Phe Ser Thr Lys Ser Trp Ser Asp Met Arg Gln Glu Val Met Phe Leu
   275 280 285
   Thr Asn Val Asn Ser Ser Ser Ser Ser Thr Gln Ile Tyr Gln Ala Val
   290 295 300
   Ser Arg Ile Val Cys Gly His Pro Glu Gly Gly Gly Leu Lys Ile Lys
   305 310 315 320
   Ser Leu Asn Trp Tyr Glu Asp Asn Asn Tyr Lys Ala Leu Phe Gly Gly
   325 330 335
   Asn Asn Thr Glu Glu Asp Val Asp Thr Phe Tyr Asp Asn Ser Thr Thr
   340 345 350
   Pro Tyr Cys Asn Asp Leu Met Lys Asn Leu Glu Ser Ser Pro Leu Ser
   355 360 365
   Arg Ile Ile Trp Lys Ala Leu Lys Pro Leu Leu Val Gly Lys Ile Leu
   370 375 380
   Tyr Thr Pro Asp Thr Pro Ala Thr Arg Gln Val Met Ala Glu Val Asn
   385 390 395 400
   Lys Thr Phe Gln Glu Leu Ala Val Phe His Asp Leu Glu Gly Met Trp
   405 410 415
   Glu Glu Leu Ser Pro Gln Ile Trp Thr Phe Met Glu Asn Ser Gln Glu
   420 425 430
   Met Asp Leu Val Arg Thr Leu Leu Asp Ser Arg Gly Asn Asp Gln Phe
   435 440 445
   Trp Glu Gln Lys Leu Asp Gly Leu Asp Trp Thr Ala Gln Asp Ile Met
   450 455 460
   Ala Phe Leu Ala Lys Asn Pro Glu Asp Val Gln Ser Pro Asn Gly Ser
   465 470 475 480
   Val Tyr Thr Trp Arg Glu Ala Phe Asn Glu Thr Asn Gln Ala Ile Gln
   485 490 495
   Thr Ile Ser Arg Phe Met Glu Cys Val Asn Leu Asn Lys Leu Glu Pro
   500 505 510
   Ile Pro Thr Glu Val Arg Leu Ile Asn Lys Ser Met Glu Leu Leu Asp
   515 520 525
   Glu Arg Lys Phe Trp Ala Gly Ile Val Phe Thr Gly Ile Thr Pro Asp
   530 535 540
   Ser Val Glu Leu Pro His His Val Lys Tyr Lys Ile Arg Met Asp Ile
   545 550 555 560
   Asp Asn Val Glu Arg Thr Asn Lys Ile Lys Asp Gly Tyr Trp Asp Pro
   565 570 575
   Gly Pro Arg Ala Asp Pro Phe Glu Asp Met Arg Tyr Val Trp Gly Gly
   580 585 590
   Phe Ala Tyr Leu Gln Asp Val Val Glu Gln Ala Ile Ile Arg Val Leu
   595 600 605
   Thr Gly Ser Glu Lys Lys Thr Gly Val Tyr Val Gln Gln Met Pro Tyr
   610 615 620
   Pro Cys Tyr Val Asp Asp Ile Phe Leu Arg Val Met Ser Arg Ser Met
   625 630 635 640
   Pro Leu Phe Met Thr Leu Ala Trp Ile Tyr Ser Val Ala Val Ile Ile
   645 650 655
   Lys Ser Ile Val Tyr Glu Lys Glu Ala Arg Leu Lys Glu Thr Met Arg
   660 665 670
   Ile Met Gly Leu Asp Asn Gly Ile Leu Trp Phe Ser Trp Phe Val Ser
   675 680 685
   Ser Leu Ile Pro Leu Leu Val Ser Ala Gly Leu Leu Val Val Ile Leu
   690 695 700
   Lys Leu Gly Asn Leu Leu Pro Tyr Ser Asp Pro Ser Val Val Phe Val
   705 710 715 720
   Phe Leu Ser Val Phe Ala Met Val Thr Ile Leu Gln Cys Phe Leu Ile
   725 730 735
   Ser Thr Leu Phe Ser Arg Ala Asn Leu Ala Ala Ala Cys Gly Gly Ile
   740 745 750
   Ile Tyr Phe Thr Leu Tyr Leu Pro Tyr Val Leu Cys Val Ala Trp Gln
   755 760 765
   Asp Tyr Val Gly Phe Ser Ile Lys Ile Phe Ala Ser Leu Leu Ser Pro
   770 775 780
   Val Ala Phe Gly Phe Gly Cys Glu Tyr Phe Ala Leu Phe Glu Glu Gln
   785 790 795 800
   Gly Ile Gly Val Gln Trp Asp Asn Leu Phe Glu Ser Pro Val Glu Glu
   805 810 815
   Asp Gly Phe Asn Leu Thr Thr Ala Val Ser Met Met Leu Phe Asp Thr
   820 825 830
   Phe Leu Tyr Gly Val Met Thr Trp Tyr Ile Glu Ala Val Phe Pro Gly
   835 840 845
   Gln Tyr Gly Ile Pro Arg Pro Trp Tyr Phe Pro Cys Thr Lys Ser Tyr
   850 855 860
   Trp Phe Gly Glu Glu Ile Asp Glu Lys Ser His Pro Gly Ser Ser Gln
   865 870 875 880
   Lys Gly Val Ser Glu Ile Cys Met Glu Glu Glu Pro Thr His Leu Arg
   885 890 895
   Leu Gly Val Ser Ile Gln Asn Leu Val Lys Val Tyr Arg Asp Gly Met
   900 905 910
   Lys Val Ala Val Asp Gly Leu Ala Leu Asn Phe Tyr Glu Gly Gln Ile
   915 920 925
   Thr Ser Phe Leu Gly His Asn Gly Ala Gly Lys Thr Thr Thr Met Ser
   930 935 940
   Ile Leu Thr Gly Leu Phe Pro Pro Thr Ser Gly Thr Ala Tyr Ile Leu
   945 950 955 960
   Gly Lys Asp Ile Arg Ser Glu Met Ser Ser Ile Arg Gln Asn Leu Gly
   965 970 975
   Val Cys Pro Gln His Asn Val Leu Phe Asp Met Leu Thr Val Glu Glu
   980 985 990
   His Ile Trp Phe Tyr Ala Arg Leu Lys Gly Leu Ser Glu Lys His Val
   995 1000 1005
   Lys Ala Glu Met Glu Gln Met Ala Leu Asp Val Gly Leu Pro Pro
   1010 1015 1020
   Ser Lys Leu Lys Ser Lys Thr Ser Gln Leu Ser Gly Gly Met Gln
   1025 1030 1035
   Arg Lys Leu Ser Val Ala Leu Ala Phe Val Gly Gly Ser Lys Val
   1040 1045 1050
   Val Ile Leu Asp Glu Pro Thr Ala Gly Val Asp Pro Tyr Ser Arg
   1055 1060 1065
   Arg Gly Ile Trp Glu Leu Leu Leu Lys Tyr Arg Gln Gly Arg Thr
   1070 1075 1080
   Ile Ile Leu Ser Thr His His Met Asp Glu Ala Asp Ile Leu Gly
   1085 1090 1095
   Asp Arg Ile Ala Ile Ile Ser His Gly Lys Leu Cys Cys Val Gly
   1100 1105 1110
   Ser Ser Leu Phe Leu Lys Asn Gln Leu Gly Thr Gly Tyr Tyr Leu
   1115 1120 1125
   Thr Leu Val Lys Lys Asp Val Glu Ser Ser Leu Ser Ser Cys Arg
   1130 1135 1140
   Asn Ser Ser Ser Thr Val Ser Cys Leu Lys Lys Glu Asp Ser Val
   1145 1150 1155
   Ser Gln Ser Ser Ser Asp Ala Gly Leu Gly Ser Asp His Glu Ser
   1160 1165 1170
   Asp Thr Leu Thr Ile Asp Val Ser Ala Ile Ser Asn Leu Ile Arg
   1175 1180 1185
   Lys His Val Ser Glu Ala Arg Leu Val Glu Asp Ile Gly His Glu
   1190 1195 1200
   Leu Thr Tyr Val Leu Pro Tyr Glu Ala Ala Lys Glu Gly Ala Phe
   1205 1210 1215
   Val Glu Leu Phe His Glu Ile Asp Asp Arg Leu Ser Asp Leu Gly
   1220 1225 1230
   Ile Ser Ser Tyr Gly Ile Ser Glu Thr Thr Leu Glu Glu Ile Phe
   1235 1240 1245
   Leu Lys Val Ala Glu Glu Ser Gly Val Asp Ala Glu Thr Ser Asp
   1250 1255 1260
   Gly Thr Leu Pro Ala Arg Arg Asn Arg Arg Ala Phe Gly Asp Lys
   1265 1270 1275
   Gln Ser Cys Leu His Pro Phe Thr Glu Asp Asp Ala Val Asp Pro
   1280 1285 1290
   Asn Asp Ser Asp Ile Asp Pro Glu Ser Arg Glu Thr Asp Leu Leu
   1295 1300 1305
   Ser Gly Met Asp Gly Lys Gly Ser Tyr Gln Leu Lys Gly Trp Lys
   1310 1315 1320
   Leu Thr Gln Gln Gln Phe Val Ala Leu Leu Trp Lys Arg Leu Leu
   1325 1330 1335
   Ile Ala Arg Arg Ser Arg Lys Gly Phe Phe Ala Gln Ile Val Leu
   1340 1345 1350
   Pro Ala Val Phe Val Cys Ile Ala Leu Val Phe Ser Leu Ile Val
   1355 1360 1365
   Pro Pro Phe Gly Lys Tyr Pro Ser Leu Glu Leu Gln Pro Trp Met
   1370 1375 1380
   Tyr Asn Glu Gln Tyr Thr Phe Val Ser Asn Asp Ala Pro Glu Asp
   1385 1390 1395
   Met Gly Thr Gln Glu Leu Leu Asn Ala Leu Thr Lys Asp Pro Gly
   1400 1405 1410
   Phe Gly Thr Arg Cys Met Glu Gly Asn Pro Ile Pro Asp Thr Pro
   1415 1420 1425
   Cys Leu Ala Gly Glu Glu Asp Trp Thr Ile Ser Pro Val Pro Gln
   1430 1435 1440
   Ser Ile Val Asp Leu Phe Gln Asn Gly Asn Trp Thr Met Lys Asn
   1445 1450 1455
   Pro Ser Pro Ala Cys Gln Cys Ser Ser Asp Lys Ile Lys Lys Met
   1460 1465 1470
   Leu Pro Val Cys Pro Pro Gly Ala Gly Gly Leu Pro Pro Pro Gln
   1475 1480 1485
   Arg Lys Gln Lys Thr Ala Asp Ile Leu Gln Asn Leu Thr Gly Arg
   1490 1495 1500
   Asn Ile Ser Asp Tyr Leu Val Lys Thr Tyr Val Gln Ile Ile Ala
   1505 1510 1515
   Lys Ser Leu Lys Asn Lys Ile Trp Val Asn Glu Phe Arg Tyr Gly
   1520 1525 1530
   Gly Phe Ser Leu Gly Val Ser Asn Ser Gln Ala Leu Pro Pro Ser
   1535 1540 1545
   His Glu Val Asn Asp Ala Ile Lys Gln Met Lys Lys Leu Leu Lys
   1550 1555 1560
   Leu Thr Lys Asp Thr Ser Ala Asp Arg Phe Leu Ser Ser Leu Gly
   1565 1570 1575
   Arg Phe Met Ala Gly Leu Asp Thr Lys Asn Asn Val Lys Val Trp
   1580 1585 1590
   Phe Asn Asn Lys Gly Trp His Ala Ile Ser Ser Phe Leu Asn Val
   1595 1600 1605
   Ile Asn Asn Ala Ile Leu Arg Ala Asn Leu Gln Lys Gly Glu Asn
   1610 1615 1620
   Pro Ser Gln Tyr Gly Ile Thr Ala Phe Asn His Pro Leu Asn Leu
   1625 1630 1635
   Thr Lys Gln Gln Leu Ser Glu Val Ala Leu Met Thr Thr Ser Val
   1640 1645 1650
   Asp Val Leu Val Ser Ile Cys Val Ile Phe Ala Met Ser Phe Val
   1655 1660 1665
   Pro Ala Ser Phe Val Val Phe Leu Ile Gln Glu Arg Val Ser Lys
   1670 1675 1680
   Ala Lys His Leu Gln Phe Ile Ser Gly Val Lys Pro Val Ile Tyr
   1685 1690 1695
   Trp Leu Ser Asn Phe Val Trp Asp Met Cys Asn Tyr Val Val Pro
   1700 1705 1710
   Ala Thr Leu Val Ile Ile Ile Phe Ile Gly Phe Gln Gln Lys Ser
   1715 1720 1725
   Tyr Val Ser Ser Thr Asn Leu Pro Val Leu Ala Leu Leu Leu Leu
   1730 1735 1740
   Leu Tyr Gly Trp Ser Ile Thr Pro Leu Met Tyr Pro Ala Ser Phe
   1745 1750 1755
   Val Phe Lys Ile Pro Ser Thr Ala Tyr Val Val Leu Thr Ser Val
   1760 1765 1770
   Asn Leu Phe Ile Gly Ile Asn Gly Ser Val Ala Thr Phe Val Leu
   1775 1780 1785
   Glu Leu Phe Thr Asn Asn Lys Leu Asn Asp Ile Asn Asp Ile Leu
   1790 1795 1800
   Lys Ser Val Phe Leu Ile Phe Pro His Phe Cys Leu Gly Arg Gly
   1805 1810 1815
   Leu Ile Asp Met Val Lys Asn Gln Ala Met Ala Asp Ala Leu Glu
   1820 1825 1830
   Arg Phe Gly Glu Asn Arg Phe Val Ser Pro Leu Ser Trp Asp Leu
   1835 1840 1845
   Val Gly Arg Asn Leu Phe Ala Met Ala Val Glu Gly Val Val Phe
   1850 1855 1860
   Phe Leu Ile Thr Val Leu Ile Gln Tyr Arg Phe Phe Ile Arg Pro
   1865 1870 1875
   Arg Pro Val Lys Ala Lys Leu Pro Pro Leu Asn Asp Glu Asp Glu
   1880 1885 1890
   Asp Val Arg Arg Glu Arg Gln Arg Ile Leu Asp Gly Gly Gly Gln
   1895 1900 1905
   Asn Asp Ile Leu Glu Ile Lys Glu Leu Thr Lys Ile Tyr Arg Arg
   1910 1915 1920
   Lys Arg Lys Pro Ala Val Asp Arg Ile Cys Ile Gly Ile Pro Pro
   1925 1930 1935
   Gly Glu Cys Phe Gly Leu Leu Gly Val Asn Gly Ala Gly Lys Ser
   1940 1945 1950
   Thr Thr Phe Lys Met Leu Thr Gly Asp Thr Pro Val Thr Arg Gly
   1955 1960 1965
   Asp Ala Phe Leu Asn Lys Asn Ser Ile Leu Ser Asn Ile His Glu
   1970 1975 1980
   Val His Gln Asn Met Gly Tyr Cys Pro Gln Phe Asp Ala Ile Thr
   1985 1990 1995
   Glu Leu Leu Thr Gly Arg Glu His Val Glu Phe Phe Ala Leu Leu
   2000 2005 2010
   Arg Gly Val Pro Glu Lys Glu Val Gly Lys Phe Gly Glu Trp Ala
   2015 2020 2025
   Ile Arg Lys Leu Gly Leu Val Lys Tyr Gly Glu Lys Tyr Ala Ser
   2030 2035 2040
   Asn Tyr Ser Gly Gly Asn Lys Arg Lys Leu Ser Thr Ala Met Ala
   2045 2050 2055
   Leu Ile Gly Gly Pro Pro Val Val Phe Leu Asp Glu Pro Thr Thr
   2060 2065 2070
   Gly Met Asp Pro Lys Ala Arg Arg Phe Leu Trp Asn Cys Ala Leu
   2075 2080 2085
   Ser Ile Val Lys Glu Gly Arg Ser Val Val Leu Thr Ser His Ser
   2090 2095 2100
   Met Glu Glu Cys Glu Ala Leu Cys Thr Arg Met Ala Ile Met Val
   2105 2110 2115
   Asn Gly Arg Phe Arg Cys Leu Gly Ser Val Gln His Leu Lys Asn
   2120 2125 2130
   Arg Phe Gly Asp Gly Tyr Thr Ile Val Val Arg Ile Ala Gly Ser
   2135 2140 2145
   Asn Pro Asp Leu Lys Pro Val Gln Glu Phe Phe Gly Leu Ala Phe
   2150 2155 2160
   Pro Gly Ser Val Leu Lys Glu Lys His Arg Asn Met Leu Gln Tyr
   2165 2170 2175
   Gln Leu Pro Ser Ser Leu Ser Ser Leu Ala Arg Ile Phe Ser Ile
   2180 2185 2190
   Leu Ser Gln Ser Lys Lys Arg Leu His Ile Glu Asp Tyr Ser Val
   2195 2200 2205
   Ser Gln Thr Thr Leu Asp Gln Val Phe Val Asn Phe Ala Lys Asp
   2210 2215 2220
   Gln Ser Asp Asp Asp His Leu Lys Asp Leu Ser Leu His Lys Asn
   2225 2230 2235
   Gln Thr Val Val Asp Val Ala Val Leu Thr Ser Phe Leu Gln Asp
   2240 2245 2250
   Glu Lys Val Lys Glu Ser Tyr Val
   2255 2260
<210> 119
   <211> 1472
   <212> PRT
   <213> Mouse
<400> 119
   Gln Ala Cys Ala Met Glu Ser Arg His Phe Glu Glu Thr Arg Gly Met
   1 5 10 15
   Glu Glu Glu Pro Thr His Leu Pro Leu Val Val Cys Val Asp Lys Leu
   20 25 30
   Thr Lys Val Tyr Lys Asn Asp Lys Lys Leu Ala Leu Asn Lys Leu Ser
   35 40 45
   Leu Asn Leu Tyr Glu Asn Gln Val Val Ser Phe Leu Gly His Asn Gly
   50 55 60
   Ala Gly Lys Thr Thr Thr Met Ser Ile Leu Thr Gly Leu Phe Pro Pro
   65 70 75 80
   Thr Ser Gly Ser Ala Thr Ile Tyr Gly His Asp Ile Arg Thr Glu Met
   85 90 95
   Asp Glu Ile Arg Lys Asn Leu Gly Met Cys Pro Gln His Asn Val Leu
   100 105 110
   Phe Asp Arg Leu Thr Val Glu Glu His Leu Trp Phe Tyr Ser Arg Leu
           115                 120                 125
   Lys Ser Met Ala Gln Glu Glu Ile Arg Lys Glu Thr Asp Lys Met Ile
       130                 135                 140
   Glu Asp Leu Glu Leu Ser Asn Lys Arg His Ser Leu Val Gln Thr Leu
   145                 150                 155                 160
   Ser Gly Gly Met Lys Arg Lys Leu Ser Val Ala Ile Ala Phe Val Gly
                   165                 170                 175
   Gly Ser Arg Ala Ile Ile Leu Asp Glu Pro Thr Ala Gly Val Asp Pro
               180                 185                 190
   Tyr Ala Arg Arg Ala Ile Trp Asp Leu Ile Leu Lys Tyr Lys Pro Gly
           195                 200                 205
   Arg Thr Ile Leu Leu Ser Thr His His Met Asp Glu Ala Asp Leu Leu
       210                 215                 220
   Gly Asp Arg Ile Ala Ile Ile Ser His Gly Lys Leu Lys Cys Cys Gly
   225                 230                 235                 240
   Ser Pro Leu Phe Leu Lys Gly Ala Tyr Lys Asp Gly Tyr Arg Leu Thr
                   245                 250                 255
   Leu Val Lys Gln Pro Ala Glu Pro Gly Thr Ser Gln Glu Pro Gly Leu
               260                 265                 270
   Ala Ser Ser Pro Ser Gly Cys Pro Arg Leu Ser Ser Cys Ser Glu Pro
           275                 280                 285
   Gln Val Ser Gln Phe Ile Arg Lys His Val Ala Ser Ser Leu Leu Val
       290                 295                 300
   Ser Asp Thr Ser Thr Glu Leu Ser Tyr Ile Leu Pro Ser Glu Ala Val
   305                 310                 315                 320
   Lys Lys Gly Ala Phe Glu Arg Leu Phe Gln Gln Leu Glu His Ser Leu
                   325                 330                 335
   Asp Ala Leu His Leu Ser Ser Phe Gly Leu Met Asp Thr Thr Leu Glu
   340 345 350
   Glu Val Phe Leu Lys Val Ser Glu Glu Asp Gln Ser Leu Glu Asn Ser
   355 360 365
   Glu Ala Asp Val Lys Glu Ser Arg Lys Asp Val Leu Pro Gly Ala Glu
   370 375 380
   Gly Leu Thr Ala Val Gly Gly Gln Ala Gly Asn Leu Ala Arg Cys Ser
   385 390 395 400
   Glu Leu Ala Gln Ser Gln Ala Ser Leu Gln Ser Ala Ser Ser Val Gly
   405 410 415
   Ser Ala Arg Gly Glu Glu Gly Thr Gly Tyr Ser Asp Gly Tyr Gly Asp
   420 425 430
   Tyr Arg Pro Leu Phe Asp Asn Leu Gln Asp Pro Asp Asn Val Ser Leu
   435 440 445
   Gln Glu Ala Glu Met Glu Ala Leu Ala Gln Val Gly Gln Gly Ser Arg
   450 455 460
   Lys Leu Glu Gly Trp Trp Leu Lys Met Arg Gln Phe His Gly Leu Leu
   465 470 475 480
   Val Lys Arg Phe His Cys Ala Arg Arg Asn Ser Lys Ala Leu Cys Ser
   485 490 495
   Gln Ile Leu Leu Pro Ala Phe Phe Val Cys Val Ala Met Thr Val Ala
   500 505 510
   Leu Ser Val Pro Glu Ile Gly Asp Leu Pro Pro Leu Val Leu Ser Pro
   515 520 525
   Ser Gln Tyr His Asn Tyr Thr Gln Pro Arg Gly Asn Phe Ile Pro Tyr
   530 535 540
   Ala Asn Glu Glu Arg Gln Glu Tyr Arg Leu Arg Leu Ser Pro Asp Ala
   545 550 555 560
   Ser Pro Gln Gln Leu Val Ser Thr Phe Arg Leu Pro Ser Gly Val Gly
   565 570 575
   Ala Thr Cys Val Leu Lys Ser Pro Ala Asn Gly Ser Leu Gly Pro Met
   580 585 590
   Leu Asn Leu Ser Ser Gly Glu Ser Arg Leu Leu Ala Ala Arg Phe Phe
   595 600 605
   Asp Ser Met Cys Leu Glu Ser Phe Thr Gln Gly Leu Pro Leu Ser Asn
   610 615 620
   Phe Val Pro Pro Pro Pro Ser Pro Ala Pro Ser Asp Ser Pro Val Lys
   625 630 635 640
   Pro Asp Glu Asp Ser Leu Gln Ala Trp Asn Met Ser Leu Pro Pro Thr
   645 650 655
   Ala Gly Pro Glu Thr Trp Thr Ser Ala Pro Ser Leu Pro Arg Leu Val
   660 665 670
   His Glu Pro Val Arg Cys Thr Cys Ser Ala Gln Gly Thr Gly Phe Ser
   675 680 685
   Cys Pro Ser Ser Val Gly Gly His Pro Pro Gln Met Arg Val Val Thr
   690 695 700
   Gly Asp Ile Leu Thr Asp Ile Thr Gly His Asn Val Ser Glu Tyr Leu
   705 710 715 720
   Leu Phe Thr Ser Asp Arg Phe Arg Leu His Arg Tyr Gly Ala Ile Thr
   725 730 735
   Phe Gly Asn Val Gln Lys Ser Ile Pro Ala Ser Phe Gly Ala Arg Val
   740 745 750
   Pro Pro Met Val Arg Lys Ile Ala Val Arg Arg Val Ala Gln Val Leu
   755 760 765
   Tyr Asn Asn Lys Gly Tyr His Ser Met Pro Thr Tyr Leu Asn Ser Leu
   770 775 780
   Asn Asn Ala Ile Leu Arg Ala Asn Leu Pro Lys Ser Lys Gly Asn Pro
   785 790 795 800
   Ala Ala Tyr Lys Ile Thr Val Thr Asn His Pro Met Asn Lys Thr Ser
   805 810 815
   Ala Ser Leu Ser Leu Asp Tyr Leu Leu Gln Gly Thr Asp Val Val Ile
   820 825 830
   Ala Ile Phe Ile Ile Val Ala Met Ser Phe Val Pro Ala Ser Phe Val
   835 840 845
   Val Phe Leu Val Ala Glu Lys Ser Thr Lys Ala Lys His Leu Gln Phe
   850 855 860
   Val Ser Gly Cys Asn Pro Val Ile Tyr Trp Leu Ala Asn Tyr Val Trp
   865 870 875 880
   Asp Met Leu Asn Tyr Leu Val Pro Ala Thr Cys Cys Val Ile Ile Leu
   885 890 895
   Phe Val Phe Asp Leu Pro Ala Tyr Thr Ser Pro Thr Asn Phe Pro Ala
   900 905 910
   Val Leu Ser Leu Phe Leu Leu Tyr Gly Trp Ser Ile Thr Pro Ile Met
   915 920 925
   Tyr Pro Ala Ser Phe Trp Phe Glu Val Pro Ser Ser Ala Tyr Val Phe
   930 935 940
   Leu Ile Val Ile Asn Leu Phe Ile Gly Ile Thr Ala Thr Val Ala Thr
   945 950 955 960
   Phe Leu Leu Gln Leu Phe Glu His Asp Lys Asp Leu Lys Val Val Asn
   965 970 975
   Ser Tyr Leu Lys Ser Cys Phe Leu Ile Phe Pro Asn Tyr Asn Leu Gly
   980 985 990
   His Gly Leu Met Glu Met Ala Tyr Asn Glu Tyr Ile Asn Glu Tyr Tyr
   995 1000 1005
   Ala Lys Ile Gly Gln Phe Asp Lys Met Lys Ser Pro Phe Glu Trp
   1010 1015 1020
   Asp Ile Val Thr Arg Gly Leu Val Ala Met Thr Val Glu Gly Phe
   1025 1030 1035
   Val Gly Phe Phe Leu Thr Ile Met Cys Gln Tyr Asn Phe Leu Arg
   1040 1045 1050
   Gln Pro Gln Arg Leu Pro Val Ser Thr Lys Pro Val Glu Asp Asp
   1055 1060 1065
   Val Asp Val Ala Ser Glu Arg Gln Arg Val Leu Arg Gly Asp Ala
   1070 1075 1080
   Asp Asn Asp Met Val Lys Ile Glu Asn Leu Thr Lys Val Tyr Lys
   1085 1090 1095
   Ser Arg Lys Ile Gly Arg Ile Leu Ala Val Asp Arg Leu Cys Leu
   1100 1105 1110
   Gly Val Cys Val Pro Gly Glu Cys Phe Gly Leu Leu Gly Val Asn
   1115 1120 1125
   Gly Ala Gly Lys Thr Ser Thr Phe Lys Met Leu Thr Gly Asp Glu
   1130 1135 1140
   Ser Thr Thr Gly Gly Glu Ala Phe Val Asn Gly His Ser Val Leu
   1145 1150 1155
   Lys Asp Leu Leu Gln Val Gln Gln Ser Leu Gly Tyr Cys Pro Gln
   1160 1165 1170
   Phe Asp Val Pro Val Asp Glu Leu Thr Ala Arg Glu His Leu Gln
   1175 1180 1185
   Leu Tyr Thr Arg Leu Arg Cys Ile Pro Trp Lys Asp Glu Ala Gln
   1190 1195 1200
   Val Val Lys Trp Ala Leu Glu Lys Leu Glu Leu Thr Lys Tyr Ala
   1205 1210 1215
   Asp Lys Pro Ala Gly Thr Tyr Ser Gly Gly Asn Lys Arg Lys Leu
   1220 1225 1230
   Ser Thr Ala Ile Ala Leu Ile Gly Tyr Pro Ala Phe Ile Phe Leu
   1235 1240 1245
   Asp Glu Pro Thr Thr Gly Met Asp Pro Lys Ala Arg Arg Phe Leu
   1250 1255 1260
   Trp Asn Leu Ile Leu Asp Leu Ile Lys Thr Gly Arg Ser Val Val
   1265 1270 1275
   Leu Thr Ser His Ser Met Glu Glu Cys Glu Ala Leu Cys Thr Arg
   1280 1285 1290
   Leu Ala Ile Met Val Asn Gly Arg Leu His Cys Leu Gly Ser Ile
   1295 1300 1305
   Gln His Leu Lys Asn Arg Phe Gly Asp Gly Tyr Met Ile Thr Val
   1310 1315 1320
   Arg Thr Lys Ser Ser Gln Asn Val Lys Asp Val Val Arg Phe Phe
   1325 1330 1335
   Asn Arg Asn Phe Pro Glu Ala His Ala Gln Gly Lys Thr Pro Tyr
   1340 1345 1350
   Lys Val Gln Tyr Gln Leu Lys Ser Glu His Ile Ser Leu Ala Gln
   1355 1360 1365
   Val Phe Ser Lys Met Glu Gln Val Val Gly Val Leu Gly Ile Glu
   1370 1375 1380
   Asp Tyr Ser Val Ser Gln Thr Thr Leu Asp Asn Val Phe Val Asn
   1385 1390 1395
   Phe Ala Lys Lys Gln Ser Asp Asn Val Glu Gln Gln Glu Ala Glu
   1400 1405 1410
   Pro Ser Ser Leu Pro Ser Pro Leu Gly Leu Leu Ser Leu Leu Arg
   1415 1420 1425
   Pro Arg Pro Ala Pro Thr Glu Leu Arg Ala Leu Val Ala Asp Glu
   1430 1435 1440
   Pro Glu Asp Leu Asp Thr Glu Asp Glu Gly Leu Ile Ser Phe Glu
   1445 1450 1455
   Glu Glu Arg Ala Gln Leu Ser Phe Asn Thr Asp Thr Leu Cys
   1460 1465 1470
<210> 120
   <211> 1704
   <212> PRT
   <213> Homo sapiens
<400> 120
   Met Ala Val Leu Arg Gln Leu Ala Leu Leu Leu Trp Lys Asn Tyr Thr
   1 5 10 15
   Leu Gln Lys Arg Lys Val Leu Val Thr Val Leu Glu Leu Phe Leu Pro
   20 25 30
   Leu Leu Phe Ser Gly Ile Leu Ile Trp Leu Arg Leu Lys Ile Gln Ser
   35 40 45
   Glu Asn Val Pro Asn Ala Thr Ile Tyr Pro Gly Gln Ser Ile Gln Glu
   50 55 60
   Leu Pro Leu Phe Phe Thr Phe Pro Pro Pro Gly Asp Thr Trp Glu Leu
   65 70 75 80
   Ala Tyr Ile Pro Ser His Ser Asp Ala Ala Lys Thr Val Thr Glu Thr
   85 90 95
   Val Arg Arg Ala Leu Val Ile Asn Met Arg Val Arg Gly Phe Pro Ser
   100 105 110
   Glu Lys Asp Phe Glu Asp Tyr Ile Arg Tyr Asp Asn Cys Ser Ser Ser
   115 120 125
   Val Leu Ala Ala Val Val Phe Glu His Pro Phe Asn His Ser Lys Glu
   130 135 140
   Pro Leu Pro Leu Ala Val Lys Tyr His Leu Arg Phe Ser Tyr Thr Arg
   145 150 155 160
   Arg Asn Tyr Met Trp Thr Gln Thr Gly Ser Phe Phe Leu Lys Glu Thr
   165 170 175
   Glu Gly Trp His Thr Thr Ser Leu Phe Pro Leu Phe Pro Asn Pro Gly
   180 185 190
   Pro Arg Glu Pro Thr Ser Pro Asp Gly Gly Glu Pro Gly Tyr Ile Arg
   195 200 205
   Glu Gly Phe Leu Ala Val Gln His Ala Val Asp Arg Ala Ile Met Glu
   210 215 220
   Tyr His Ala Asp Ala Ala Thr Arg Gln Leu Phe Gln Arg Leu Thr Val
   225 230 235 240
   Thr Ile Lys Arg Phe Pro Tyr Pro Pro Phe Ile Glu Asp Pro Phe Leu
   245 250 255
   Val Ala Ile Gln Tyr Gln Leu Pro Leu Leu Leu Leu Leu Ser Phe Thr
   260 265 270
   Tyr Thr Ala Leu Thr Ile Ala Arg Ala Val Val Gln Glu Lys Glu Arg
   275 280 285
   Arg Leu Lys Glu Tyr Met Arg Met Met Gly Leu Ser Ser Trp Leu His
   290 295 300
   Trp Ser Ala Trp Phe Leu Leu Phe Phe Leu Phe Leu Leu Ile Ala Ala
   305 310 315 320
   Ser Phe Met Thr Leu Leu Phe Cys Val Lys Val Lys Pro Asn Val Ala
   325 330 335
   Val Leu Ser Arg Ser Asp Pro Ser Leu Val Leu Ala Phe Leu Leu Cys
   340 345 350
   Phe Ala Ile Ser Thr Ile Ser Phe Ser Phe Met Val Ser Thr Phe Phe
   355 360 365
   Ser Lys Ala Asn Met Ala Ala Ala Phe Gly Gly Phe Leu Tyr Phe Phe
   370 375 380
   Thr Tyr Ile Pro Tyr Phe Phe Val Ala Pro Arg Tyr Asn Trp Met Thr
   385 390 395 400
   Leu Ser Gln Lys Leu Cys Ser Cys Leu Leu Ser Asn Val Ala Met Ala
   405 410 415
   Met Gly Ala Gln Leu Ile Gly Lys Phe Glu Ala Lys Gly Met Gly Ile
   420 425 430
   Gln Trp Arg Asp Leu Leu Ser Pro Val Asn Val Asp Asp Asp Phe Cys
   435 440 445
   Phe Gly Gln Val Leu Gly Met Leu Leu Leu Asp Ser Val Leu Tyr Gly
   450 455 460
   Leu Val Thr Trp Tyr Met Glu Ala Val Phe Pro Gly Gln Phe Gly Val
   465 470 475 480
   Pro Gln Pro Trp Tyr Phe Phe Ile Met Pro Ser Tyr Trp Cys Gly Lys
   485 490 495
   Pro Arg Ala Val Ala Gly Lys Glu Glu Glu Asp Ser Asp Pro Glu Lys
   500 505 510
   Ala Leu Arg Asn Glu Tyr Phe Glu Ala Glu Pro Glu Asp Leu Val Ala
   515 520 525
   Gly Ile Lys Ile Lys His Leu Ser Lys Val Phe Arg Val Gly Asn Lys
   530 535 540
   Asp Arg Ala Ala Val Arg Asp Leu Asn Leu Asn Leu Tyr Glu Gly Gln
   545 550 555 560
   Ile Thr Val Leu Leu Gly His Asn Gly Ala Gly Lys Thr Thr Thr Leu
   565 570 575
   Ser Met Leu Thr Gly Leu Phe Pro Pro Thr Ser Gly Arg Ala Tyr Ile
   580 585 590
   Ser Gly Tyr Glu Ile Ser Gln Asp Met Val Gln Ile Arg Lys Ser Leu
   595 600 605
   Gly Leu Cys Pro Gln His Asp Ile Leu Phe Asp Asn Leu Thr Val Ala
   610 615 620
   Glu His Leu Tyr Phe Tyr Ala Gln Leu Lys Gly Leu Ser Arg Gln Lys
   625 630 635 640
   Cys Pro Glu Glu Val Lys Gln Met Leu His Ile Ile Gly Leu Glu Asp
                   645                 650                 655
   Lys Trp Asn Ser Arg Ser Arg Phe Leu Ser Gly Gly Met Arg Arg Lys
               660                 665                 670
   Leu Ser Ile Gly Ile Ala Leu Ile Ala Gly Ser Lys Val Leu Ile Leu
           675                 680                 685
   Asp Glu Pro Thr Ser Gly Met Asp Ala Ile Ser Arg Arg Ala Ile Trp
       690                 695                 700
   Asp Leu Leu Gln Arg Gln Lys Ser Asp Arg Thr Ile Val Leu Thr Thr
   705                 710                 715                 720
   His Phe Met Asp Glu Ala Asp Leu Leu Gly Asp Arg Ile Ala Ile Met
                   725                 730                 735
   Ala Lys Gly Glu Leu Gln Cys Cys Gly Ser Ser Leu Phe Leu Lys Gln
               740                 745                 750
   Lys Tyr Gly Ala Gly Tyr His Met Thr Leu Val Lys Glu Pro His Cys
           755                 760                 765
   Asn Pro Glu Asp Ile Ser Gln Leu Val His His His Val Pro Asn Ala
       770                 775                 780
   Thr Leu Glu Ser Ser Ala Gly Ala Glu Leu Ser Phe Ile Leu Pro Arg
   785                 790                 795                 800
   Glu Ser Thr His Arg Phe Glu Gly Leu Phe Ala Lys Leu Glu Lys Lys
                   805                 810                 815
   Gln Lys Glu Leu Gly Ile Ala Ser Phe Gly Ala Ser Ile Thr Thr Met
               820                 825                 830
   Glu Glu Val Phe Leu Arg Val Gly Lys Leu Val Asp Ser Ser Met Asp
           835                 840                 845
   Ile Gln Ala Ile Gln Leu Pro Ala Leu Gln Tyr Gln His Glu Arg Arg
       850                 855                 860
   Ala Ser Asp Trp Ala Val Asp Ser Asn Leu Cys Gly Ala Met Asp Pro
   865                 870                 875                 880
   Ser Asp Gly Ile Gly Ala Leu Ile Glu Glu Glu Arg Thr Ala Val Lys
                   885                 890                 895
   Leu Asn Thr Gly Leu Ala Leu His Cys Gln Gln Phe Trp Ala Met Phe
               900                 905                 910
   Leu Lys Lys Ala Ala Tyr Ser Trp Arg Glu Trp Lys Met Val Ala Ala
           915                 920                 925
   Gln Val Leu Val Pro Leu Thr Cys Val Thr Leu Ala Leu Leu Ala Ile
       930                 935                 940
   Asn Tyr Ser Ser Glu Leu Phe Asp Asp Pro Met Leu Arg Leu Thr Leu
   945                 950                 955                 960
   Gly Glu Tyr Gly Arg Thr Val Val Pro Phe Ser Val Pro Gly Thr Ser
                   965                 970                 975
   Gln Leu Gly Gln Gln Leu Ser Glu His Leu Lys Asp Ala Leu Gln Ala
               980                 985                 990
   Glu Gly Gln Glu Pro Arg Glu Val  Leu Gly Asp Leu Glu  Glu Phe Leu
           995                 1000                1005
   Ile Phe  Arg Ala Ser Val Glu  Gly Gly Gly Phe Asn  Glu Arg Cys
       1010                1015                1020
   Leu Val  Ala Ala Ser Phe Arg  Asp Val Gly Glu Arg  Thr Val Val
       1025                1030                1035
   Asn Ala  Leu Phe Asn Asn Gln  Ala Tyr His Ser Pro  Ala Thr Ala
       1040                1045                1050
   Leu Ala  Val Val Asp Asn Leu  Leu Phe Lys Leu Leu  Cys Gly Pro
       1055                1060                1065
   His Ala  Ser Ile Val Val Ser  Asn Phe Pro Gln Pro  Arg Ser Ala
       1070                1075                1080
   Leu Gln Ala Ala Lys Asp Gln Phe Asn Glu Gly Arg Lys Gly Phe
   1085 1090 1095
   Asp Ile Ala Leu Asn Leu Leu Phe Ala Met Ala Phe Leu Ala Ser
   1100 1105 1110
   Thr Phe Ser Ile Leu Ala Val Ser Glu Arg Ala Val Gln Ala Lys
   1115 1120 1125
   His Val Gln Phe Val Ser Gly Val His Val Ala Ser Phe Trp Leu
   1130 1135 1140
   Ser Ala Leu Leu Trp Asp Leu Ile Ser Phe Leu Ile Pro Ser Leu
   1145 1150 1155
   Leu Leu Leu Val Val Phe Lys Ala Phe Asp Val Arg Ala Phe Thr
   1160 1165 1170
   Arg Asp Gly His Met Ala Asp Thr Leu Leu Leu Leu Leu Leu Tyr
   1175 1180 1185
   Gly Trp Ala Ile Ile Pro Leu Met Tyr Leu Met Asn Phe Phe Phe
   1190 1195 1200
   Leu Gly Ala Ala Thr Ala Tyr Thr Arg Leu Thr Ile Phe Asn Ile
   1205 1210 1215
   Leu Ser Gly Ile Ala Thr Phe Leu Met Val Thr Ile Met Arg Ile
   1220 1225 1230
   Pro Ala Val Lys Leu Glu Glu Leu Ser Lys Thr Leu Asp His Val
   1235 1240 1245
   Phe Leu Val Leu Pro Asn His Cys Leu Gly Met Ala Val Ser Ser
   1250 1255 1260
   Phe Tyr Glu Asn Tyr Glu Thr Arg Arg Tyr Cys Thr Ser Ser Glu
   1265 1270 1275
   Val Ala Ala His Tyr Cys Lys Lys Tyr Asn Ile Gln Tyr Gln Glu
   1280 1285 1290
   Asn Phe Tyr Ala Trp Ser Ala Pro Gly Val Gly Arg Phe Val Ala
   1295 1300 1305
   Ser Met Ala Ala Ser Gly Cys Ala Tyr Leu Ile Leu Leu Phe Leu
   1310 1315 1320
   Ile Glu Thr Asn Leu Leu Gln Arg Leu Arg Gly Ile Leu Cys Ala
   1325 1330 1335
   Leu Arg Arg Arg Arg Thr Leu Thr Glu Leu Tyr Thr Pro Met Pro
   1340 1345 1350
   Val Leu Pro Glu Asp Gln Asp Val Ala Asp Glu Arg Thr Arg Ile
   1355 1360 1365
   Leu Ala Pro Ser Pro Asp Ser Leu Leu His Thr Pro Leu Ile Ile
   1370 1375 1380
   Lys Glu Leu Ser Lys Val Tyr Glu Gln Arg Val Pro Leu Leu Ala
   1385 1390 1395
   Val Asp Arg Leu Ser Leu Ala Val Gln Lys Gly Glu Cys Phe Gly
   1400 1405 1410
   Leu Leu Gly Phe Asn Gly Ala Gly Lys Thr Thr Thr Phe Lys Met
   1415 1420 1425
   Leu Thr Gly Glu Glu Ser Leu Thr Ser Gly Asp Ala Phe Val Gly
   1430 1435 1440
   Gly His Arg Ile Ser Ser Asp Val Gly Lys Val Arg Gln Arg Ile
   1445 1450 1455
   Gly Tyr Cys Pro Gln Phe Asp Ala Leu Leu Asp His Met Thr Gly
   1460 1465 1470
   Arg Glu Met Leu Val Met Tyr Ala Arg Leu Arg Gly Ile Pro Glu
   1475 1480 1485
   Arg His Ile Gly Ala Cys Val Glu Asn Thr Leu Arg Gly Leu Leu
   1490 1495 1500
   Leu Glu Pro His Ala Asn Lys Leu Val Arg Thr Tyr Ser Gly Gly
   1505 1510 1515
   Asn Lys Arg Lys Leu Ser Thr Gly Ile Ala Leu Ile Gly Glu Pro
   1520 1525 1530
   Ala Val Ile Phe Leu Asp Glu Pro Ser Thr Gly Met Asp Pro Val
   1535 1540 1545
   Ala Arg Arg Leu Leu Trp Asp Thr Val Ala Arg Ala Arg Glu Ser
   1550 1555 1560
   Gly Lys Ala Ile Ile Ile Thr Ser His Ser Met Glu Glu Cys Glu
   1565 1570 1575
   Ala Leu Cys Thr Arg Leu Ala Ile Met Val Gln Gly Gln Phe Lys
   1580 1585 1590
   Cys Leu Gly Ser Pro Gln His Leu Lys Ser Lys Phe Gly Ser Gly
   1595 1600 1605
   Tyr Ser Leu Arg Ala Lys Val Gln Ser Glu Gly Gln Gln Glu Ala
   1610 1615 1620
   Leu Glu Glu Phe Lys Ala Phe Val Asp Leu Thr Phe Pro Gly Ser
   1625 1630 1635
   Val Leu Glu Asp Glu His Gln Gly Met Val His Tyr His Leu Pro
   1640 1645 1650
   Gly Arg Asp Leu Ser Trp Ala Lys Val Phe Gly Ile Leu Glu Lys
   1655 1660 1665
   Ala Lys Glu Lys Tyr Gly Val Asp Asp Tyr Ser Val Ser Gln Ile
   1670 1675 1680
   Ser Leu Glu Gln Val Phe Leu Ser Phe Ala His Leu Gln Pro Pro
   1685 1690 1695
   Thr Ala Glu Glu Gly Arg
   1700

## Description

### BACKGROUND OF THE INVENTION

Macular degeneration affects approximately 1.7 million individuals in the U.S. and is the most common cause of acquired visual impairment in those over the age of 65. Stargardt disease (STGD; McKusick Mendelian Inheritance (MIM) #248200) is arguably the most common hereditary recessive macular dystrophy and is characterized by juvenile to young adult onset, central visual impairment, progressive bilateral atrophy of the macular retinal pigment epithelium (RPE) and neuroepithelium, and the frequent appearance of orange-yellow flecks distributed around the macula and/or the midretinal periphery (Stargardt, 1909; Anderson *et al*., 1995). A clinically similar retinal disorder (Fundus Flavimaculatus, FFM, Franceschetti, 1963) often displays later age of onset and slower progression (Fishman, 1976; Noble and Carr, 1979). From linkage analysis, it has been concluded that STGD and FFM are most likely allelic autosomal recessive disorders with slightly different clinical manifestations caused by mutation(s) of a gene at chromosome 1p13-p21 (Gerber *et al.,* 1995; Anderson *et al.,* 1995). The STGD gene has been localized to a 4 cM region flanked by the recombinant markers *DIS435* and *DIS236* and a complete yeast artificial chromosome (YAC) contig of the region has been constructed (Anderson *et al.,* 1995). Recently, the location of the STGD/FFM locus on human chromosome 1p has been refined to a 2 cM interval between polymorphic markers *D1S406* and *DIS236* by genetic linkage analysis in an independent set of STGD families (Hoyng *et al.,* 1996). Autosomal dominant disorders with somewhat similar clinical phenotypes to STGD, identified in single large North American pedigrees, have been mapped to chromosome 13q34 (STGD2; MIM #153900; Zhang *et al.,* 1994) and to chromosome 6q11-q14 (STGD3; MIM #600110; Stone *et al.,* 1994), although these conditions are not characterized by the pathognomonic dark choroid observed by fluorescein angiography (Gass, 1987).

Members of the superfamily of mammalian ATP binding cassette (ABC) transporters are being considered as possible candidates for human disease phenotypes. The ABC superfamily includes genes whose products are transmembrane proteins involved in energy-dependent transport of a wide spectrum of substrates across membranes (Childs and Ling, 1994; Dean and Allikmets, 1995). Many disease-causing members of this superfamily result in defects in the transport of specific substrates (CFTR, Riordan *et al.,* 1989; ALD, Mosser *et al.,* 1993; SUR, Thomas *et al*., 1995; PMP70, Shimozawa *et al.,* 1992; TAP2, de la Salle *et al.,* 1994). In eukaryotes, ABC genes encode typically four domains that include two conserved ATP-binding domains (ATP) and two domains with multiple transmembrane (TM) segments (Hyde *et al.* 1990). The ATP-binding domains of ABC genes contain motifs of characteristic conserved residues (Walker A and B motifs) spaced by 90-120 amino acids. Both this conserved spacing and the "Signature" or "C" motif just upstream of the Walker B site distinguish members of the ABC superfamily from other ATP-binding proteins (Hyde *et al*., 1990; Michaelis and Berkower, 1995). These features have allowed the isolation of new ABC genes by hybridization, degenerate PCR, and inspection of DNA sequence databases (Allikmets *et al.,* 1993, 1995; Dean *et al.,* 1994; Luciani *et al.,* 1994).

The characterization of twenty-one new members of the ABC superfamily may permit characterization and functions assigned to these genes by determining their map locations and their patterns of expression (Allikmets *et al*., 1996). That many known ABC genes are involved in inherited human diseases suggests that some of these new loci will also encode proteins mutated in specific genetic disorders. Despite regionally localizing a gene by mapping, the determination of the precise localization and sequence of one gene nonetheless requires choosing the certain gene from about 250 genes, four to about five million base pairs, from within the regionally localized chromosomal site.

While advancements have been made as described above, mutations in retina-specific ABC transporter (ABCR) in patients with recessive macular dystrophy STGD/FFM have not yet been identified to Applicant's knowledge. That ABCR expression is limited to photoreceptors, as determined by the present invention, provides evidence as to why ABCR has not yet been sequenced. Further, the ABC1 subfamily of ABC transporters is not represented by any homolog in yeast (Michaelis and Berkower, 1995), suggesting that these genes evolved to perform specialized functions in multicellular organisms, which also lends support to why the ABCR gene has been difficult to identify. Unlike ABC genes in bacteria, the homologous genes in higher eukaryotes are much less well studied. The fact that prokaryotes contain a large number of ABC genes suggests that many mammalian members of the superfamily remain uncharacterized. The task of studying eukaryote ABC genes is more difficult because of the significantly higher complexity of eukaryotic systems and the apparent difference in function of even highly homologous genes. While ABC proteins are the principal transporters of a number of diverse compounds in bacterial cells, in contrast, eukaryotes have evolved other mechanisms for the transport of many amino acids and sugars. Eukaryotes have other reasons to diversify the role of ABC genes, for example, performing such functions as ion transport, toxin elimination, and secretion of signaling molecules.

Accordingly, there remains a need for the identification of the sequence of the gene, which in mutated forms is associated with retinal and/or macular degenerative diseases, including Stargardt Disease and Fundus Flavimaculatus, for example, in order to provide enhanced diagnoses and improved prognoses and interventional therapies for individuals affected with such diseases.
The following documents relate to a photoreceptor cell-specific ATP-binding transporter gene (ABCR) and mutations thereof, and its role in Stargardt macular dystrophy:
Allikmets R, *et al*, Science, US, vol. 277, 19 September 1997, pages 1805-1807;
Allikmets R *et al*: Nature Genetics, vol. 15, no. 3, March 1997 (1997-03), pages 236-246;
Gerber S *et al*: Genomics, vol. 48, 1998, pages 139-142;
Azarian S m *et al*: FEBS Letters, Elsevier Science Pubilshers, Amsterdam, NL vol. 409. no. 2, 9 June 1997 pages 247-252;
Nasonkin Igor *et al:* Human Genetics, vol. 102, no. 1, January 1998 (1998-01); and
Sun H. *et al*., Nature Genetics, 17 September 1997, Vol. 17, pages 15-16;

### SUMMARY OF THE INVENTION

The present invention provides sequences encoding an ATP binding cassette transporter. The invention provides nucleic acid sequences according an ATP binding cassette protein according to any one of claims 1 to 6. Nucleic acid sequences, including SEQ ID NO: 1 which is a genomic sequence, and SEQ ID NOS: 2 and 5 which are cDNA sequences, are sequences to which the present invention is directed.

A further aspect of the present invention provides ATP binding cassette transporter polypeptides and/or proteins according to claim 13. SEQ ID NOS: 3 and 6 are novel polypeptides of the invention produced from nucleotide sequences encoding the ATP binding cassette transporter. Also within the scope of the present invention is a purified ATP binding cassette transporter.

The present invention also provides an expression vector comprising a nucleic acid sequence encoding an ATP binding cassette transporter, a transformed host cell capable of expressing a nucleic acid sequence encoding an ATP binding cassette transporter, a cell culture capable of expressing an ATP binding cassette transporter, and a protein preparation comprising an ATP binding cassette transporter.

The present invention is also directed to a method of screening for an agent that modifies ATP binding cassette transporter comprising combining purified ATP binding cassette transporter with an agent suspected of modifying ATP binding cassette transporter and observing a change in at least one characteristic associated with ATP binding cassette transporter. The present invention provides methods of identifying an agent that inhibits macular degeneration comprising combining purified ATP binding cassette transporter from a patient suspected of having macular degeneration and an agent suspected interacting with the ATP binding cassette transporter and observing an inhibition in at least one of the characteristics of diseases associated with the ATP binding cassette transporter. In addition, the present invention provides for methods of identifying an agent that induces onset of at least one characteristic associated with ATP binding cassette transporter comprising combining purified wild-type ATP binding cassette transporter with an agent suspected of inducing a macular degenerative disease and observing the onset of a characteristic associated with macular degeneration.

### BRIEF DESCRIPTION OF THE FIGURES

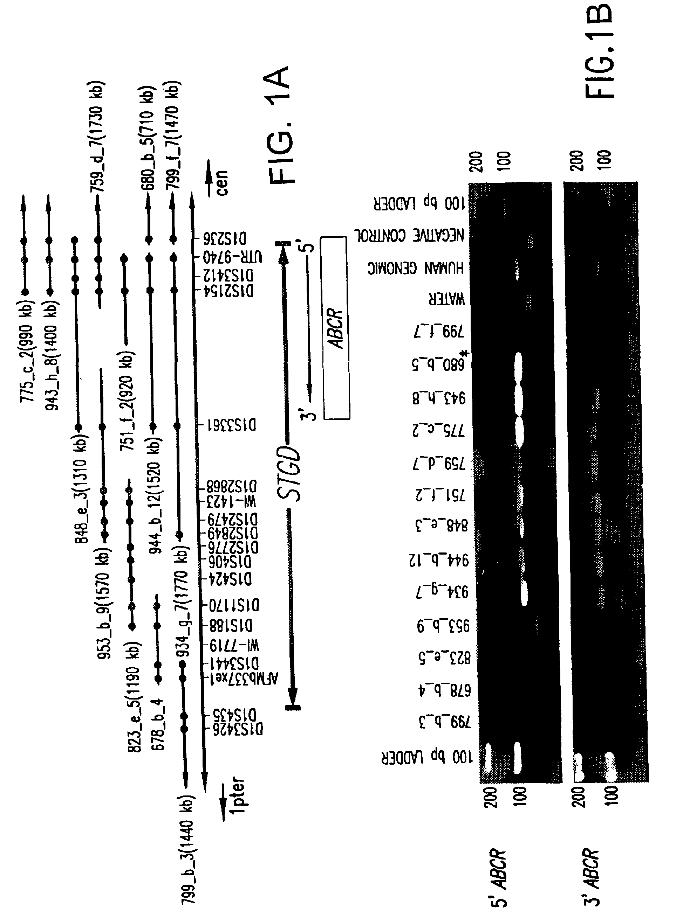

**Figure 1A and 1B** displays the ABCR gene and amplification products. **Figure 1A** displays a physical map of the *ABCR* gene. Mega-YAC clones from the CEPH mega-YAC genomic library (Bellane-Chantelot *et al.,* 1992) encompassing the 4cM critical region for STGD are represented by horizontal bars with shaded circles indicating confirmed positives for STSs by landmark mapping. The individual STS markers and their physical order are shown below the YACs with arrows indicating the centromeric (cen) and telomeric (lpter) direction (Anderson et al., 1995). The horizontal double head arrow labeled STGD indicates the refined genetic interval delineated by historical recombinants (Anderson et al., 1995). Figure 1B displays the results of agarose gel electrophoresis of PCR amplification products with primers from the 5' (GGTCTTCGTGTGTGGTCATT, SEQ ID NO: 114, GGTCCAGTTCTTCCAGAG, SEQ ID NO: 115, labeled 5' ABCR) or 3' (ATCCTCTGACTCAGCAATCACA, SEQIDNO:116, TUCAATTACAAATGCAATGG, SEQ ID NO: 117, labeled 3' ABCR) regions of ABCR on the 13 different YAC DNA templates indicated as diagonals above the gel. The asterisk denotes that YAC 680~b~5 was positive for the 5' ABCR PCR but negative for the 3' ABCR PCR. These data suggest the ABCR gene maps within the interval delineated by markers D1 S3361 - D1S236 and is transcribed toward the telomere, as depicted by the open horizontal box.

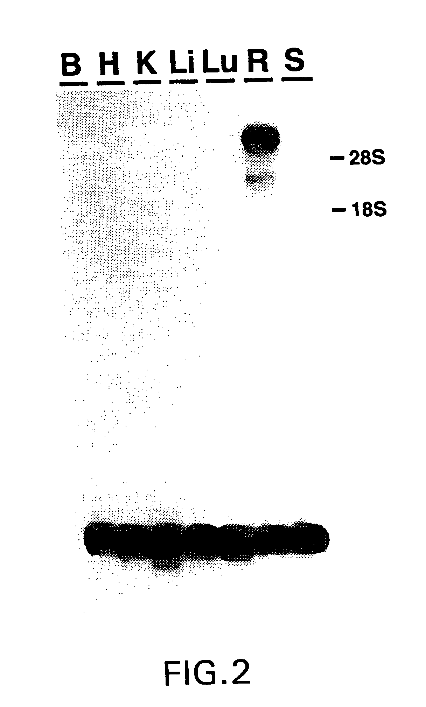

Figure 2 exhibits the size and tissue distribution of ABCR transcripts in the adult rat. A blot of total RNA from the indicated tissues was hybridized with a 1.6 kb mouse Abcr probe (top) and a ribosomal protein S26 probe (bottom; Kuwano et al., 1985). The ABCR probe revealed a predominant transcript of approximately 8 kb that is found in retina only. The mobility of the 28S and 18S ribosomal RNAs are indicated at the right. B, brain; H, heart; K, kidney; Li, liver; Lu, lung; R, retina; S, spleen.

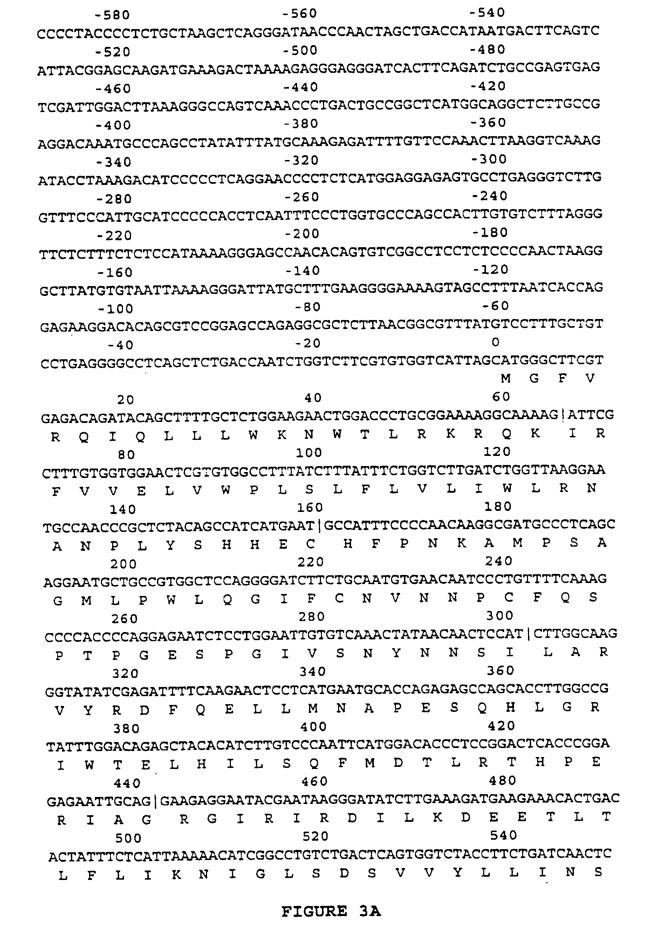

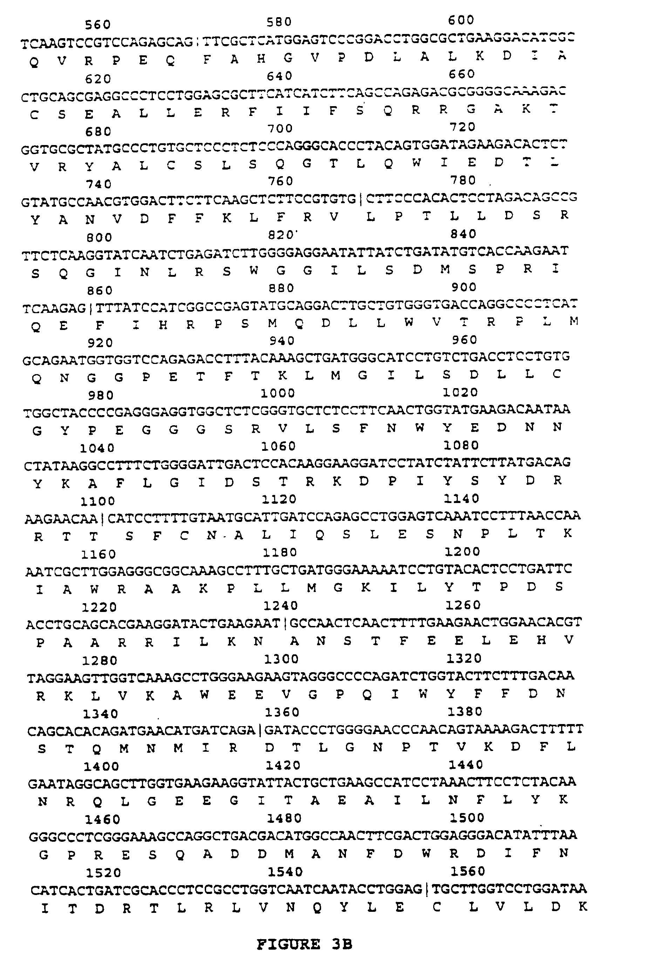

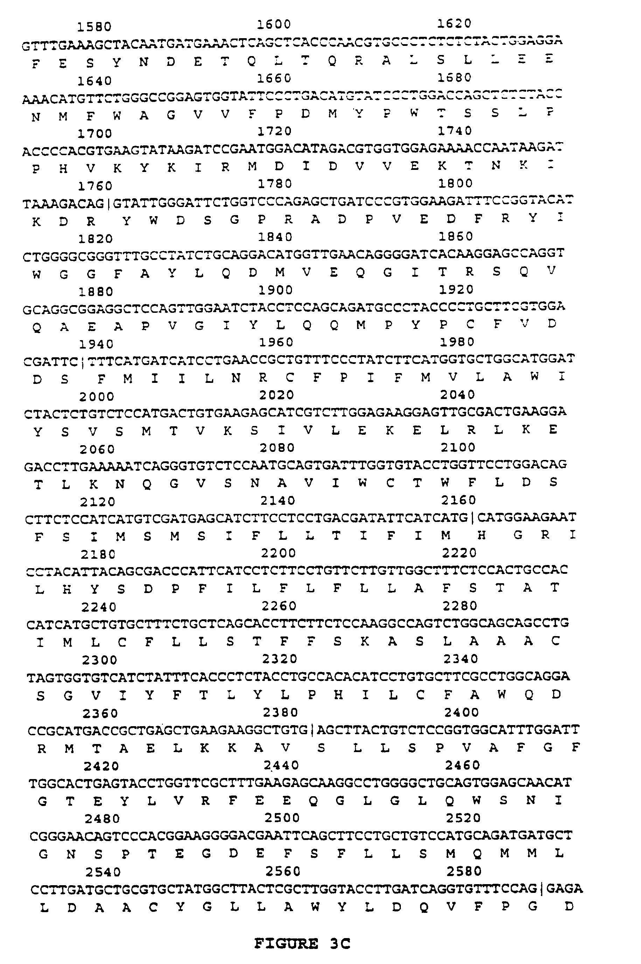

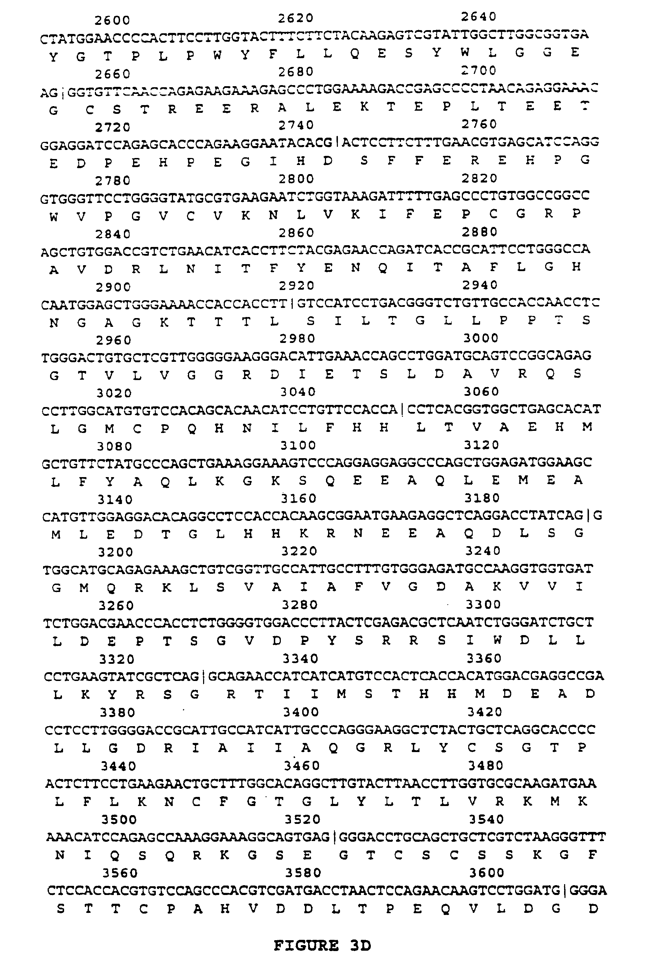

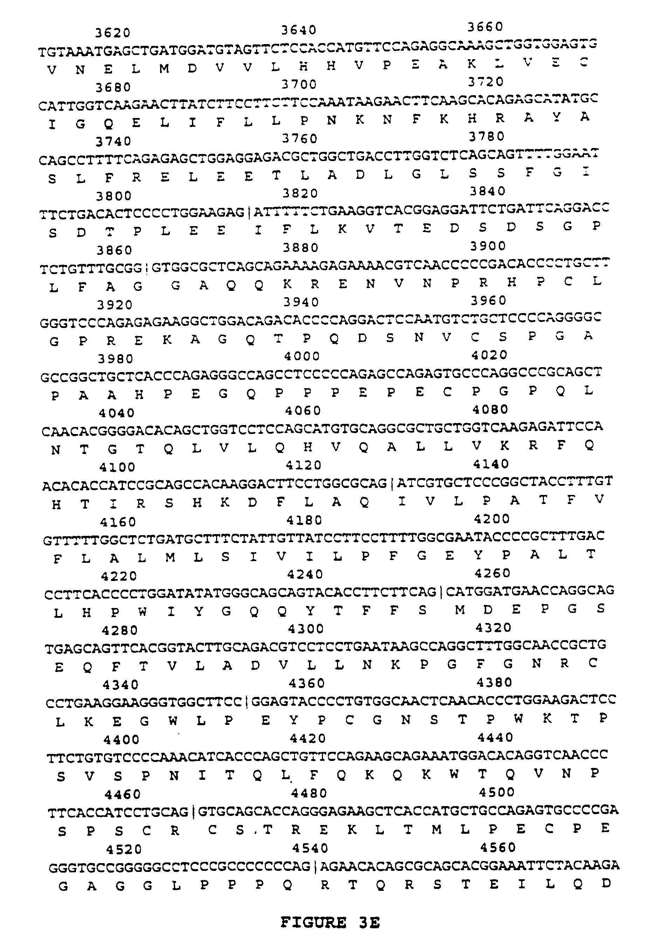

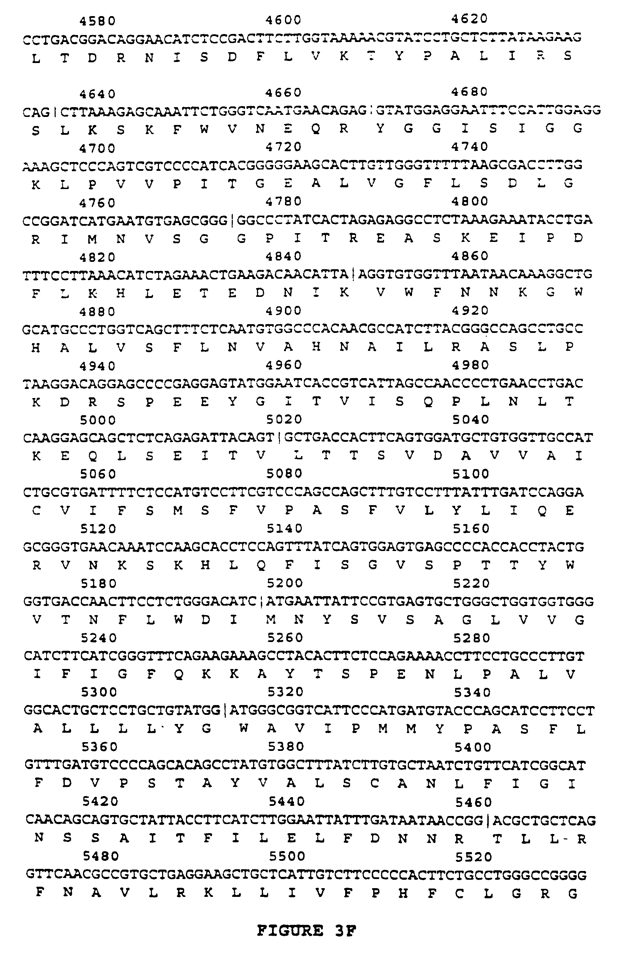

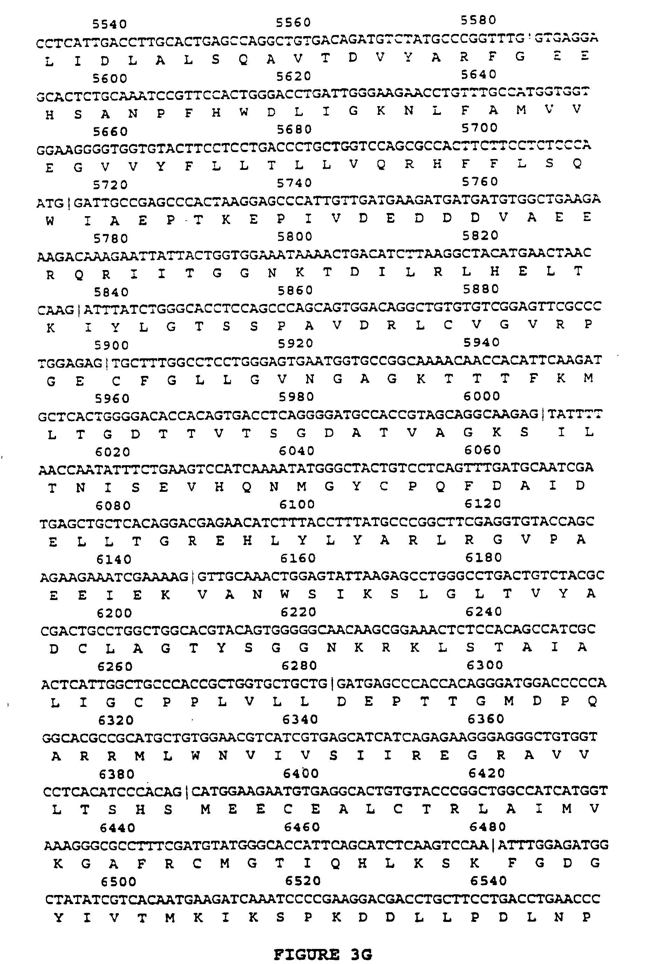

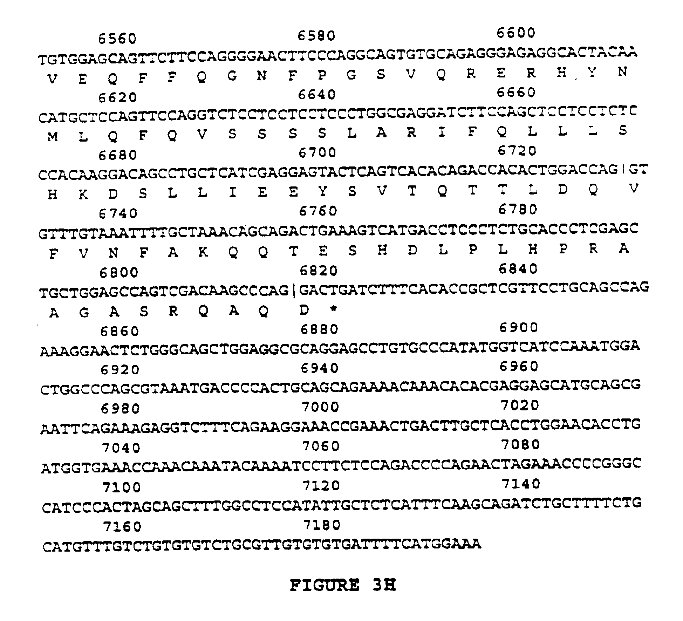

Figure 3 shows the sequence of the ABCR coding region within the genomic ABCR sequence, SEQ ID NO: 1. The sequence of the ABCR cDNA, SEQ ID NO: 2, is shown with the predicted protein sequence, SEQ ID NO: 3, in one-letter amino acid code below. The location of splice sites is shown by the symbol |.

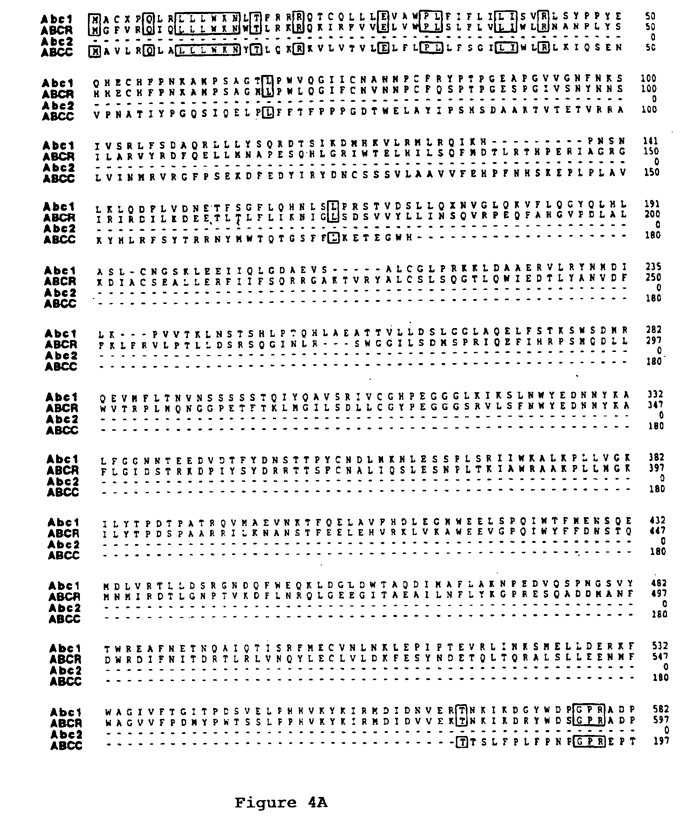

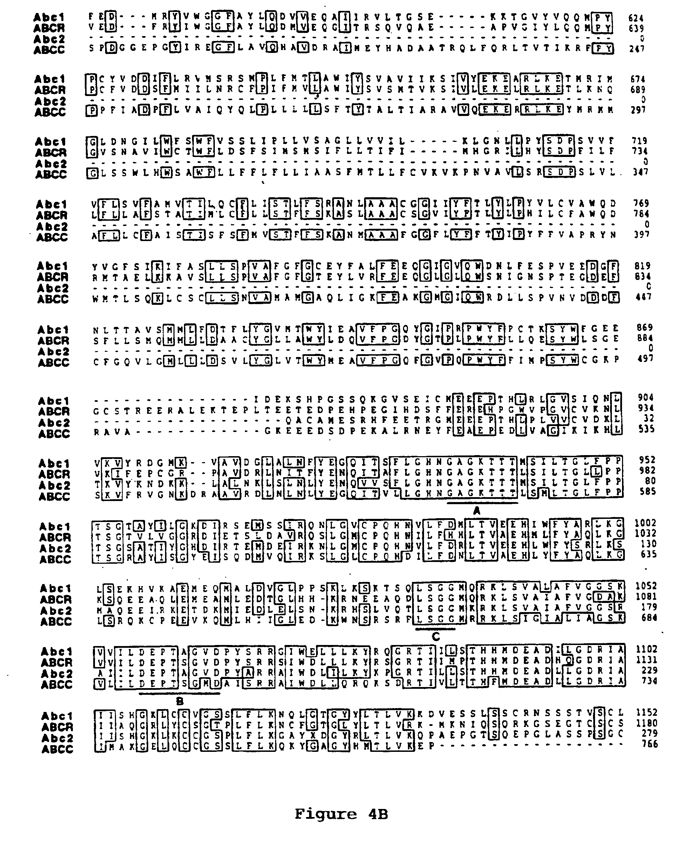

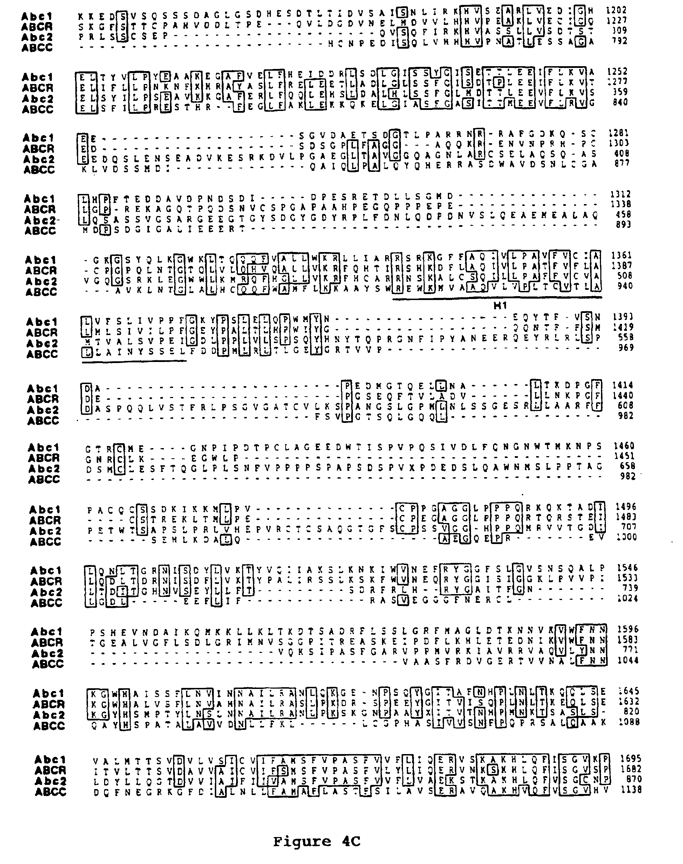

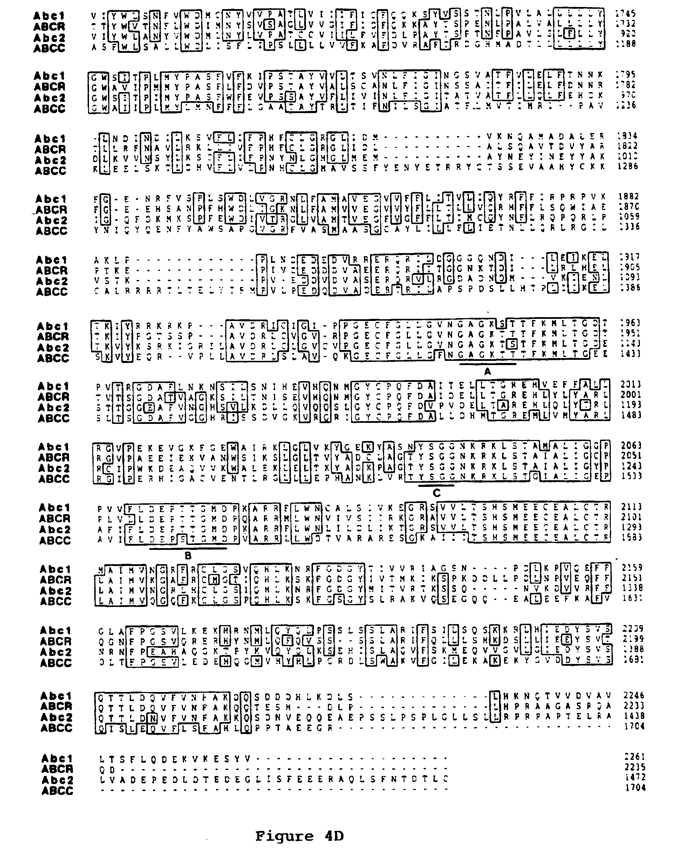

Figure 4 displays the alignment of the ABCR protein, SEQ ID NO: 3, with other members of the ABC 1 subfamily. The deduced amino acid sequence of ABCR is shown aligned to known human and mouse proteins that are members of the same subfamily. Abc1, mouse Abc1 (SEQ ID NO: 118), Abc2, mouse Abc2 (SEQ ID NO:119), and ABCC, human ABC gene (SEQ ID NO:120). The Walker A and B motifs and the Signature motif C are designated by underlining and the letters A, B, and C, respectively.

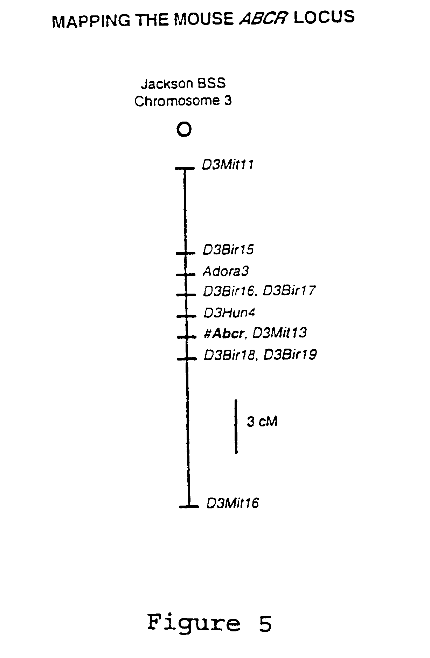

Figure 5 exhibits the location of Abcr from a Jackson BSS Backcross showing a portion of mouse chromosome 3. The map is depicted with the centromere toward the top. A 3 cM scale bar is also shown. Loci mapping to the same position are listed in alphabetical order.

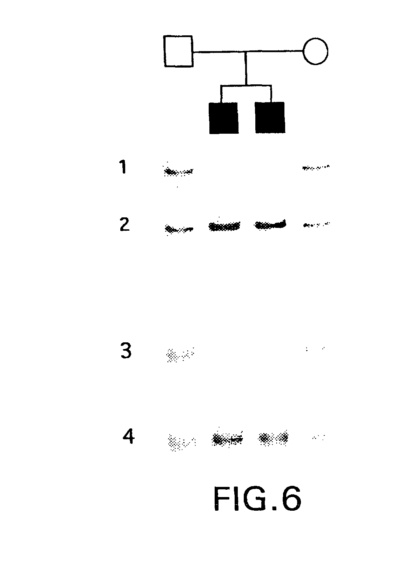

**Figure 6** shows the segregation of SSCP variants in exon 49 of the ABCR gene in kindred AR293. Sequence analysis of SSCP bands revealed the existence of wild-type sequence (bands 1 and 3) and mutant sequence (bands 2 and 4). DNA sequencing revealed a 15 base pair deletion, while the affected children (lanes 2 and 3) are homozygous. Haplotype analysis demonstrated homozygosity at the STGD locus in the two affected individuals.

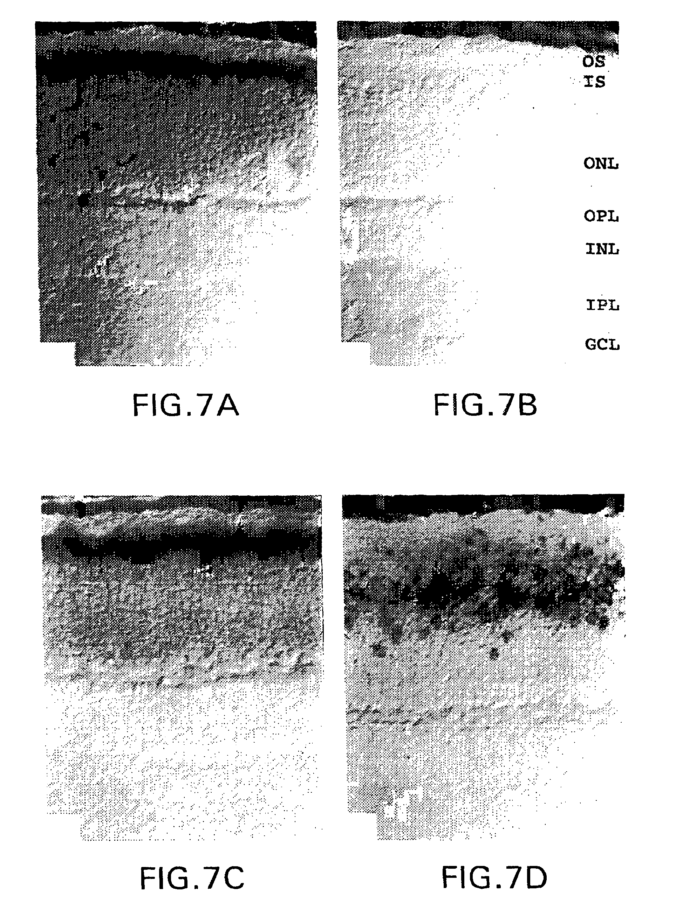

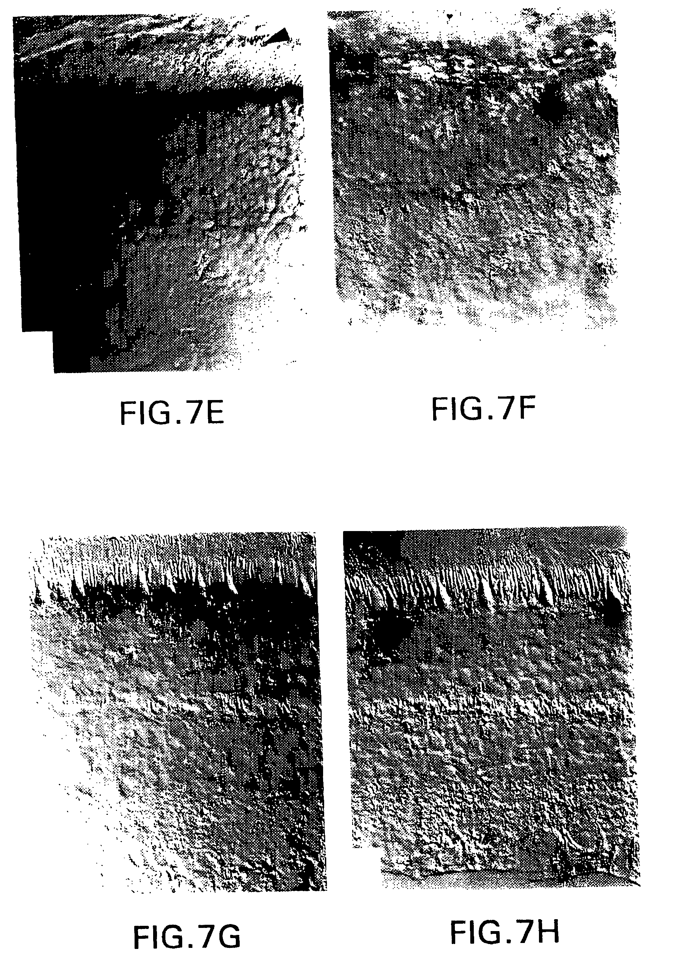

**Figure 7A-H** shows the localization of *ABCR* transcripts to photoreceptor cells. *In situ* hybridization was performed with digoxygenin-labeled riboprobes and visualized using an alkaline phosphatase conjugated anti-digoxygenin antibody. Figure 7A-D displays hybridization results of retina and choroid from a pigmented mouse (C57B16); Figure 7E and 7F shows hybridization results of retina and choroid from an albino rat; and Figure 7G and 7H exhibits hybridization results of retina from a macaque monkey. Figure 7A, 7E, and 7G display results from a mouse *abcr* antisense probe; Figure 7B exhibit results from a mouse *abcr* sense probe; Figure 7C shows results from a macaque rhodopsin antisense probe; and Figure 7D, 7F, and 7H display results from a mouse blue cone pigment antisense probe. *ABCR* transcripts are localized to the inner segments of the photoreceptor cell layer, a pattern that matches the distribution of rhodopsin transcripts but is distinct from the distribution of cone visual pigment transcripts. Hybridization is not observed in the RPE or choroid, as seen most clearly in the albino rat eye (arrowhead in Figure 7E). The retinal layers indicated in Figure 7B are: OS, outer segments; IS, inner segments; ONL, outer nuclear layer; OPL, outer plexiform layer; INL, inner nuclear layer; IPL, inner plexiform layer; GCL, ganglion cell layer.

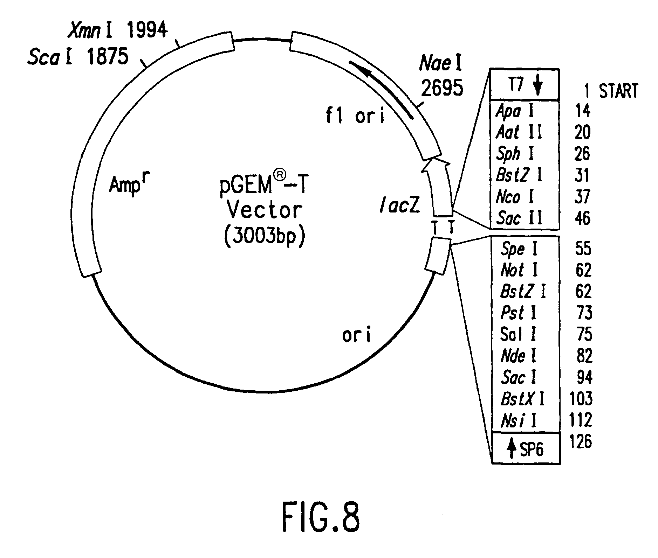

**Figure 8** provides a pGEM®-T Vector map.

### DETAILED DESCRIPTION OF THE INVENTION

The present invention is directed to the nucleic acid and protein sequences encoding ATP binding cassette transporter. The ATP binding cassette transporter of the present invention is retina specific ATP binding cassette transporter (ABCR); more particularly, ABCR may be isolated from retinal cells, preferably photoreceptor cells. The invention provides nucleic acid sequences encoding ABCR according to claims 1 to 6. The present invention provides nucleotide sequences of *ABCR* including genomic sequences, SEQ ID NO: 1, and cDNA sequences SEQ ID NO: 2 and 5. Novel polypeptide sequences, SEQ ID NOS: 3 and 6, for ABCR, are the translated products of SEQ ID NOS: 2 and 5, respectively, and are also included in the present invention.

SEQ ID NO: 1 provides the human genomic DNA sequence of ABCR. SEQ ID NOS: 2 and 5 provide wild-type cDNA sequences of human ABCR, which result in translated products SEQ ID NOS: 3 and 6, respectively. While not intending to be bound by any particular theory or theories of operation, it is believed that SEQ ID NOS: 2 and 5 are isoforms of ABCR cDNA. The difference between SEQ ID NOS: 2 and 5 may be accounted for by an additional sequence in SEQ ID NO: 2 which is added between bases 4352 and 4353 of SEQ ID NO: 5. This difference is thought to arise from alternative splicing of the nascent transcript of ABCR. in which an alternative exon 30, SEQ ID NO: 4, is excluded. This alternative exon encodes an additional 38 amino acids, SEQ ID NO: 11.

Nucleic acids within in the scope of the present invention include cDNA, RNA, genomic DNA, fragments or portions within the sequences, antisense oligonucleotides. Sequences encoding the ABCR also include amino acid, polypeptide, and protein sequences. Variations in the nucleic acid and polypeptide sequences of the present invention are within the scope of the present invention and include N terminal and C terminal extensions, transcription and translation modifications, and modifications in the cDNA sequence to facilitate and improve transcription and translation efficiency. In addition, changes within the wild-type sequences identified herein which changed sequence retains substantially the same wild-type activity, such that the changed sequences are substantially similar to the ABCR sequences identified, are also considered within the scope of the present invention. Mismatches, insertions, and deletions which permit substantial similarity to the ABCR sequences, such as similarity in residues in hydrophobicity, hydrophilicity, basicity, and acidity, will be known to those of skill in the art once armed with the present disclosure. In addition, the isolated, or purified, sequences of the present invention may be natural, recombinant, synthetic, or a combination thereof. Wild-type activity associated with the ABCR sequences of the present invention include, *inter alia*, all or part of a sequence, or a sequence substantially similar thereto, that codes for ATP binding cassette transporter.

The genomic, SEQ ID NO: 1, and cDNA, SEQ ID NOS: 2 and 5, sequences are identified in Figure 3 A-H and encode ABCR, certain mutations of which are responsible for the class of retinal disorders known as retinal or macular degenerations. Macular degeneration is characterized by macular dystrophy, various alterations of the peripheral retina, central visual impairment, progressive bilateral atrophy of the macular retinal pigment epithelium (RPE) and neuroepithelium, frequent appearance of orange-yellow flecks distributed around the macula and/or the midretinal periphery, and subretinal deposition of lipofuscin-like material. Retinal and macular degenerative diseases include and are not limited to Stargardt Disease, Fundus Flavimaculatus, age-related macular degeneration, and may include disorders variously called retinitis pigmentosa, combined rod and cone dystrophies, cone dystrophies and degenerations, pattern dystrophy, bull's eye maculopathies, and various other retinal degenerative disorders, some induced by drugs, toxins, environmental influences, and the like. Stargardt Disease is an autosomal recessive retinal disorder characterized by juvenile to adult-onset macular and retinal dystrophy. Fundus Flavimaculatus often displays later age of onset and slower progression. Some environmental insults and drug toxicities may create similar retinal degenerations. Linkage analysis reveals that Stargardt Disease and Fundus Flavimaculatus may be allelic autosomal recessive disorders with slightly different clinical manifestations. The identification of the ABCR gene suggests that different mutations within ABCR may be responsible for these clinical phenomena.

The present invention is also directed to a method of screening for an agent that modifies ATP binding cassette transporter comprising combining purified ATP binding cassette transporter with an agent suspected of modifying ATP binding cassette transporter and observing a change in at least one characteristic associated with ATP binding cassette transporter.

"Modify" and variations thereof include changes such as and not limited to inhibit, suppress, delay, retard, slow, suspend, obstruct, and restrict, as well as induce, encourage, provoke, and cause. Modify may also be defined as complete inhibition such that macular degeneration is arrested, stopped, or blocked. Modifications may, directly or indirectly, inhibit or substantially inhibit, macular degeneration or induce, or substantially induce, macular degeneration, under certain circumstances.

Methods of identifying an agent that inhibits macular degeneration are embodied by the present invention and comprise combining purified ATP binding cassette transporter from a patient suspected of having macular degeneration and an agent suspected of interacting with the ATP binding cassette transporter and observing an inhibition in at least one of the characteristics of diseases associated with the ATP binding cassette transporter. Accordingly, such methods serve to reduce or prevent macular degeneration, such as in human patients. In addition, the present invention provides for methods of identifying an agent that induces onset of at least one characteristic associated with ATP binding cassette transporter comprising combining purified wild-type ATP binding cassette transporter with an agent suspected of inducing a macular degenerative disease and observing the onset of a characteristic associated with macular degeneration. Thus, such methods provide methods of using laboratory animals to determine causative agents of macular degeneration. The ATP binding cassette transporter may be provided for in the methods identified herein in the form of nucleic acids, such as and not limited to SEQ ID NOS: 1, 2, and 5 or as an amino acid, SEQ ID NOS: 3 and 6, for example. Accordingly, transcription and translation inhibitors may be separately identified. Characteristics associated with macular degeneration include and are not limited to central visual impairment, progressive bilateral atrophy of the macular retinal pigment epithelium (RPE) and neuroepithelium, and the frequent appearance of orange-yellow flecks distributed around the macula and/or the midretinal periphery. Accordingly, observing one or more of the characteristics set forth above results in identification of an agent that induces macular degeneration, whereas reduction or inhibition of at least one of the characteristics results in identification of an agent that inhibits macular degeneration.

Mutational analysis of *ABCR* in Stargardt Disease families revealed thus far seventy four mutations including fifty four single amino acid substitutions, five nonsense mutations resulting in early truncation of the protein, six frame shift mutations resulting in early truncation of the protein, three in-frame deletions resulting in loss of amino acid residues from the protein, and six splice site mutations resulting in incorrect processing of the nascent RNA transcript, see Table 2. Compound heterozygotes for mutations in *ABCR* were found in forty two families. Homozygous mutations were identified in three families with consanguineous parentage. Accordingly, mutations in wild-type *ABCR* which result in activities that are not associated with wild-type *ABCR* are herein referred to as sequences which are associated with macular degeneration. Such mutations include missense mutations, deletions, insertions, substantial differences in hydrophobicity, hydrophilicity, acidity, and basicity. Characteristics which are associated with retinal or macular degeneration include and are not limited to those characteristics set forth above.

Mutations in wild-type ABCR provide a method of detecting macular degeneration. Retinal or macular degeneration may be detected by obtaining a sample comprising patient nucleic acids from a patient tissue sample; amplifying retina-specific ATP binding cassette receptor specific nucleic acids from the patient nucleic acids to produce a test fragment; obtaining a sample comprising control nucleic acids from a control tissue sample; amplifying control nucleic acids encoding wild-type retina-specific ATP binding cassette receptor to produce a control fragment; comparing the test fragment with the control fragment to detect the presence of a sequence difference in the test fragment, wherein a difference in the test fragment indicates macular degeneration. Mutations in the test fragment, including and not limited to each of the mutations identified above, may provide evidence of macular degeneration. The invention provides nucleic acid sequences encoding mutant ABCR proteins according to claims 2-6.

A purified ABCR protein is also provided by the present invention. The purified ABCR protein may have an amino acid sequence as provided by SEQ ID NOS: 3 and 6.

The present invention is directed to ABCR sequences obtained from mammals from the Order Rodentia, including and not limited to hamsters, rats, and mice; Order Logomorpha, such as rabbits; more particularly the Order Carnivora, including Felines (cats) and Canines (dogs); even more particularly the Order Artiodactyla, Bovines (cows) and Suines (pigs); and the Order Perissodactyla, including Equines (horses); and most particularly the Order Primates, Ceboids and Simoids (monkeys) and Anthropoids (humans and apes). The mammals of most preferred embodiments are humans.

Generally, the sequences of the invention may be produced in host cells transformed with an expression vector comprising a nucleic acid sequence encoding *ABCR.* The transformed cells are cultured under conditions whereby the nucleic acid sequence coding for *ABCR* is expressed. After a suitable amount of time for the protein to accumulate, the protein may be purified from the transformed cells.

A gene coding for *ABCR* may be obtained from a cDNA library. Suitable libraries can be obtained from commercial sources such as Clontech, Palo Alto, CA. Libraries may also be prepared using the following non-limiting examples: hamster insulin-secreting tumor (HIT), mouse αTC-6, and rat insulinoma (RIN) cells. Positive clones are then subjected to DNA sequencing to determine the presence of a DNA sequence coding for *ABCR.* DNA sequencing is accomplished using the chain termination method of Sanger *et al., Proc. Nat'l. Acad. Sci, US.A.,* **1977**, *74,* 5463. The DNA sequence encoding *ABCR* is then inserted into an expression vector for later expression in a host cell.

Expression vectors and host cells are selected to form an expression system capable of synthesizing ABCR. Vectors including and not limited to baculovirus vectors may be used in the present invention. Host cells suitable for use in the invention include prokaryotic and eukaryotic cells that can be transformed to stably contain and express ABCR. For example, nucleic acids coding for the recombinant protein may be expressed in prokaryotic or eukaryotic host cells, including the most commonly used bacterial host cell for the production of recombinant proteins, *E. coli*. Other microbial strains may also be used, however, such as *Bacillus subtilis*, and other enterobacteriaceae such as *Salmonella typhimurium* or *Serratia marcescens*, various species of *Pseudomonas*, or other bacterial strains.

The preferable eukaryotic system is yeast, such as *Saccharomyces cerevisiae.* Yeast artificial chromosome (YAC) systems are able to accommodate the large size of ABCR gene sequence or genomic clone. The principle of the YAC system is similar to that used in conventional cloning of DNA. Large fragments of cDNA are ligated into two "arms" of a YAC vector, and the ligation mixture is then introduced into the yeast by transformation. Each of the arms of the YAC vector carries a selectable marker as well as appropriately oriented sequences that function as telomeres in yeast. In addition, one of the two arms carries two small fragments that function as a centromere and as an origin of replication (also called an ARS element-autonomously replicating sequences). Yeast transformants that have taken up and stably maintained an artificial chromosome are identified as colonies on agar plates containing the components necessary for selection of one or both YAC arms. YAC vectors are designed to allow rapid identification of transformants that carry inserts of genomic DNA. Insertion of genomic DNA into the cloning site interrupts a suppressor tRNA gene and results in the formation of red rather than white colonies by yeast strains that carry an amber *ade*2 gene.

To clone in YAC vectors, genomic DNA from the test organism is prepared under conditions that result in relatively little shearing such that its average size is several million base pairs. The cDNA is then ligated to the arms of the YAC vector, which has been appropriately prepared to prevent self-ligation. As an alternative to partial digestion with *EcoRI,* YAC vectors may be used that will accept genomic DNA that has been digested to completion with rarely cutting restriction enzymes such as *Not*I or *Mlu*I.

In addition, insect cells, such as *Spodoptera frugiperda*; chicken cells, such as E3C/O and SL-29; mammalian cells, such as HeLa, Chinese hamster ovary cells (CHO), COS-7 or MDCK cells and the like may also be used. The foregoing list is illustrative only and is not intended in any way to limit the types of host cells suitable for expression of the nucleic acid sequences of the invention.

As used herein, expression vectors refer to any type of vector that can be manipulated to contain a nucleic acid sequence coding for *ABCR,* such as plasmid expression vectors, viral vectors, and yeast expression vectors. The selection of the expression vector is based on compatibility with the desired host cell such that expression of the nucleic acid encoding *ABCR* results. Plasmid expression vectors comprise a nucleic acid sequence of the invention operably linked with at least one expression control element such as a promoter. In general, plasmid vectors contain replicon and control sequences derived from species compatible with the host cell. To facilitate selection of plasmids containing nucleic acid sequences of the invention, plasmid vectors may also contain a selectable marker such as a gene coding for antibiotic resistance. Suitable examples include the genes coding for ampicillin, tetracycline, chloramphenicol, or kanamycin resistance.

Suitable expression vectors, promoters, enhancers, and other expression control elements are known in the art and may be found in Sambrook *et al., Molecular Cloning: A Laboratory Manual*, second edition, Cold Spring Harbor Laboratory Press, Cold Spring Harbor, NY (1989), incorporated herein by reference in its entirety.

Transformed host cells containing a DNA sequence encoding *ABCR* may then be grown in an appropriate medium for the host. The cells are then grown until product accumulation reaches desired levels at which time the cells are then harvested and the protein product purified in accordance with conventional techniques. Suitable purification methods include, but are not limited to, SDS PAGE electrophoresis, phenylboronate-agarose, reactive green 19-agarose, concanavalin A sepharose, ion exchange chromatography, affinity chromatography, electrophoresis, dialysis and other methods of purification known in the art.

Protein preparations, of purified or unpurified ABCR by host cells, are accordingly produced which comprise ABCR and other material such as host cell components and/or cell medium, depending on the degree of purification of the protein.

The invention also includes a transgenic non-human animal, including and not limited to mammals, such as and not limited to a mouse, rat, or hamster, comprising a sequence encoding ABCR, or fragment thereof that substantially retains ABCR activity, introduced into the animal or an ancestor of the animal. The sequence may be wild-type or mutant and may be introduced into the animal at the embryonic or adult stage. The sequence is incorporated into the genome of an animal such that it is chromosomally incorporated into an activated state. A transgenic non-human animal has germ cells and somatic cells that contain an ABCR sequence. Embryo cells may be transfected with the gene as it occurs naturally, and transgenic animals are selected in which the gene has integrated into the chromosome at a locus which results in activation. Other activation methods include modifying the gene or its control sequences prior to introduction into the embryo. The embryo may be transfected using a vector containing the gene.

In addition, a transgenic non-human animal may be engineered wherein *ABCR* is suppressed. For purposes of the present invention, suppression of *ABCR* includes, and is not limited to strategies which cause *ABCR* not to be expressed. Such strategies may include and are not limited to inhibition of protein synthesis, pre-mRNA processing, or DNA replication. Each of the above strategies may be accomplished by antisense inhibition of *ABCR* gene expression. Many techniques for transferring antisense sequences into cells are known to those of skill, including and not limited to microinjection, viral-mediated transfer, somatic cell transformation, transgene integration, and the like, as set forth in Pinkert, Carl, *Transgenic Animal Technology,* **1994**, Academic Press, Inc., San Diego, CA, incorporated herein by reference in its entirety.

Further, a transgenic non-human animal may be prepared such that *ABCR* is knocked out. For purposes of the present invention, a knock-out includes and is not limited to disruption or rendering null the *ABCR* gene. A knock-out may be accomplished, for example, with antisense sequences for *ABCR.* The *ABCR* gene may be knocked out by injection of an antisense sequence for all or part of the *ABCR* sequence such as an antisense sequence for all or part of SEQ ID NO: 2. Once *ABCR* has been rendered null, correlation of the *ABCR* to macular degeneration may be tested. Sequences encoding mutations affecting the *ABCR* may be inserted to test for alterations in various retinal and macular degenerations exhibited by changes in the characteristics associated with retinal and macular degeneration.

A non-human ABCR knock-out may be engineered by inserting synthetic DNA into the animal chromosome by homologous recombination. In this method, sequences flanking the target and insert DNA are identical, allowing strand exchange and crossing over to occur between the target and insert DNA. Sequences to be inserted typically include a gene for a selectable marker, such as drug resistance. Sequences to be targeted are typically coding regions of the genome, in this case part of the ABCR gene. In this process of homologous recombination, targeted sequences are replaced with insert sequences thus disrupting the targeted gene and rendering it nonfunctional. This nonfunctional gene is called a null allele of the gene.

To create the knockout mouse, a DNA construct containing the insert DNA and flanking sequences is made. This DNA construct is transfected into pluripotent embryonic stem cells competent for recombination. The identical flanking sequences align with one another, and chromosomal recombination occurs in which the targeted sequence is replaced with the insert sequence, as described in Bradley, A., Production and Analysis of Chimeric Mice, in *Teratocarcinomas and Embryonic Stem Cells - A Practical Approach,* **1987**, E. Roberson, Editor, IRC Press, pages 113-151. The stem cells are injected into an embryo, which is then implanted into a female animal and allowed to be born. The animals may contain germ cells derived from the injected stem cells, and subsequent matings may produce animals heterozygous and homozygous for the disrupted gene.

Transgenic non-human animals may also be useful for testing nucleic acid changes to identify additional mutations responsible for macular degeneration. A transgenic non-human animal may comprise a recombinant *ABCR.*

The term "purified", when used to describe the state of nucleic acid sequences of the invention, refers to nucleic acid sequences substantially free of nucleic acid not coding for *ABCR* or other materials normally associated with nucleic acid in non-recombinant cells, i.e., in its "native state."

The term "purified" or "in purified form" when used to describe the state of an ABCR nucleic acid, protein, polypeptide, or amino acid sequence, refers to sequences substantially free, to at least some degree, of cellular material or other material normally associated with it in its native state. Preferably the sequence has a purity (homogeneity) of at least about 25% to about 100%, More preferably the purity is at least about 50%, when purified in accordance with standard techniques known in the art.

In accordance with methods of the present invention, methods of detecting retinal or macular degenerations in a patient are provided comprising obtaining a patient tissue sample for testing. The tissue sample may be solid or liquid, a body fluid sample such as and not limited to blood, skin, serum, saliva, sputum, mucus, bone marrow, urine, lymph, and a tear; and feces. In addition, a tissue sample from amniotic fluid or chorion may be provided for the detection of retinal or macular degeneration in utero in accordance with the present invention.

A test fragment is defined herein as an amplified sample comprising *ABCR-*specific nucleic acids from a patient suspected of having retinal or macular degeneration. A control fragment is an amplified sample comprising normal or wild-type *ABCR*-specific nucleic acids from an individual not suspected of having retinal or macular degeneration.

The method of amplifying nucleic acids may be the polymerase chain reaction using a pair of primers wherein at least one primer within the pair is selected from the group consisting of SEQ ID NOS: 40-61, 64, 65, 78, 79, 98-105, 108, and 109. When the polymerase chain reaction is the amplification method of choice, a pair of primers may be used such that one primer of the pair is selected from the group consisting of SEQ ID NOS: 40-61, 64, 65, 78, 79, 98-105, 108, and 109.

Nucleic acids, such as DNA (such as and not limited to genomic DNA and cDNA) and/or RNA (such as and not limited to mRNA), are obtained from the patient sample. Preferably RNA is obtained.

Nucleic acid extraction is followed by amplification of the same by any technique known in the art. The amplification step includes the use of at least one primer sequence which is complementary to a portion of *ABCR*-specific expressed nucleic acids or sequences on flanking intronic genomic sequences in order to amplify exon or coding sequences. Primer sequences useful in the amplification methods include and are not limited to SEQ ID NOS: 40-61, 64, 65, 78, 79, 98-105, 108, and 109, which may be used in the amplification methods. Any primer sequence of about 10 nucleotides to about 35 nucleotides, more preferably about 15 nucleotides to about 30 nucleotides, even more preferably about 17 nucleotides to about 25 nucleotides may be useful in the amplification step of the methods of the present invention. In addition, mismatches within the sequences identified above, which achieve the methods of the invention, such that the mismatched sequences are substantially complementary and thus hybridizable to the sequence sought to be identified, are also considered within the scope of the disclosure. Mismatches which permit substantial similarity to 40-61, 64, 65, 78, 79, 98-105, 108 and 109 12-113, such as and not limited to sequences with similar hydrophobicity, hydrophilicity, basicity, and acidity, will be known to those of skill in the art once armed with the present disclosure. The primers may also be unmodified or modified. Primers may be prepared by any method known in the art such as by standard phosphoramidite chemistry. See Sambrook *et al*., *supra*.

The method of amplifying nucleic acids may be the polymerase chain reaction using a pair of primers wherein at least one primer within the pair is selected from the group consisting of 40-61, 64, 65, 78, 79, 98-105, 108 and 109. When the polymerase chain reaction is the amplification method of choice, a pair of primers may be used such that one primer of the pair is selected from the group consisting of SEQ ID NOS: 40-61, 64, 65, 78, 79, 98-105, 108 and 109.

When an amplification method includes the use of two primers, a first primer and a second primer, such as in the polymerase chain reaction, one of the first primer or second primer may be selected from the group consisting of SEQ ID NOS: 40-61, 64, 65, 78, 79, 98-105, 108 and 109. Any primer pairs which copy and amplify nucleic acids between the pairs pointed toward each other and which are specific for *ABCR* may be used in accordance with the methods of the present invention.

A number of template dependent processes are available to amplify the target sequences of interest present in a sample. One of the best known amplification methods is the polymerase chain reaction (PCR) which is described in detail in U.S. Patents 4,683,195, 4,683,202 and 4,800,159, and in Innis *et al., PCR Protocols*, Academic Press, Inc., San Diego CA, 1990. Briefly, in PCR, two primer sequences are prepared which are complementary to regions on opposite complementary strands of the target sequence. An excess of deoxynucleoside triphosphates are added to a reaction mixture along with a DNA polymerase (e.g., *Taq* polymerase). If the target sequence is present in a sample, the primers will bind to the target and the polymerase will cause the primers to be extended along the target sequence by adding on nucleotides. By raising and lowering the temperature of the reaction mixture, the extended primers will dissociate from the target to form reaction products, excess primers will bind to the target and to the reaction products and the process is repeated. Alternatively, a reverse transcriptase PCR amplification procedure may be performed in order to quantify the amount of mRNA amplified. Polymerase chain reaction methodologies are well known in the art.

Another method for amplification is the ligase chain reaction (referred to as LCR), disclosed in EPA No. 320,308. In LCR, two complementary probe pairs are prepared, and in the presence of the target sequence, each pair will bind to opposite complementary strands of the target such that they abut. In the presence of a ligase, the two probe pairs will link to form a single unit. By temperature cycling, as in PCR, bound ligated units dissociate from the target and then serve as "target sequences" for ligation of excess probe pairs. U.S. Patent 4,883,750, describes an alternative method of amplification similar to LCR for binding probe pairs to a target sequence.

Qbeta Replicase, described in PCT Application No. PCT/US87/00880, may also be used as still another amplification method in the present invention. In this method, a replicative sequence of RNA which has a region complementary to that of a target is added to a sample in the presence of an RNA polymerase. The polymerase will copy the replicative sequence which can then be detected.

An isothermal amplification method, in which restriction endonucleases and ligases are used to achieve the amplification of target molecules that contain nucleotide 5'-[ alpha -thio]triphosphates in one strand of a restriction site (Walker, G. T., *et al., Proc. Natl. Acad, Sci. (U.S.A.)* **1992,** *89*:392-396), may also be useful in the amplification of nucleic acids in the present invention.

Strand Displacement Amplification (SDA) is another method of carrying out isothermal amplification of nucleic acids which involves multiple rounds of strand displacement and synthesis, i.e. nick translation. A similar method, called Repair Chain Reaction (RCR) is another method of amplification which may be useful in the present invention and which involves annealing several probes throughout a region targeted for amplification, followed by a repair reaction in which only two of the four bases are present. The other two bases can be added as biotinylated derivatives for easy detection. A similar approach is used in SDA.

*ABCR*-specific nucleic acids can also be detected using a cyclic probe reaction (CPR). In CPR, a probe having a 3' and 5' sequences of non-*ABCR* specific DNA and middle sequence of *ABCR* specific RNA is hybridized to DNA which is present in a sample. Upon hybridization, the reaction is treated with RNaseH, and the products of the probe identified as distinctive products, generate a signal which is released after digestion. The original template is annealed to another cycling probe and the reaction is repeated. Thus, CPR involves amplifying a signal generated by hybridization of a probe to a *ABCR*-specific expressed nucleic acid.

Still other amplification methods described in GB Application No. 2 202 328, and in PCT Application No. PCT/US89/01025, may be used in accordance with the present invention. In the former application, "modified" primers are used in a PCR like, template and enzyme dependent synthesis. The primers may be modified by labeling with a capture moiety (e.g., biotin) and/or a detector moiety (e.g., enzyme). In the latter application, an excess of labeled probes are added to a sample. In the presence of the target sequence, the probe binds and is cleaved catalytically. After cleavage, the target sequence is released intact to be bound by excess probe. Cleavage of the labeled probe signals the presence of the target sequence.

Other nucleic acid amplification procedures include transcription-based amplification systems (TAS) (Kwoh D., *et al., Proc. Natl. Acad Sci. (U.S.A.)* **1989**, *86*:1173, Gingeras T. R., *et al.,* PCT Application WO 88/10315, including nucleic acid sequence based amplification (NASBA) and 3SR. In NASBA, the nucleic acids can be prepared for amplification by standard phenol/chloroform extraction, heat denaturation of a clinical sample, treatment with lysis buffer and minispin columns for isolation of DNA and RNA or guanidinium chloride extraction of RNA. These amplification techniques involve annealing a primer which has *ABCR*-specific sequences. Following polymerization, DNA/RNA hybrids are digested with RNase H while double stranded DNA molecules are heat denatured again. In either case the single stranded DNA is made fully double stranded by addition of second *ABCR*-specific primer, followed by polymerization. The double stranded DNA molecules are then multiply transcribed by a polymerase such as T7 or SP6. In an isothermal cyclic reaction, the RNAs are reverse transcribed into double stranded DNA, and transcribed once again with a polymerase such as T7 or SP6. The resulting products, whether truncated or complete, indicate *ABCR*-specific sequences.

Davey, C., *et al*., European Patent Application Publication No. 329,822, disclose a nucleic acid amplification process involving cyclically synthesizing single-stranded RNA ("ssRNA"), ssDNA, and double-stranded DNA ("dsDNA") which may be used in accordance with the present invention. The ssRNA is a first template for a first primer oligonucleotide, which is elongated by reverse transcriptase (RNA-dependent DNA polymerase). The RNA is then removed from resulting DNA:RNA duplex by the action of ribonuclease H (RNase H, an RNase specific for RNA in a duplex with either DNA or RNA). The resultant ssDNA is a second template for a second primer, which also includes the sequences of an RNA polymerase promoter (exemplified by T7 RNA polymerase) 5' to its homology to its template. This primer is then extended by DNA polymerase (exemplified by the large "Klenow" fragment of *E*. *coli* DNA polymerase I), resulting as a double-stranded DNA ("dsDNA") molecule, having a sequence identical to that of the original RNA between the primers and having additionally, at one end, a promoter sequence. This promoter sequence can be used by the appropriate RNA polymerase to make many RNA copies of the DNA. These copies can then re-enter the cycle leading to very swift amplification. With proper choice of enzymes, this amplification can be done isothermally without addition of enzymes at each cycle. Because of the cyclical nature of this process, the starting sequence can be chosen to be in the form of either DNA or RNA.

Miller, H. I., *et al*., PCT application WO 89/06700 disclose a nucleic acid sequence amplification scheme based on the hybridization of a promoter/primer sequence to a target single-stranded DNA ("ssDNA") followed by transcription of many RNA copies of the sequence. This scheme is not cyclic; i.e. new templates are not produced from the resultant RNA transcripts. Other amplification methods include "race" disclosed by Frohman, M. A., In: *PCR Protocols: A Guide to Methods and Applications* **1990**, Academic Press, N.Y.) and "one-sided PCR" (Ohara, O., *et al., Proc. Natl. Acad. Sci. (U.S.A.)* **1989**, *86*:5673-5677).

Methods based on ligation of two (or more) oligonucleotides in the presence of nucleic acid having the sequence of the resulting "di-oligonucleotide", thereby amplifying the di-oligonucleotide (Wu, D. Y. *et al*., *Genomics* **1989**, *4*:560,) may also be used in the amplification step of the present invention.

Test fragment and control fragment may be amplified by any amplification methods known to those of skill in the art, including and not limited to the amplification methods set forth above. For purposes of the present invention, amplification of sequences encoding patient and wild-type *ABCR* includes amplification of a portion of a sequence such as and not limited to a portion of an *ABCR* sequence of SEQ ID NO: 1, such as sequence of a length of about 10 nucleotides to about 1,000 nucleotides, more preferably about 10 nucleotides to about 100 nucleotides, or having at least 10 nucleotides occurring anywhere within the SEQ ID NO: 1, where sequence differences are known to occur within *ABCR* test fragments. Thus, for example, a portion of the sequence encoding *ABCR* of a patient sample and a control sample may be amplified to detect sequence differences between these two sequences.

Following amplification of the test fragment and control fragment, comparison between the amplification products of the test fragment and control fragment is carried out. Sequence changes such as and not limited to nucleic acid transition, transversion, and restriction digest pattern alterations may be detected by comparison of the test fragment with the control fragment.

Alternatively, the presence or absence of the amplification product may be detected. The nucleic acids are fragmented into varying sizes of discrete fragments. For example, DNA fragments may be separated according to molecular weight by methods such as and not limited to electrophoresis through an agarose gel matrix. The gels are then analyzed by Southern hybridization. Briefly, DNA in the gel is transferred to a hybridization substrate or matrix such as and not limited to a nitrocellulose sheet and a nylon membrane. A labeled probe encoding an *ABCR* mutation is applied to the matrix under selected hybridization conditions so as to hybridize with complementary DNA localized on the matrix. The probe may be of a length capable of forming a stable duplex. The probe may have a size range of about 200 to about 10,000 nucleotides in length, preferably about 500 nucleotides in length, and more preferably about 2,454 nucleotides in length. Mismatches which permit substantial similarity to the probe, such as and not limited to sequences with similar hydrophobicity, hydrophilicity, basicity, and acidity, will be known to those of skill in the art once armed with the present disclosure. Various labels for visualization or detection are known to those of skill in the art, such as and not limited to fluorescent staining, ethidium bromide staining for example, avidin/biotin, radioactive labeling such as ³²P labeling, and the like. Preferably, the product, such as the PCR product, may be run on an agarose gel and visualized using a stain such as ethidium bromide. See Sambrook *et al., supra*. The matrix may then be analyzed by autoradiography to locate particular fragments which hybridize to the probe. Yet another alternative is the sequencing of the test fragment and the control fragment to identify sequence differences. Methods of nucleic acid sequencing are known to those of skill in the art, including and not limited to the methods of Maxam and Gilbert, *Proc. Natl. Acad. Sci., USA* **1977,** *74*, 560-564 and Sanger, *Proc. Natl. Acad. Sci., USA* **1977,** *74*, 5463-5467.

A pharmaceutical composition comprising all or part of a sequence for ABCR may be delivered to a patient suspected of having retinal or macular degeneration. The sequence may be an antisense sequence. The composition of the present invention may be administered alone or may generally be administered in admixture with a pharmaceutical carrier. The pharmaceutically-acceptable carrier may be selected with regard to the intended route of administration and the standard pharmaceutical practice. The dosage will be about that of the sequence alone and will be set with regard to weight, and clinical condition of the patient. The proportional ratio of active ingredient to carrier will naturally depend, *inter alia,* on the chemical nature, solubility, and stability of the sequence, as well as the dosage contemplated.

The sequences of the invention may be employed in the method of the invention singly or in combination with other compounds, including and not limited to other sequences set forth in the present invention. The method of the invention may also be used in conjunction with other treatments such as and not limited to antibodies, for example. For *in vivo* applications the amount to be administered will also depend on such factors as the age, weight, and clinical condition of the patient. The composition of the present invention may be administered by any suitable route, including as an eye drop, inoculation and injection, for example, intravenous, intraocular, oral, intraperitoneal, intramuscular, subcutaneous, topically, and by absorption through epithelial or mucocutaneous linings, for example, conjunctival, nasal, oral, vaginal, rectal and gastrointestinal.

The mode of administration of the composition may determine the sites in the organism to which the compound will be delivered. For instance, topical application may be administered in creams, ointments, gels, oils, emulsions, pastes, lotions, and the like. For parenteral administration, the composition may be used in the form of sterile aqueous or non-aqueous solution which may contain another solute, for example, sufficient salts, glucose or dextrose to make the solution isotonic. A non-aqueous solution may be comprise an oil, for example. For oral mode of administration, the present invention may be used in the form of tablets, capsules, lozenges, troches, powders, syrups, elixirs, aqueous solutions and suspension, and the like. Various disintegrants, such as starch, and lubricating agents may be used. For oral administration in capsule form, useful diluents are lactose and high molecular weight polyethylene glycols. When aqueous suspensions are required for oral use, certain sweetening and/or flavoring agents may be added.

A diagnostic kit for detecting retinal or macular degeneration comprising in one or more containers at least one primer which is complementary to an *ABCR* sequence and a means for visualizing amplified DNA is also within the scope of the present invention. Alternatively, the kit may comprise two primers. In either case, the primers may be selected from the group consisting of SEQ ID NOS: 40-61, 64, 65, 78, 79, 98-105, 108, and 109, for example. The diagnostic kit may comprise a pair of primers wherein one primer within said pair is complementary to a region of the *ABCR* gene, wherein one of said pair of primers is selected from the group consisting of SEQ ID NO: 40-61, 64, 65, 78, 79, 98-105, 108, and 109, a probe specific to the amplified product, and a means for visualizing amplified DNA, and optionally including one or more size markers, and positive and negative controls. The diagnostic kit of the present invention may comprise one or more of a fluorescent dye such as ethidium bromide stain, ³²P, and biotin, as a means for visualizing or detecting amplified DNA. Optionally the kit may include one or more size markers, positive and negative controls, restriction enzymes, and/or a probe specific to the amplified product.

The following examples are illustrative but are not meant to be limiting of the invention.

### EXAMPLES:

### Identification of the ABCR as a Candidate Gene for STGD

One of the 21 new human genes from the ABC superfamily, hereafter called *ABCR* (retina-specific ABC transporter), was identified (Allikmets *et al.* 1996) among expressed sequence tags (ESTs) obtained from 5,000 human retina cDNA clones (Wang, Y., Macke, J.P., Abella, B.S., Andreasson, K., Worley, P., Gilbert, D.J., Copeland, N.G., Jenkins, N.A., and Nathans, J. (1996)) and among ESTs obtained from human retina cDNA clones by the I.M.A.G.E. consortium (Lennon *et al.,* 1996). *ABCR* is closely related to the previously described mouse and human *ABC1* and *ABC2* genes (Luciani *et al.,* 1994; Allikmets *et al.,* 1995). To determine whether *ABCR* might cause a disease, the gene was mapped with a whole genome radiation hybrid panel (GeneBridge 4; Research Genetics, Huntsville, AL). *ABCR* mapped to the human chromosome 1p13-p21 region, close to microsatellite markers *D1S236* and *D1S188.* To define further the location of the gene, PCR primers, 3'UTR-For 5'ATCCTCTGACTCAGCAATCACA, SEQ ID NO: 7, and 3'UTR-Rev 5'TTGCAATTACAAATGCAATGG, SEQ ID NO: 8, from the putative 3' untranslated region were used to screen YACs from the previously described contig between these anonymous markers (Anderson *et al*., 1995). At least 12 YACs contain the 3' end of the *ABCR* gene, including 924_e_9, 759_d_7, 775_c_2, 782_b_4, 982_g_5, 775_b_2, 765_a_3, 751_f_2, 848_e_3, 943_h_8, 934_g_7, and 944_b_12 (Figure 1). These YACs delineate a region containing the STGD gene between markers *D1S3361* and *D1S236* (Anderson *et al*., 1995).

### Expression of the ABCR Gene

Additional support suggesting thatABCR is a candidate STGD gene came from expression studies and inspection of the EST databases.

Searches of the dbEST (Boguski *et al.,* 1993) database were performed with BLAST on the NCBI file server (Altschul *et al.,* 1990). Amino acid alignments were generated with PILEUP (Feng and Doolittle, 1987). Sequences were analyzed with programs of the Genetics Computer Group package (Devereaux *et al*., 1984) on a VAX computer.

Clones corresponding to the mouse ortholog of the human *ABCR* gene were isolated from the mouse retina cDNA library and end-sequenced. The chromosomal location of the mouse *ABCR* gene was determined on The Jackson Laboratory (Bar Harbor, ME) interspecific backcross mapping panel (C57BL/6JEi X SPRET/Ei)F1 X SPRET/Ei (Rowe *et al.,* 1994) known as Jackson BSS. Mapping was performed by SSCP analysis with the primers MABCR1F 5'ATC CAT ACC CTT CCC ACT CC, SEQ ID NO: 9, and MABCR1R 5' GCA GCA GAA GAT AAG CAC ACC, SEQ ID NO. 10. The allele pattern of the *Abcr* was compared to the 250 other loci mapped previously in the Jackson BSS cross (http://www.jax.org).

DNA fragments used as probes were purified on a 1% low-melting temperature agarose gel. The probe sequences are set forth within the genomic sequence of SEQ ID NO: 1 and Figure 3A-H. DNA was labeled directly in agarose with the Random Primed DNA Labeling Kit (Boehringer Mannheim, Indianapolis, IN) and hybridized to multiple tissue Northern blot and a Master blot (Clontech, Palo Alto, CA), according to the manufacturer's instructions. Each blot contained 2 µg of poly A⁺ RNA from various human tissues. Total RNA was isolated from adult rat tissues using the guanidinium thiocyanate method (Chomczynski and Saachi, 1987) and resolved by agarose gel electrophoresis in the presence of formaldehyde (Sambrook *et al*., 1989). Hybridization with the mouse *ABCR* probe was performed in 50% formamide, 5X SSC at 42°C, and filters were washed in 0.1X SSC at 68°C.

Hybridization of a 3' *ABCR* cDNA probe to a multiple tissue Northern blot and a MasterBlot (Clontech, Palo Alto, CA) indicated that the gene was not expressed detectably in any of the 50 non-retinal fetal and adult tissues examined, consistent with the observation that all 12 of the *ABCR* clones in the EST database originated from retinal cDNA libraries. Furthermore, screening cDNA libraries from both developing mouse eye and adult human retina with *ABCR* probes revealed an estimated at 0.1 %-1 % frequency of *ABCR* clones of all cDNA clones in the library. Hybridization of the *ABCR* probe to a Northern blot containing total RNA from rat retina and other tissues showed that the expression of this gene is uniquely retina-specific (Figure 2). The transcript size is estimated to be 8 kb.

### Sequence and Exon/Intron Structure of the ABCR cDNA

Several ESTs that were derived from retina cDNA libraries and had high similarity to the mouse *Abc1* gene were used to facilitate the assembly of most of the *ABCR* cDNA sequence. Retina cDNA clones were linked by RT-PCR, and repetitive screening of a human retina cDNA library with 3' and 5' PCR probes together with 5' RACE were used to characterize the terminal sequences of the gene.

cDNA clones containing *ABCR* sequences were obtained from a human retina cDNA library (Nathans *et al.,* 1986) and sequenced fully. Primers were designed from the sequences of cDNA clones from 5' and 3' regions of the gene and used to link the identified cDNA clones by RT-PCR with retina QUICK-Clone cDNA (Clontech, Palo Alto, CA) as a template. PCR products were cloned into pGEM®-T vector (Promega, Madison, WI). Mouse *ABCR* cDNA clones were obtained from screening a developing mouse eye cDNA library (H. Sun, A. Lanahan, and J. Nathans, unpublished). The pGEM®-T Vector is prepared by cutting pGEM®-5Zf(+) DNA with EcoR V and adding to a 3' terminal thymidine to both ends. These single 3'-T overhangs at the insertion site greatly improve the efficiency ofligation of PCR products because of the nontemplate-dependent addition of a single deoxyadenosine (A) to the 3'-ends of PCR products by many thermostable polymerases. The pGEM®-5Zf(+) Vector contains the origin of replication of the filamentous phage f1 and can be used to produce ssDNA. The plasmid also contains T7 and SP6 RNA polymerase promoters flanking a multiple cloning region within the α-peptide coding region for the enzyme β-galactosidase. Insertional inactivation of the α-peptide allows recombinant clones to be identified directly by color screening on indicator plates. cDNA clones from various regions of the *ABCR* gene were used as probes to screen a human genomic library in Lambda FIX II (#946203, Stratagene, LaJolla. CA). Overlapping phage clones were mapped by *Eco*RI and *Bam*HI digestion. A total of 6.9 kb of the *ABCR* sequence was assembled. (Figure 3 A-H) resulting in a 6540 bp (2180 amino acid) open reading frame.

Screening of a bacteriophage lambda human genomic library with cDNA probes yielded a contig that spans approximately 100 kb and contains the majority of the *ABCR* coding region. The exon/intron structure of all fifty one exons of the gene were characterized by direct sequencing of genomic and cDNA clones. Intron sizes were estimated from the sizes of PCR products using primers from adjacent exons with genomic phage clones as templates.

Primers for the cDNA sequences of the *ABCR* were designed with the PRIMER program (Lincoln *et al*., 1991). Both *ABCR* cDNA clones and genomic clones became templates for sequencing. Sequencing was performed with the Taq Dyedeoxy Terminator Cycle Sequencing kit (Applied Biosystems, Foster City, CA), according to the manufacturer's instructions. Sequencing reactions were resolved on an ABI 373A automated sequencer. Positions of introns were determined by comparison between genomic and cDNA sequences. Primers for amplification of individual exons were designed from adjacent intron sequences 20-50 bp from the splice site and are set forth in Table 1.

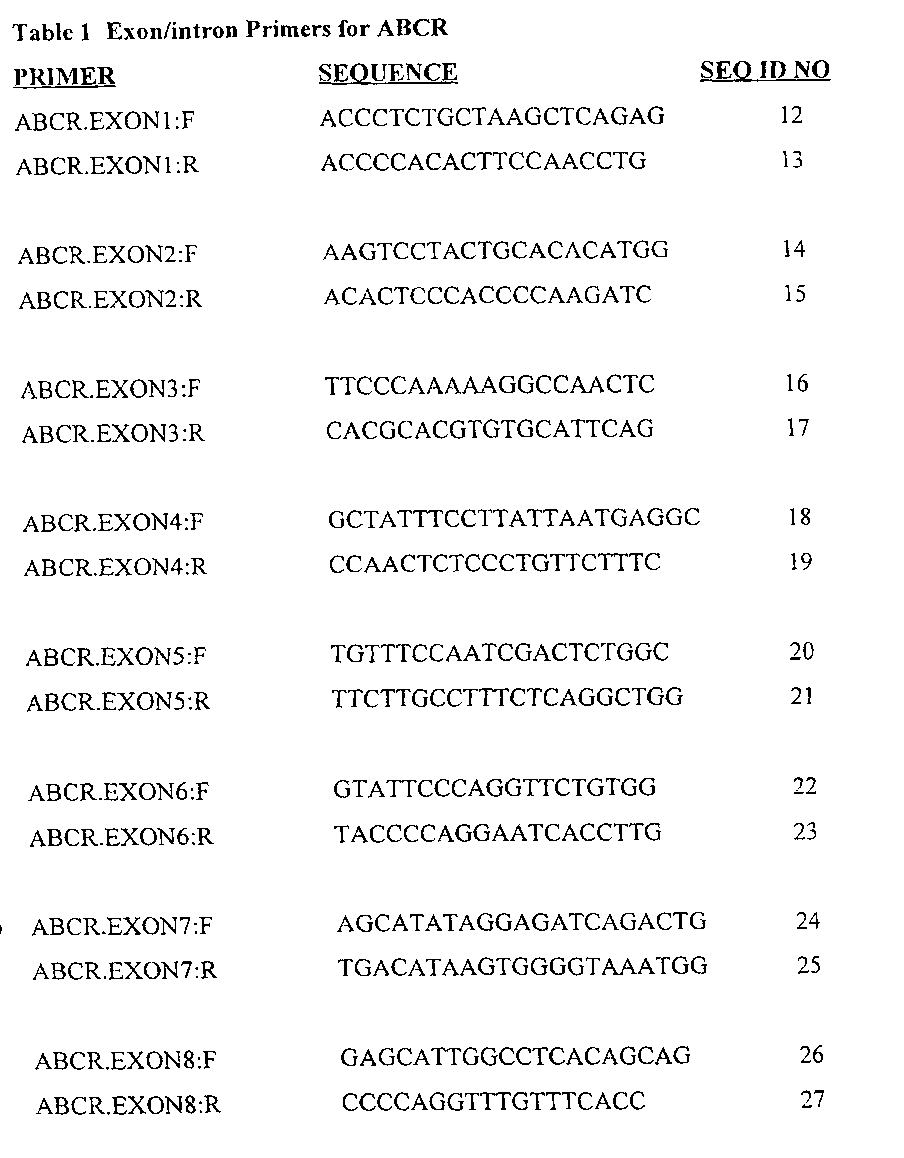

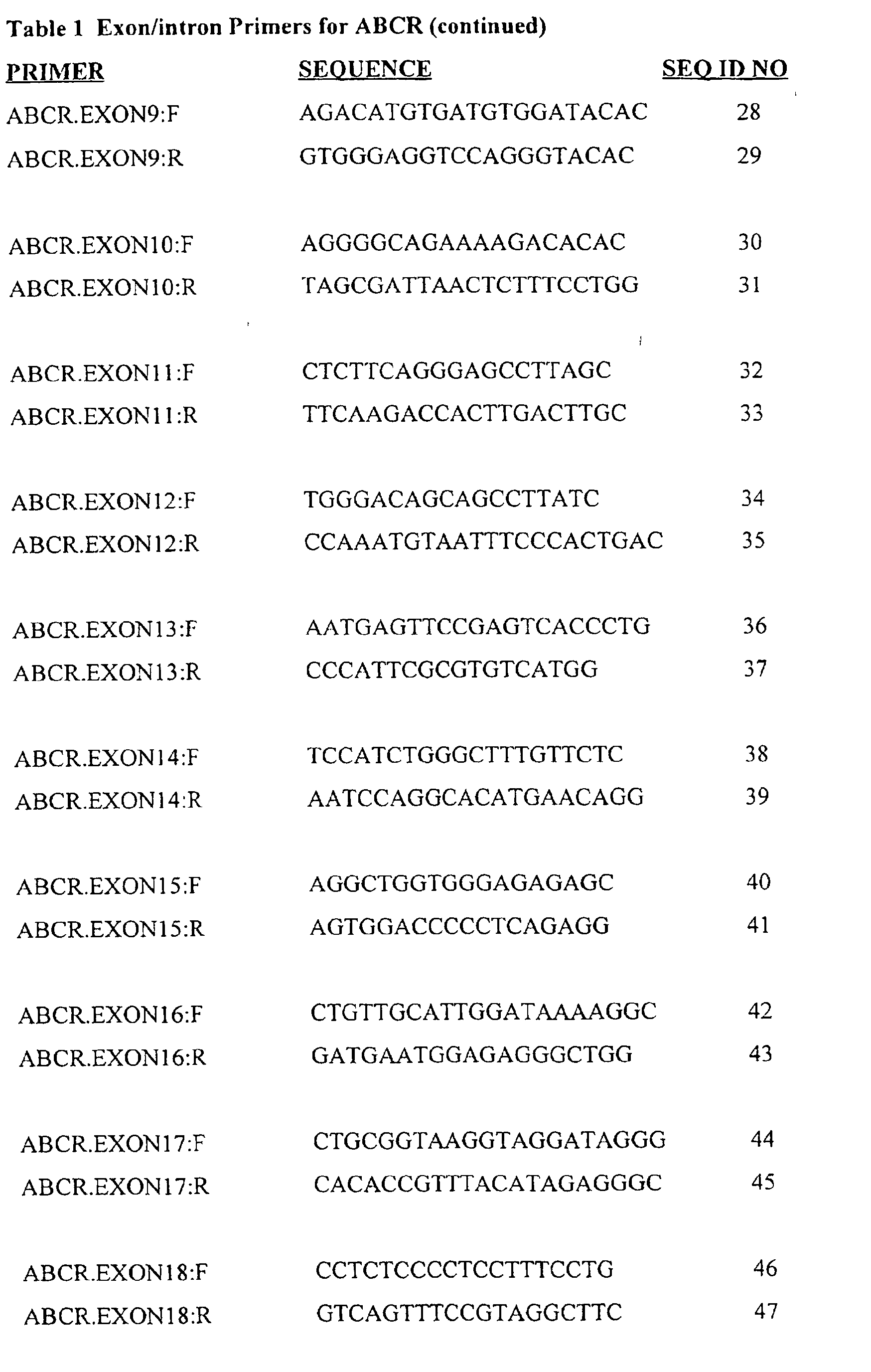

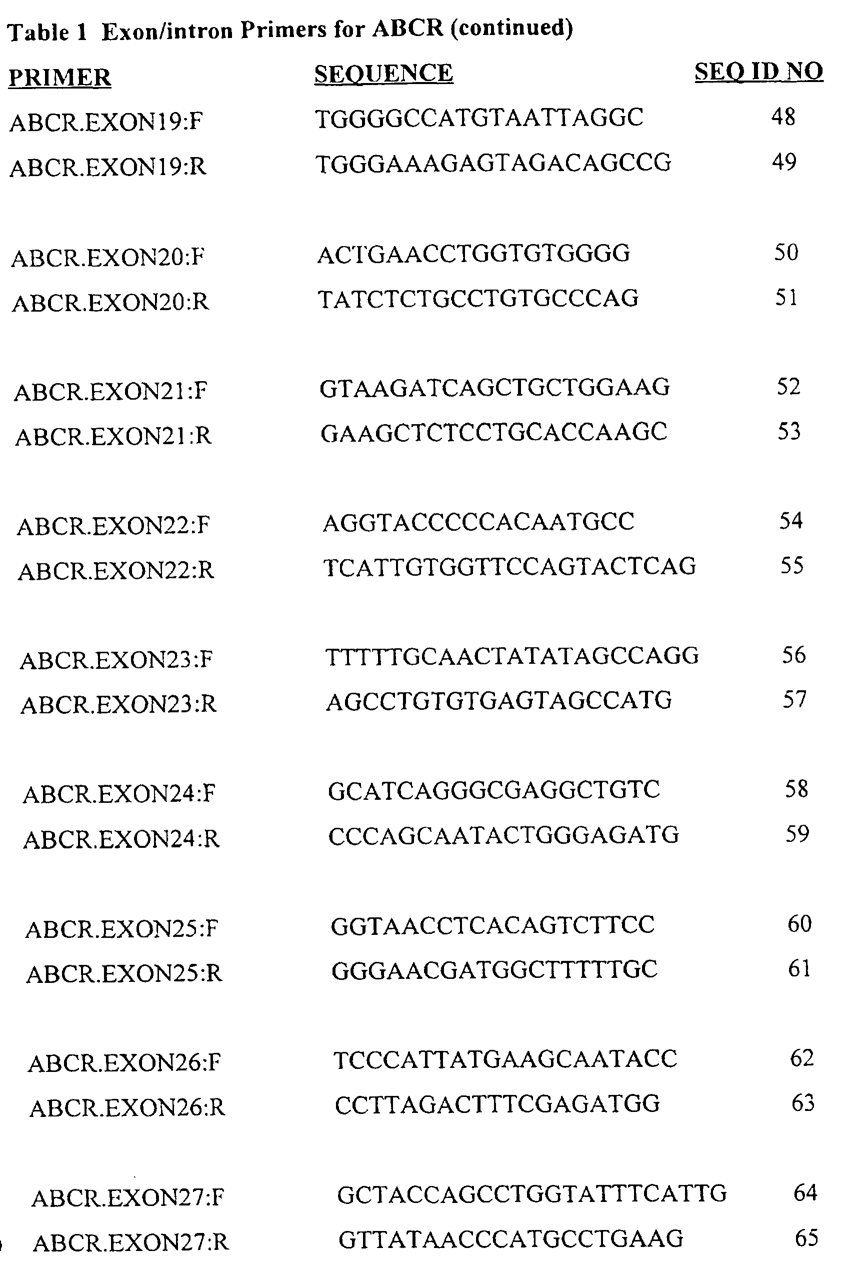

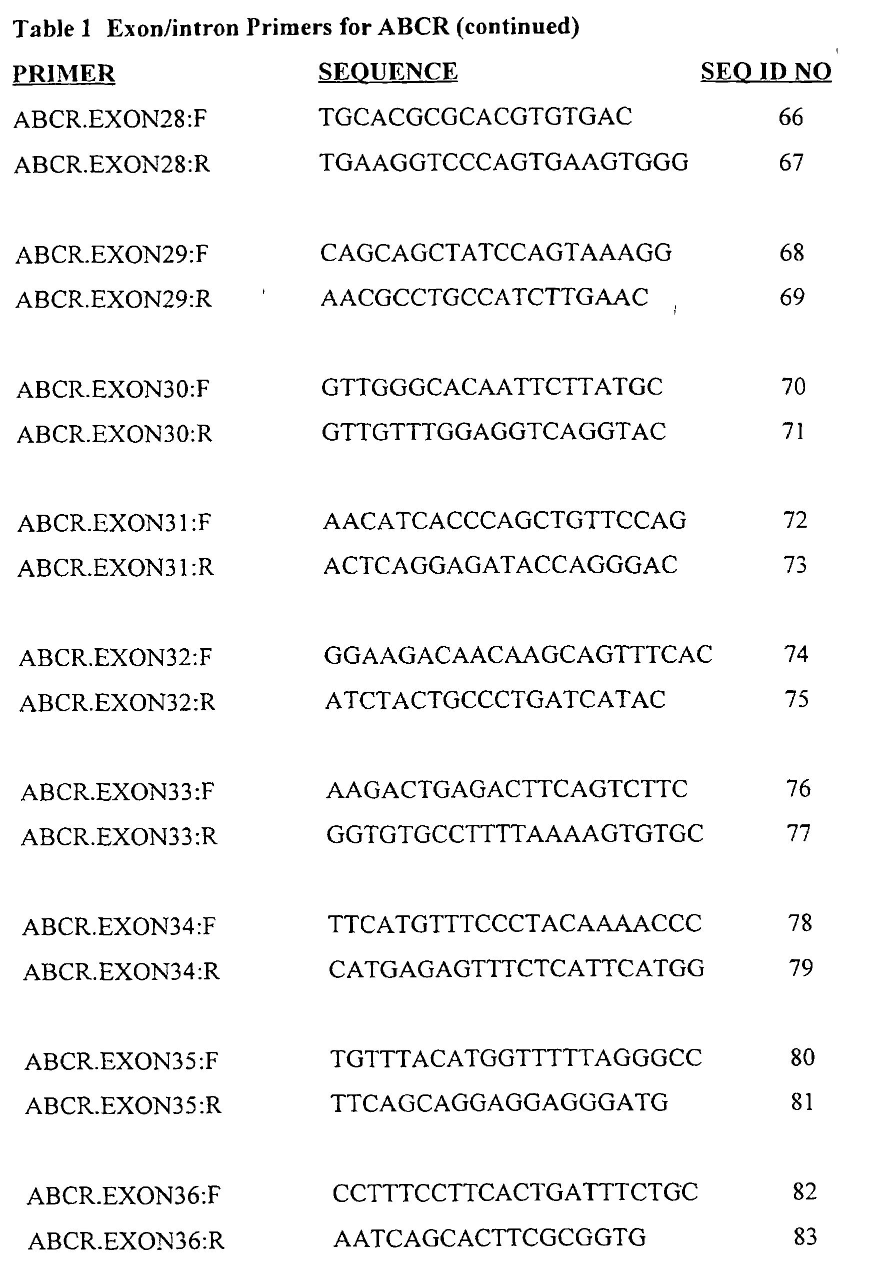

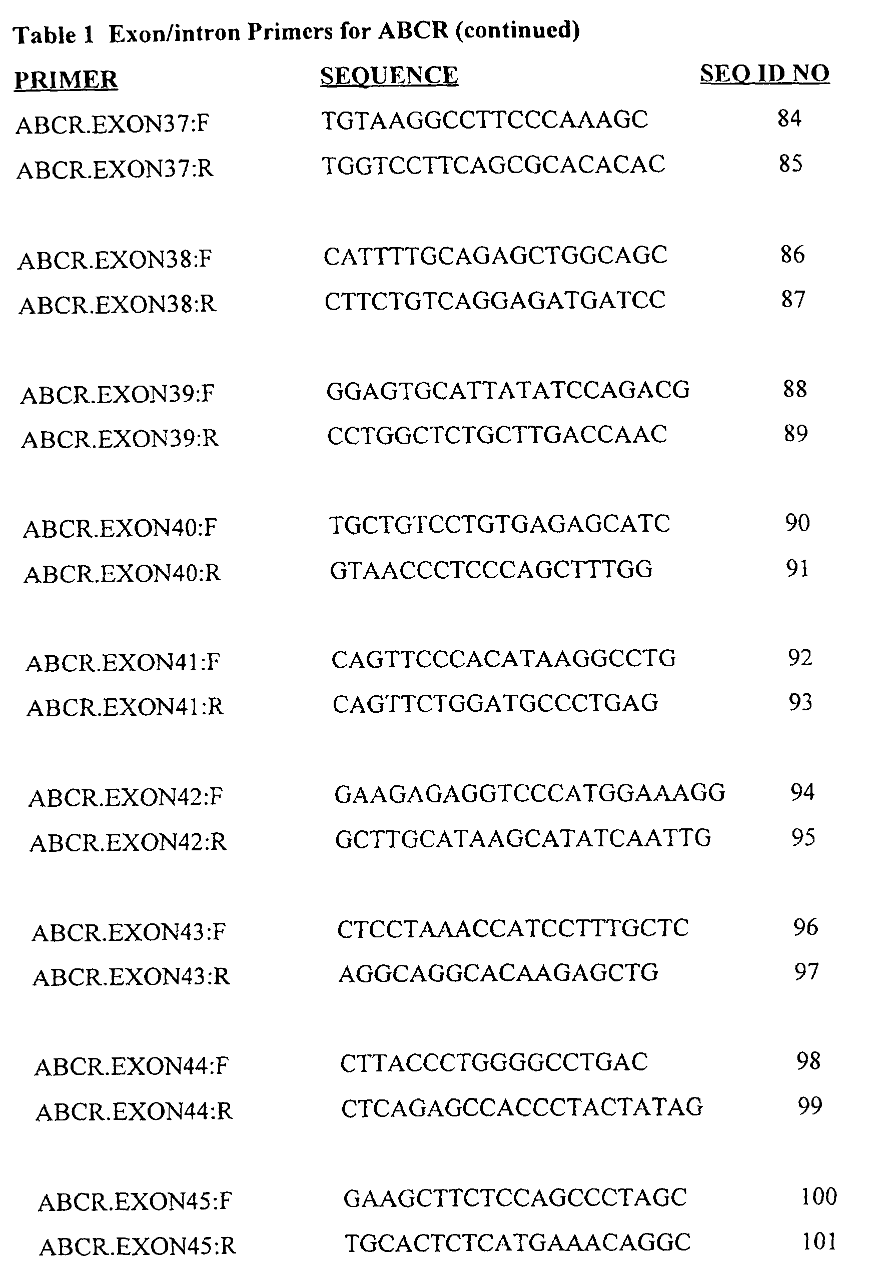

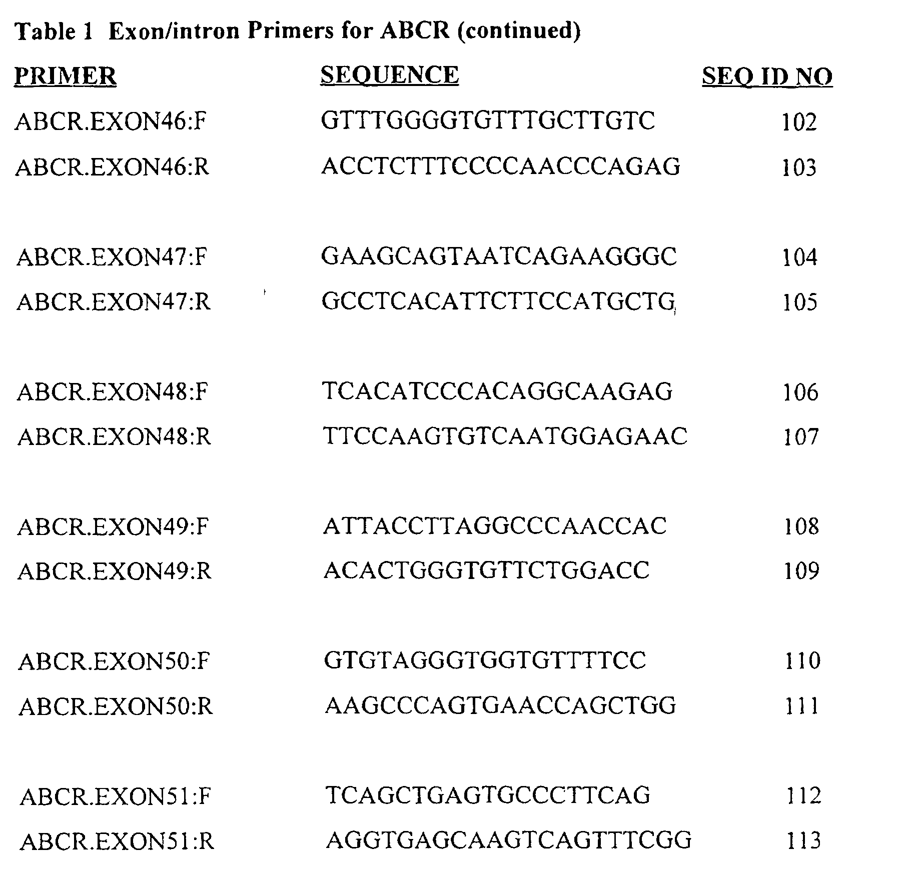

In Table 1, "F" indicates forward, i.e., 5' to 3', "R" indicates reverse, i.e., 3' to 5'. PCR conditions were 95°C for 8 minutes; 5 cycles at 62 °C for 20 seconds, 72°C for 30 seconds; 35 cycles at 60°C for 20 seconds, 72°C for 30 seconds; 72°C for 5 minutes (except that ^{a} was performed at 94° C for 5 minutes); 5 cycles at 94° C for 40 seconds; 60° C for 30 seconds; 72 ° C for 20 seconds; 35 cycles at 94 ° C for 40 seconds; 56 ° C for 30 seconds; 72 ° C for 20 seconds, and 72 ° C for 5 minutes.

Amplification of exons was performed with AmpliTaq Gold polymerase in a 25 µl volume in 1X PCR buffer supplied by the manufacturer (Perkin Elmer, Foster City, CA). Samples were heated to 95°C for 10 minutes and amplified for 35-40 cycles at 96°C for 20 seconds; 58°C for 30 seconds; and 72°C for 30 seconds. PCR products were analyzed on 1-1.5% agarose gels and in some cases digested with an appropriate restriction enzymes to verify their sequence. Primer sequences and specific reaction conditions are set forth in Table 1. The sequence of the *ABCR* cDNA has been deposited with GenBank under accession # U88667.

### Homology to ABC Superfamily Members

A BLAST search revealed that *ABCR* is most closely related to the previously characterized mouse *Abc1* and *Abc2* genes (Luciani *et al*., 1994) and to another human gene (*ABCC*) which maps to chromosome 16p13.3 (Klugbauer and Hofmann, 1996). These genes, together with *ABCR* and a gene from *C. elegans* (GenBank #Z29117), form a subfamily of genes specific to multicellular organisms and not represented in yeast (Michaelis and Berkower, 1995: Allikmets *et al*., 1996). Alignment of the cDNA sequence of *ABCR* with the *Abc1, Abc2,* and *ABCC* genes revealed, as expected, the highest degree of homology within the ATP-binding cassettes. The predicted amino acid identity of the *ABCR* gene to mouse *Abc1* was 70% within the ATP-binding domains; even within hydrophobic membrane-spanning segments, homology ranged between 55 and 85% (Figure 4 A-D). The putative *ABCR* initiator methionine shown in Figures 3 A-H and 4 A-D corresponds to a methionine codon at the 5' end of *Abc1* (Luciani *et al*., 1994).

*ABCR* shows the composition of a typical full-length ABC transporter that consists of two transmembrane domains (TM), each with six membrane spanning hydrophobic segments, as predicted by a hydropathy plot (data not shown), and two highly conserved ATP-binding domains (Figures 3 A-H and 4 A-D). In addition, the HH1 hydrophobic domain, located between the first ATP and second TM domain and specific to this subfamily (Luciani *et al.,* 1994), showed a predicted 57% amino acid identity (24 of 42 amino acids) with the mouse *Abc1* gene.

5 To characterize the mouse ortholog of *ABCR*, cDNA clones from a developing mouse eye library were isolated. A partial sequence of the mouse cDNA was utilized to design PCR primers to map the mouse *Abcr* gene in an interspecific backcross mapping panel (Jackson BSS). The allele pattern of *Abcr* was compared to 2450 other loci mapped previously in the Jackson BSS cross; linkage was found to the distal end of chromosome 3 o (Figure 5). No recombinants were observed between *Abcr* and *D13Mit13.* This region of the mouse genome is syntenic with human chromosome 1p13-p21. Thus far, no eye disease phenotype has been mapped to this region of mouse chromosome 3.

### Compound Heterozygous and Homozygous Mutations in STGD Patients

One hundred forty-five North American and three Saudi Arabian families with STGD/FFM were examined. Among these, at least four were consanguineous families in which the parents were first cousins. Entry criteria for the characterization of the clinical and angiographic diagnosis of Stargardt disease, ascertainment of the families, and methodology for their collection, including the consanguineous families from Saudi Arabia, were as provided in Anderson *et al.,* 1995; and Anderson, 1996.

Mutational analysis of the *ABCR* gene was pursued in the above identified one hundred forty-eight STGD families previously ascertained by strict definitional criteria and shown to be linked to chromosome 1p (Anderson *et al.,* 1995; Anderson, 1996). To date, all 51 exons have been used for mutation analysis.

Mutations were detected by a combined SSCP (Orita *et al.,* 1989) and heteroduplex analysis (White *et al.,* 1992) under optimized conditions (Glava 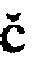 and Dean, 1993). Genomic DNA samples (50 ng) were amplified with AmpliTaq Gold polymerase in 1X PCR buffer supplied by the manufacturer (Perkin Elmer, Foster City, CA) containing [α -³²P] dCTP. Samples were heated to 95°C for 10 minutes and amplified for 35-40 cycles at 96°C for 20 seconds; 58°C for 30 seconds; and 72 °C for 30 seconds. Products were diluted in 1:3 stop solution, denatured at 95°C for 5 minutes, chilled in ice for 5 minutes, and loaded on gels. Gel formulations include 6% acrylamide:Bis (2.6% cross-linking), 10% glycerol at room temperature, 12W; and 10% acrylamide:Bis (1.5% cross-linking), at 4°C, 70W. Gels were run for 2-16 hours (3000 Vh/100 bp), dried, and exposed to X-ray film for 2-12 hours. Some exons were analyzed by SSCP with MDE acrylamide (FMC Bioproducts, Rockland, ME) with and without 10% glycerol for 18 hours, 4 watts at room temperature with α-P³²⁻dCTP labeled DNA. Heteroduplexes were identified from the double-stranded DNA at the bottom of the gels, and SSCPs were identified from the single-stranded region. Samples showing variation were compared with other family members to assess segregation of the alleles and with at least 40 unrelated control samples, from either Caucasian or Saudi Arabian populations, to distinguish mutations from polymorphisms unrelated to STGD. PCR products with SSCP or heteroduplex variants were obtained in a 25 µl volume, separated on a 1% agarose gel, and isolated by a DNA purification kit (PGC Scientific, Frederick. MD). Sequencing was performed on an ABI sequencer with both dye primer and dye terminator chemistry.

Some mutations were identified with a heteroduplex analysis protocol (Roa *et al*., 1993). Equimolar amounts of control and patient PCR products were mixed in 0.2 ml tubes. Two volumes of PCR product from a normal individual served as a negative control, and *MPZ* exon 3 from patient BAB731 as a positive control (Roa *et al*., 1996). Samples were denatured at 95°C for 2 minutes and cooled to 35 °C at a rate of 1 °C/minute. Samples were loaded onto 1.0 mm thick, 40 cm MDE gels (FMC Bioproducts, Rockland, ME), electrophoresed at 600-800 V for 15-20 hours, and visualized with ethidium bromide. Samples showing a variant band were reamplified with biotinylated forward and reverse primers and immobilized on streptavidin-conjugated beads (Warner *et al.* 1996). The resulting single strands were sequenced by the dideoxy-sequencing method with Sequenase 2.0 (Amersham, Arlington Heights, IL).

A total of seventy five mutations were identified, the majority representing missense mutations in conserved amino acid positions. However, several insertions and deletions representing frameshifts were also found (Table 2). The sequence of two mutations are shown in Figure 6A and 6B. Two missense alterations (D847H. R943Q) were found in at least one control individual, suggesting that they are neutral polymorphisms. The remaining mutations were found in patients having macular degeneration and were not found in at least 220 unrelated normal controls (440 chromosomes), consistent with the interpretation that these alterations represent disease-causing mutations, not polymorphisms. One of the mutations, 5892+1 G-T, occurs in family AR144 in which one of the affected children is recombinant for the flanking marker *DIS236* (Anderson *et al.,* 1995). This mutation, however, is present in the father as well as in both affected children. Therefore, the *ABCR* gene is non-recombinant with respect to the Stargardt disease locus.

The mutations are scattered throughout the coding sequence of the *ABCR* gene (see Table 2 and Figure 3 A-H), although clustering within the conserved regions of the ATP-binding domains is noticeable. Homozygous mutations were detected in three likely consanguineous families, two Saudi Arabian and one North American (Anderson *et al*., 1995), in each of which only the affected individuals inherited the identical disease allele (Table 2; Figure 6C). Forty two compound heterozygous families were identified in which the two disease alleles were transmitted from different parents to only the affected offspring (Table 2).

**Table 2. Mutations in the ABCR gene in STGD Families**

| | | | |
|---|---|---|---|
| **Nucleotide** | **Amino Acid** | **#Families** | **Exon** |
| 0223T->G | C75G | 1 | 3 |
| 0634C->T | R212C | 1 | 6 |
| 0664de113 | fs | 1 | 6 |
| 0746A->G | D249G | 1 | 6 |
| 1018T->G | Y340D | 2 | 8 |
| 1411G->A | E471K | 1 | 11 |
| 1569T->G | D523E | 1 | 12 |
| 1715G->A | R572Q | 2 | 12 |
| 1715G->C | R572P | 1 | 12 |
| 1804C->T | R602W | 1 | 13 |
| 1822T->A | F6081 | 1 | 13 |
| 1917C->A | Y639X | 1 | 13 |
| 2453G->A | G818E | 1 | 16 |
| 2461T->A | W821R | 1 | 16 |
| 2536G- > C | D846H | 1 | 16 |
| 2588G->C | G863A | 11 | 17 |
| 2791G->A | V931M | 1 | 19 |
| 2827C->T | R943W | 1 | 19 |
| 2884delC | fs | 1 | 19 |
| 2894A->G | N965S | 3 | 19 |
| 3083C->T | A1028V | 14 | 21 |
| 3211delGT | fs | 1 | 22 |
| 3212C->T | S1071L | 1 | 22 |
| 3215T->C | V1072A | 1 | 22 |
| 3259G->A | E1087K | 1 | 22 |
| 3322C->T | R1108C | 6 | 22 |
| 3364G->A | E1122K | 1 | 23 |
| 3385G->T | R1129C | 1 | 23 |
| 3386G->T | R1129L | 1 | 23 |
| 3602T->G | L1201R | 1 | 24 |
| 3610G->A | D1204N | 1 | 25 |
| 4139C->T | P1380L | 2 | 28 |
| 4195G->A | E1399K | 1 | 28 |
| 4222T->C | W1408R | 3 | 28 |
| 4232insTATG | fs | 1 | 28 |
| 4253 + 5G- > T | splice | 1 | 28 |
| 4297G- > A | V1433I | 1 | 29 |
| 4316G->A | G1439D | 1 | 29 |
| 4319T->C | F1440S | 1 | 29 |
| 4346G- > A | W1449X | 1 | 29 |
| 4462T->C | C1488R | 1 | 30 |
| 4469G->A | C 1490Y | 1 | 31 |
| 4577C->T | T1526M | 6 | 32 |
| 4594G->A | D1532N | 2 | 32 |
| 4947delC | fs | 1 | 36 |
| 5041de115 | VVAIC1681del | 1 | 37 |
| 5196+2T->C | splice | 1 | 37 |
| 5281de19 | PAL1761del | 1 | 38 |
| 5459G->C | R1820P | 1 | 39 |
| 5512C->T | H1838Y | 1 | 40 |
| 5527C->T | R1843W | 1 | 40 |
| 5585+1G->A | splice | 1 | 41 |
| 5657G->A | G1886E | 1 | 41 |
| 5693G->A | R1898H | 4 | 41 |
| 5714+5G->A | splice | 8 | 41 |
| 5882G->A | G1961E | 16 | 43 |
| 5898+1G->A | splice | 3 | 43 |
| 5908C->T | L1970F | 1 | 44 |
| 5929G->A | G1977S | 1 | 44 |
| 6005+1G->T | splice | 1 | 44 |
| 6079C->T | L2027F | 11 | 45 |
| 6088C->T | R2030X | 1 | 45 |
| 6089G->A | R2030Q | 1 | 45 |
| 6112C->T | R2038W | 1 | 45 |
| 6148G->C | V2050L | 2 | 46 |
| 6166A->T | K2056X | 1 | 46 |
| 6229C->T | R2077W | 1 | 46 |
| 6286G->A | E2096K | 1 | 47 |
| 6316C->T | R2106C | 1 | 47 |
| 6391G->A | E2131K | 1 | 48 |
| 6415C->T | R2139W | 1 | 48 |
| 6445C->T | R2149X | 1 | 48 |
| 6543del36 | 1181de112 | 1 | 49 |
| 6709delG | fs | 1 | 49 |

Mutations are named according to standard nomenclature. The column headed "Exon" denotes which of the 51 exons of ABCR contain the mutation. The column headed "# Families" denotes the number of Stargardt families which displayed the mutation. The column headed "Nucleotide" gives the base number starting from the A in the initiator ATG, followed by the wild type sequence and an arrow indicating the base it is changed to; del indicates a deletion of selected bases at the given position in the ABCR gene; ins indicates an insertion of selected bases at the given position; splice donor site mutations are indicated by the number of the last base of the given exon, followed by a plus sign and the number of bases into the intron where the mutation occurs. The column headed "Amino Acid" denotes the amino acid change a given mutation causes; fs indicates a frameshift mutation leading to a truncated protein; splice indicates a splice donor site mutation; del indicates an in-frame deletion of the given amino acids.

Mutations are named according to standard nomenclature. Exon numbering according to the nucleotide position starting from the A in the initiator ATG.

### In Situ Hybridization

STGD is characterized histologically by a massive accumulation of a lipofuscin-like substance in the retinal pigment epithelium (RPE). This characteristic has led to the suggestion that STGD represents an RPE storage disorder (Blacharski *et al*., 1988). It was therefore of interest that *ABCR* transcripts were found to be abundant in the retina. To identify the site(s) of *ABCR* gene expression at higher resolution and to determine whether the gene is also expressed in the RPE, the distribution of *ABCR* transcripts was visualized by *in situ* hybridization to mouse, rat, bovine, and macaque ocular tissues.

*In situ* hybridization with digoxigenin-labeled riboprobes was performed as described by Schaeren-Wiemers and Gerfin-Moser, 1993. For mouse and rat, unfixed whole eyes were frozen and sectioned; macaque retinas were obtained following cardiac perfusion with paraformaldehyde as described (Zhou *et al.,* 1996). An extra incubation of 30 min in 1 % Triton X-100, 1X PBS was applied to the fixed monkey retina sections immediately after the acetylation step. The templates for probe synthesis were: (1) a 1.6 kb fragment encompassing the 3' end of the mouse *Abcr* coding region, (2) a full length cDNA clone encoding the mouse blue cone pigment (Chiu *et al*., 1994), and (3) a macaque rhodopsin coding region segment encoding residues 133 to 254 (Nickells, R. W., Burgoyne, C.F., Quigley, H.A., and Zack, D.J. (1995)).

This analysis showed that *ABCR* transcripts are present exclusively within photoreceptor cells (Figure 7). *ABCR* transcripts are localized principally to the rod inner segments, a distribution that closely matches that of rhodopsin gene transcripts. Interestingly, *ABCR* hybridization was not observed at detectable levels in cone photoreceptors, as judged by comparisons with the hybridization patterns obtained with a blue cone pigment probe (compare Figure 7A and Figure 7D, Figure 7E with Figure 7F and Figure 7G with Figure 7H). Because melanin granules might obscure a weak hybridization signal in the RPE of a pigmented animal, the distribution of *ABCR* transcripts was also examined in both albino rats and albino mice. In these experiments, the *ABCR* hybridization signal was seen in the photoreceptor inner segments and was unequivocally absent from the RPE (Figure 7E). Given that *ABCR* transcripts in each of these mammals, including a primate, are photoreceptor-specific, it is highly likely that the distribution of *ABCR* transcripts conforms to this pattern as well in the human retina.

Various modifications of the invention in addition to those shown and described herein will be apparent to those skilled in the art from the foregoing description.

### References

Allikmets, R., Singh, N., Sun, H., Shroyer, N.F., Hutchinson, A., Chidambaram, A., Gerrard, B., Baird, L., Stauffer, D., Peiffer, A., Rattner, A., Smallwood, P., Li, Y., Anderson, K.L., Lewis, R.A., Nathans, J., Leppert, M., Dean, M., Lupski, J.R., (1997) A photoreceptor cell-specific ATP-binding transporter gene (ABCR) is mutated in recessive Stargardt macular dystrophy. Nature Genetics. 15(3):236-46.

Allikmets, R., Shroyer, N.F., Singh, N., Seddon, J.M., Lewis, R.A.,Bernsteinm P.S., Peiffer, A., Zabriskie, N.A., Li, Y., Hutchinson, A., Dean, M., Lupski, J.R., Leppert, M., (1997) Mutation of the Stargardt disease gene (ABCR) in age-related macular degeneration. Science. 277(5333):1805-7.

Allikmets, R., Gerrard, B., Hutchinson, A., and Dean, M. (1996). Characterization of the human ABC superfamily: Isolation and mapping of 21 new genes using the Expressed Sequence Tag database. Hum. Mol. Genet. *5*, 1649-1655.

Allikmets, R., Gerrard, B., Glavac, D., Ravnik-Glavac, M., Jenkins, N.A., Gilbert, D.J., Copeland, N.G., Modi, W., and Dean, M. (1995). Characterization and mapping of three new mammalian ATP-binding transporter genes from an EST database. Mam. Genome 6, 114-117.

Allikmets, R., Gerrard, B., Court, D. and Dean, M. (1993). Cloning and organization of the *abc* and *mdl* genes of *Escherichia coli:* relationship to eukaryotic multidrug resistance. Gene *136*, 231-236.

Altschul, S.F., Gish, W., Miller, W., Myers, E.W., and Lipman, D.J. (1990). Basic local alignment search tool. J. Mol. Biol. *215*, 403-410.

Anderson, K.L., Baird, L., Lewis, R.A., Chinault, A.C., Otterud, B., Leppert, M., and Lupski, J.R. (1995). A YAC contig encompassing the recessive Stargardt disease gene (*STGD*) on chromosome 1p. Am. J. Hum. Genet. *57*, 1351-1363.

Anderson, K.L. (July 30, 1996) Towards the Isolation of Genes for Recessively Inherited Ocular.Disorders; Bardet-Biedl Syndrome, Leber Congenital Amaurosis, Primary Congenital Glaucoma, and Stargardt Disease. Ph.D. Thesis. Baylor College of Medicine.

Anderson, R.E. and Maude, M.B. (1971). Lipids of ocular tissues: the effects of essential fatty acid deficiency on the phospholipids of the photoreceptor membranes of rat retina. Arch. Biochem. Biophys. *151*, 270-276.

Azarian, S.M., Travis, G.H., (1997) The photoreceptor rim protein is an ABC transporter encoded by the gene for recessive Stargardt's disease (ABCR). FEBS Letters. 409(2):247-52.

Bellanne-Chantelot, C., *et al.* (1992). Mapping the Whole Human Genome by Fingerprinting Yeast Artificial Chromosomes. Cell *70*, 1059-1068.

Birnbach, C.D., Järveläinen, M., Possin, D.E., and Milam, A.H. (1994). Histopathology and immunocytochemistry of the neurosensory retina in fundus flavimaculatus. Ophthalmology *101*, 1211-1219.

Blacharski, D.A. (1988). Fundus flavimaculatus. In Retinal Dystrophies and Degenerations, D.A. Newsome, ed. (New York: Raven Press), pp. 135-159.

Boguski, M.S., Lowe, T.M., and Tolstoshev, C.M. (1993), dbEST-database for "expressed sequence tags". Nature Genet. *4*, 332-333.

Chen, Z.-Y., Battinelli, E.M., Hendricks, R.W., Powell, J.F., Middleton-Price, H., Sims, K.B., Breakfield, X.O., and Craig, I.W. (1993). Norrie disease gene: characterization of deletions and possible function. Genomics *16*, 533-535.

Childs, S., and Ling, V. (1994). The MDR superfamily of genes and its biological implications. In Important Advances in Oncology, V.T. DeVita, S. Hellman and S.A. Rosenberg, eds. (Philadelphia, Pennsylvania: Lippincott Company), pp. 21-36.

Chiu, M. I., Zack, D.J., Wang, Y., and Nathans, J. (1994). Murine and bovine blue cone pigment genes: cloning and characterization of two new members of the S family of visual pigments. Genomics *21*, 440-443.

Chomczynski, P. and Sacchi, N. (1987). Single-step method of RNA isolation by acid guanidinium thiocyanate-phenol-chloroform extraction. Anal. Biochem. *162*, 156-159.

Daemen, F.J.M. (1973). Vertebrate rod outer segment membranes. Biochem. Biophys. Acta *300*, 255-288.

de la Salle, H., Hanau, D., Fricker, D., Urlacher, A., Kelly, A., Salamero, J., Powis, S.H., Donato, L., Bausinger, H., Laforet, M., Jeras, M., Spehner, D., Bieber, T., Falkenrodt, A., Cazenave, J.-P., Trowsdale, J., and Tongio, M.-M. (1994). Homozygous human TAP peptide transporter mutation in HLA class I deficiency. Science *265*, 237-241.

Dean, M., and Allikmets, R. (1995). Evolution of ATP-binding cassette transporter genes. Curr. Opin. Genet. Dev. 5, 779-785.

Dean, M., Allikmets, R., Gerrard, B., Stewart, C., Kistler, A., Shafer, B., Michaelis, S., and Strathern, J. (1994). Mapping and sequencing of two yeast genes belonging to the ATP-binding cassette superfamily. Yeast *10*, 377-383.

Devereaux, J., Haeberli, P., and Smithies, O. (1984). A comprehensive set of sequence analysis programs for the VAX. Nucleic Acids Res. *12*, 387-395.

Dowling, J. E. (1960). Chemistry of visual adaptation in the rat. Nature *188*, 114-118.

Dryja, T.P. and Li, T. (1995). Molecular genetics of retinitis pigmentosa. Hum. Mol. Genet. *4*, 1739-1743.

Eagle, R.C. Jr., Lucier, A.C., Bemadino, V.B. Jr., and Yanoff, M. (1980). Retinal pigment epithelial abnormalities in Fundus Flavimaculatus: A light and electron microscopic study. Ophthalmology *87,* 1189-1200.

Feeney, L. (1978). Lipofuscin and melanin of human retinal pigment epithelium. Fluorescence, enzyme cytochemical, and ultrastructural studies Invest. Ophthalmol. Vis. Sci. *17*, 583-600.

Feng, D.-F., and Doolittle, R.F. (1987). Progressive sequence alignment as a prerequisite to correct phyllogenetic trees. J. Mol. Evol. *25,* 351-360.

Fishman, G.A. (1976). Fundus flavimaculatus: a clinical classification. Arch. Ophthalmol. 94, 2061-2067.

Fong, S.-L., Liou, G.I., Landers, R.A., Alvarez, R.A., and Bridges, C.D. (1984). Purification and characterization of a retinol-binding glycoprotein synthesized and secreted by bovine neural retina. J. Biol. Chem. 259, 6534-6542.

Franceschetti, A. (1963). Über tapeto-retinale Degenerationen im Kindesalter. In H. von Sautter, ed. Entwicklung und Fortschritt in der Augenheilkunde. (Stuttgart: Ferdinand Enke) pp. 107-120.

Gass, J.D.M. (1987). Stargardt's disease (Fundus Flavimaculatus) in Stereoscopic Atlas of Macular Diseases: Diagnosis and Treatment, Volume 1, 3rd edition, pages 256-261. The C.V. Mosby Company, St Louis, MO.

Gerber, S., Rozet, J.-M., Bonneau, D., Souied, E., Camuzat, A., Dufier, J.-L., Amalric, P., Weissenbach, J., Munnich, A., and Kaplan, J. (1995). A gene for late-onset fundus flavimaculatus with macular dystrophy maps to chromosome 1p13. Am. J. Hum. Genet. *56*, 396-399.

Glava 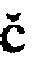 , D., and Dean, M. (1993). Optimization of the single-strand conformation polymorphism (SSCP) technique for detection of point mutations. Hum. Mutat. 2, 404-414.

Hayes, K.C. (1974). Retinal degeneration in monkeys induced by deficiencies of vitamin E or A. Invest. Ophthalmology *13*, 499-510.

Hettema, E.H., van Roermund, C.W.T., Distel, B., van den Berg, M., Vilela, C., Rodrigues-Pousada, C., Wanders, R.J.A., and Tabak, H.F. (1996). The ABC transporter proteins Pat1 and Pat2 are required for import of long-chain fatty acids into peroxisomes of Saccharomyces cerevisiae. EMBO J. *15*, 3813-3822.

Hoyng, C.B., Poppelaars, F., van de Pol, T.J.R., Kremer, H., Pinckers, A.J.L.G., Deutman, A.F., and Cremers, F.P.M. (1996). Genetic fine mapping of the gene for recessive Stargardt disease. Hum. Genet. 98, 500-504.

Hyde, S.C., Emsley, P., Hartshorn, M.J., Mimmack, M.M., Gileadi, U., Pearce, S.R., Gallagher, M.P., Gill, D.R., Hubbard, R.E., and Higgins, C.F. (1990). Structural model of ATP-binding proteins associated with cystic fibrosis, multidrug resistance and bacterial transport. Nature 346, 362-365.

Klein, B.A. and Krill, A.E. (1967). Fundus Flavimaculatus: clinical, functional, and histopathologic observations. Am. J. Ophthalmol. *64*, 3-23.

Klugbauer, N., and Hofmann, F. (1996). Primary structure of a novel ABC transporter with a chromosomal localization on the band encoding the multidrug resistance-associated protein. FEBS Lett. *391,* 61-65.

Kuwano, Y., Nakanishi, O., Nabeshima, Y.-I., Tanaka, T., and Ogata, K. (1985). Molecular cloning and nucleotide sequence of DNA complementary to rat ribosomal protein S26 messenger RNA. J. Biochem. (Tokyo) *97*: 983-992.

Lennon, G., Auffray, C., Polymeropoulos, M. and Soares, M.B. (1996). The I.M.A.G.E. consortium: An integrated molecular analysis of genomes and their expression. Genomics *33*, 151-152.

Lincoln, A.L., Daly, M., and Lander, E. (1991). PRIMER: a computer program for automatically selecting PCR primers. Whitehead Institute Technical Report.

Lopez, P.F., Maumenee, I.H., de la Cruz, Z., Green, W.R. (1990). Autosomal-dominant fundus flavimaculatus: clinicopathologic correlation. Ophthalmology *97*, 798-809.

Luciani, M.-F., Denizot, F., Savary, S., Mattei, M.G., and Chimini, G. (1994). Cloning of two novel ABC transporters mapping on human chromosome 9. Genomics *21*, 150-159.

Luciani, M.-F., and Chimini, G. (1996). The ATP binding cassette transporter ABC1, is required for the engulfment of corpses generated by apoptotic cell death. EMBO J. *15*, 226-235.

McDonnell, P.J., Kivlin, J.D., Maumenee, I.H., and Green, W.R. (1986). Fundus flavimaculatus without maculopathy: a clinicopathologic study. Ophthalmology *93*, 116-119.

Meindl, A., Berger, W., Meitinger, T., van de Pol, D., Achatz, H., Dorner, C., Hasseman, M., Hellebrand, H., Gal, A., Cremers, F., and Ropers, H.H. (1992). Norrie disease is caused by mutations in an extracellular protein resembling c-terminal globular domain of mucins. Nat. Genet. *2*, 139-143.

Meindl, A., Dry, K., Herrmann, K., Manson, F., Ciccodicola, A., Edgar, A., Carvalho, M.R.S., Achatz, H., Hellebrand, H., Lennon, A., Mibliaccio, C., Porter, K., Zrenner, E., Bird, A., Jay, M., Lorenz, B., Wittwer, B., D'urso, M., Meitinger, T., and Wright, A. (1996). A gene (RPGR) with homology to the RCC1 guanine nucleotide exchange factor is mutated in X-linked retinitis pigmentosa (RP3). Nat. Genet. *13*, 35-42.

Michaelis, S., and Berkower, C. (1995). Sequence comparison of yeast ATP binding cassette (ABC) proteins. In Cold Spring Harbor Symposium on Quantitative Biology, vol.LX: Protein Kinesis - The Dynamics of Protein Trafficking and Stability. (Cold Spring Harbor Laboratory Press, Cold Spring Harbor).

Mosser, J., Douar, A.-M., Sarde, C.-O., Kioschis, P., Feil, R., Moser, H., Poustka, A.-M., Mandel, J.-L., and Aubourg, P. (1993). Putative X-linked adrenoleukodystrophy gene shares unexpected homology with ABC transporters. Nature *361*, 726-730.

Nathans, J., Thomas, D., and Hogness, D.S. (1986). Molecular genetics of human color vision: the genes encoding blue, green, and red pigments. Science *232*, 193-202.

Nickells, R. W., Burgoyne, C.F., Quigley, H.A., and Zack, D.J. (1995). Cloning and characterization of rodopsin cDNA from the old world monkey, Macaca fasciclaris. Investigative Ophthalmology and Visual Science 36:72-82.

Noble, K.G., and Carr, R.E. (1979). Stargardt's disease and fundus flavimaculatus. Arch. Ophthalmol. *97*, 1281-1285.

Orita, M., Suzuki, Y., Sekiya, T., and Hayashi, K. (1989). Rapid and sensitive detection of point mutations and DNA polymorphisms using the polymerase chain reaction. Genomics 5, 874-879.

Rando, R.R. (1990). The chemistry of vitamin A and vision. Angew. Chem. Int. Ed. Engl. *29*, 461-480.

Riordan, J.R., Rommens, J.M., Kerem, B.-S., Alon, N., Rozmahel, R., Grzelczak, Z., Zielenski, J., Lok, S., Plavsic, N., Chou, J.-L., Drumm, M.L., Ianuzzi, M.C., Collins, F.S., and Tsui, L.-C. (1989). Identification of the cystic fibrosis gene: cloning and characterization of complementary DNA. Science *245*, 1066-1073.

Roa, B.B., Garcia, C.A., Suter, U., Kulpa, D.A., Wise, C.A., Mueller, J., Welcher, A.A., Snipes, G.J., Shooter, E.M., Patel, P.I., and Lupski, J.R. (1993). Charcot-Marie-Tooth disease type 1A, association with a spontaneous point mutation in the *PMP22* gene. New Eng. J. Med. *329,* 96-101.

Roa, B.B., Warner, L.E., Garcia, C.A., Russo, D., Lovelace, R., Chance P.F., and Lupski, J.R. (1996). Myelin protein zero (*MPZ*) gene mutations in nonduplication type 1 Charcot-Marie-Tooth disease. Hum. Mut. 7, 36-45.

Rowe, L.B., Nadeau, J.H., Turner, R., Frankel, W.N., Letts, V.A., Eppig, J., Ko, M.S.H., Thurston, S.J., and Birkenmeier, E.H. (1994). Maps from two interspecific backcross DNA panels available as a community genetic mapping resource. Mamm. Genome *5*, 253-274.

Sambrook, J., Fritsch, E.F., and Maniatis, T. (1989). Molecular Cloning. (Cold Spring Harbor, New York: Cold Spring Harbor Laboratory).

Schaeren-Wiemers, N., and Gerfin-Moser, A. (1993). A single protocol to detect transcripts of various types and expression levels in neural tissue and culture cells: in situ hybridization using digoxigenin-labeled cRNA probes. Histochemistry *100*, 431-440.

Seabra, M.C., Brown, M.S., and Goldstein, J.L. (1993). Retinal degeneration in choroideremia: deficiency of Rab geranylgeranyl transferase. Science *259*, 377-381.

Shani, N. and Valle, D. (1996). A *Saccharomyces cerevisiae* homolog of the human adrenoleukodystrophy transporter is a heterodimer of two half ATP-binding cassette transporters. Proc. Natl. Acad. Sci. USA *93*, 11901-11906.

Shimozawa, N., Tsukamoto, T., Suzuki, Y., Orii, T., Shirayoshi, Y., Mori, T., and Fujiki, Y. (1992). A human gene responsible for Zellweger syndrome that affects peroxisome assembly. Science *255,* 1132-1134.

Smit, J.J.M., Schinkel, A.H., Oude Elferink, R.P.J., Groen, A.K., Wagenaar, E., van Deemter, L., Mol, C.A.A.M., Ottenhoff, R., van der Lugt, N.M.T., van Roon, M.A., van der Valk, M.A., Offerhaus, G.J.A., Berns, A.J.M., and Borst, P. (1993). Homozygous disruption of the murine mdr2 P-glycoprotein gene leads to a complete absence of phospholipid from bile and to liver disease. Cell *75*,451-462.

Stargardt, K. (1909). Über familiäre, progressive Degeneration in der Maculagegend des Auges. Albrecht von Graefes Arch. Klin. Exp. Ophthalmol. *71*, 534-550.

Steinmetz, R.L., Garner, A., Maguire, J.I., and Bird, A.C. (1991). Histopathology of incipient fundus flavimaculatus. Ophthalmology *98*, 953-956.

Stone, E.M., Nichols, B.E., Kimura, A.E., Weingeist, T.A., Drack, A., and Sheffield, V.C. (1994). Clinical features of a Stargardt-like dominant progressive macular dystrophy with genetic linkage to chromosome 6q. Arch. Ophthalmol. *112,* 765-772.

Sun, H., Nathans, J., (1997) Stargardt's ABCR is localized to the disc membrane of retinal rod outer segments. Nature Genetics. 17(1): 15-6.

Thomas, P.M., Cote, G.J., Wohllk, N., Haddad, B., Mathew, P.M., Rabl, W., Aguilar-Bryan, L., Gagel, R.F., and Bryan, J. (1995). Mutations in the sulfonylurea receptor gene in familial persistent hyperinsulinemic hypoglycemia of infancy. Science *268*, 426-429.

Valle, D. and Simell, O. (1995). The hyperornithinemias. In The Metabolic and Molecular Basis of Inherited Disease, Scriver, C.R., Beaudet, A.L., Sly, W.S., and Valle, D., eds. (New York: McGraw Hill), pp. 1147-1185.

van Helvoort, A., Smith, A.J., Sprong, H., Fritzsche, I., Schinkel, A.H., Borst, P., and van Meer, G. (1996). MDR1 P-glycoprotein is a lipid translocase of broad specificity, while MDR3 P-glycoprotein specifically translocates phosphatidylcholine. Cell *87*, 507-517.

Wang, Y., Macke, J.P., Abella, B.S., Andreasson, K., Worley, P., Gilbert, D.J., Copeland, N.G., Jenkins, N.A., and Nathans, J. (1996). A large family of putative transmembrane receptors homologous to the product of the Drosophilal tissue polarity gene frizzled. J. Biol. Chem. 271:4468-4476.

Warner, L.E., Hilz, M.J., Appel, S.H., Killian, J.M., Kolodny, E.H., Karpati, G., Carpenter, S., Watters, G.V., Wheeler, C., Witt, D., Bodell, A., Nelis, E., Van Broeckhoven, C., and Lupski, J.R. (1996). Clinical phenotypes of different *MPZ* (P₀) mutations may include Charcot-Marie-Tooth type 1B, Dejerine-Sottas, and congenital hypomyelination. Neuron *17*, 451-460.

Weber, B.H.F., Vogt, G., Pruett, R.C., Stohr, H., and Felbor, U. (1994). Mutations in the tissue inhibitor of metalloproteinase-3 (TIMP3) in patients with Sorsby's fundus dystrophy. Nat. Genet. 8, 352-356.

Weiter, J.J., Delori, F.C., Wing, G.L., and Fitch, K.A. (1986). Retinal pigment epithelial lipofuscin and melanin and choroidal melanin in human eyes. Invest. Ophthalmol. Vis. Sci. *27*, 145-152.

White, M.B., Carvalho, M., Derse, D., O'Brien, S.J., and Dean, M. (1992). Detecting single base substitutions as heteroduplex polymorphisms. Genomics *12*, 301-306.

Wing, G.L., Blanchard, G.C., and Weiter, J.J. (1978). The topography and age relationship of lipofuscin concentration in the retinal pigment epithelium. Invest. Ophthalmol. Vis. Sci. *17,* 601-607.

Zhang, K., Bither, P.P., Park, R., Donoso, L.A., Seidman. J.G., and Seidman, C.E. (1994). A dominant Stargardt's macular dystrophy locus maps to chromosome 13q34. Arch. Ophthalmol. *112*, 759-764.

Zhou, H., Yoshioka, T., and Nathans, J. (1996). Retina-derived POU-domain factor-1: a complex POU-domain gene implicated in the development of retinal ganglion and amacrine cells. J. Neurosci. *16*, 2261-2274.

Zinn, K. M. and Marmor, M.F. (1979). The Retinal Pigment Epithelium. (Harvard University Press, Cambridge. MA). pp. 521.

## Claims

1. A nucleic acid sequence encoding a retina-specific ATP binding cassette transporter wherein said nucleic acid sequence comprises SEQ ID NO:4.

2. The nucleic acid sequence according to claim 1 wherein said ATP-binding cassette transporter comprises an amino acid selected from the group consisting of 818 (G or E), 863 (G or A), 931 (V or M), 1028 (A or V), 1072 (V or A), 1087 (E or K), 2027 (L or F), 2038 (R or W), 2050 (V or L), 2077 (R or W), and 2106 (R or C) according to numbering of the sequence in Figure 3.

3. The nucleic acid sequence of claim 1 comprising a nucleotide selected from the group consisting of 2453 (G or A), 2588 (G or C), 2791 (G or A), 3083 (C or T), 3215 (T or C), 3259 (G or A), 6079 (C or T), 6112 (C or T), 6148 (G or C), 6229 (C or T), and 6316 (C or T) according to the numbering of the sequence in Figure 3.

4. The nucleic acid sequence of claim 3 wherein said nucleotide results in a missense mutation.

5. The nucleic acid sequence of claim 3 wherein said nucleotide results in a splice site.

6. The nucleic acid sequence of claim 1 comprising a nucleotide that results in a frameshift mutation.

7. A vector comprising a nucleic acid sequence according to claim 1.

8. A cell culture obtained by transforming a cell with an expression vector according to claim 7.

9. A host cell capable of recombinantly expressing a nucleic acid sequence according to claim 1.

10. A cell culture capable of recombinantly expressing a nucleic acid sequence according to claim 1.

11. A protein preparation comprising a recombinantly expressed polypeptide having an amino acid sequence encoded by a nucleic acid sequence according to claim 1.

12. A pharmaceutical composition comprising an effective amount of a nucleic acid sequence according to claim 1, and a pharmaceutically acceptable carrier.

13. An isolated polypeptide comprising a retina-specific ATP-binding cassette transporter which polypeptide is encoded by the nucleic acid sequence of any one of claims 1 to 3.

14. The isolated polypeptide according to claim 13 wherein the polypeptide comprises a mutation associated with macular degeneration.

15. A transgenic non-human mammal comprising a recombinant sequence comprising a nucleic acid sequence of according to claim 1 introduced into said mammal or an ancestor of said mammal, or comprising a suppressed retina-specific ATP binding cassette transporter gene comprising SEQ ID NO:4.

16. A method of screening for an agent that modulates macular degeneration comprising combining purified retina-specific ATP binding cassette transporter comprising an amino acid sequence encoded by a nucleic acid sequence according to claim 1 and at least one agent suspected of altering retina-specific ATP binding cassette transporter and observing an alteration in the activity of said purified retina-specific ATP binding cassette transporter.

17. A method of screening for an agent according to claim 16 wherein the method is a method of screening for an agent that inhibits macular degeneration, the method comprising combining the purified retina-specific ATP binding cassette transporter and at least one agent suspected of activating retina-specific ATP binding cassette transporter and observing an activation of said purified retina-specific ATP binding cassette transporter.

18. The method according to claim 17 wherein said purified retina-specific ATP binding cassette transporter originates from a patient suspected of having macular degeneration.

19. The method of claim 17 wherein the activation of said purified retina-specific ATP binding cassette transporter is observed by an inhibition of a characteristic associated with macular degeneration selected from the group consisting of inhibition of central visual impairment, inhibition of progressive bilateral atrophy of the macular retinal pigment epithelium, inhibition of progressive bilateral atrophy of the neuroepithelium, inhibition of macula flecks, inhibition of midretinal periphery flecks, and inhibition of retina-specific ATP binding cassette transporter transcripts in photoreceptor cells.

20. The method of claim 19 wherein said macular degeneration is selected from the group consisting of Stargardt Disease, Fundus Flavimaculatus, and age-related macular degeneration.

21. The method of claim 17 further comprising the step of amplifying said nucleic acid sequence encoding a retina-specific ATP binding cassette transporter using an oligonucleotide primer that specifically hybridizes to said sequence.

22. The method of claim 17 further comprising the step of amplifying said nucleic acid sequence encoding a retina-specific ATP binding cassette transporter using an oligonucleotide primer that specifically hybridizes to 17 nucleotides to 25 nucleotides of said sequence.

23. A method of screening for an agent according to claim 16 wherein the method is a method of screening for an agent that activates macular degeneration, the method comprising combining the purified wild-type retina-specific ATP binding cassette transporter and at least one agent suspected of activating macular degeneration and observing an inhibition in said purified wild-type retina-specific ATP binding cassette transporter.

24. The method of claim 23 wherein said inhibition of said purified retina-specific ATP binding cassette transporter is observed by a characteristic associated with macular degeneration selected from the group consisting of central visual impairment, bilateral atrophy of the macular retinal pigment epithelium, bilateral atrophy of the neuroepithelium, macula flecks, midretinal periphery flecks, and retina-specific ATP binding cassette transporter transcripts in photoreceptor cells.

25. An *in vitro* method of diagnosing an individual with macular degeneration or lack thereof comprising detecting the presence or absence of a mutation associated with macular degeneration in a retina-specific ATP binding cassette transporter-encoding nucleic acid according to claim 1 in a tissue sample of said individual.

26. The method of claim 25 wherein said tissue sample is selected from the group consisting of blood, skin, serum, saliva, sputum, mucus, bone marrow, urine, lymph, a tear, chorion, and amniotic fluid.

27. The method of claim 25 wherein said mutation is selected from the group consisting of: 2453 (G→A), 2588 (G→C), 2791 (G→A), 2884 del C, 3083 (C→T), 3259 (G→A), 5041del15, 5196 +2(T→C), 6005 + 1 (G→T), 6079 (C→T), 6112 (C→T), 6148 (G→C), 6229 (C→T), and 6316 (C→T) according to the numbering of the sequence in Figure 3.

28. The method of claim 25 wherein said mutation results in an amino acid sequence difference selected from the group consisting of G818E, G863A, V931M, A1028V, V1072A, E1087K, VVAIC1681del, L2027F, R2038W, V2050L, R2077W, and R2106C according to the numbering of the sequence in Figure 3.

29. The method of claim 25 wherein the mutation is selected from the group consisting of a missense mutation, an intragenic deletion, an intragenic insertion, a frame shift mutation, and a splice site mutation.

30. The method of claim 29 wherein said mutation is a missense mutation.

31. The method of claim 29 wherein said mutation is a frameshift mutation.

32. The method of claim 29 wherein said mutation is a splice site mutation.

33. The method of claim 25 further comprising extracting RNA or DNA from said sample.

34. The method of claim 25 further comprising the step of amplifying said nucleic acid sequence encoding a retina-specific ATP binding cassette transporter.

35. The method of claim 34 wherein said amplification step comprises performing the polymerase chain reaction.

36. The method of claim 25 further comprising the step of amplifying said nucleic acid sequence encoding a retina-specific ATP binding cassette transporter using an oligonucleotide primer that specifically hybridizes to said nucleic acid sequence.

37. The method of claim 25 further comprising the step of amplifying said nucleic acid sequence encoding a retina-specific ATP binding cassette transporter using a primer having a nucleic acid sequence selected from the group consisting of SEQ ID NOs: 40-61, 64, 65, 78, 79, 98-105, 108, and 109.

38. The method of claim 34 further comprising the step of comparing said amplified nucleic acid sequence from said individual with a control nucleic acid sequence encoding wild-type retina-specific ATP binding cassette transporter wherein a mutation in said amplified nucleic acid sequence from said individual as compared to said nucleic acid sequence encoding wild-type retina-specific ATP binding cassette transporter indicates macular degeneration.

39. An *in vitro* method of diagnosing macular degeneration in an individual comprising detecting the presence of a mutated version of a retina-specific ATP binding cassette transporter encoded by a nucleic acid sequence of claim 1.

40. Use of a nucleic acid sequence according to claim 1, or a fragment thereof, in the manufacture of a medicament for the treatment of macular degeneration.

41. The use of claim 40 wherein said nucleotide encodes a wild-type ATP-binding cassette transporter having an amino acid sequence encoded by SEQ ID NO:4.

42. The use of claim 40 wherein the medicament is for treatment of a human.

43. The use of claim 40 wherein said macular degeneration is due to Stargardt disease, Fundus Flavimaculatus, age-related macular degeneration, retinitis pigmentosa, combined rod and cone dystrophies, cone dystrophies, pattern dystrophy, or bull's eye maculopathy.

44. The use of claim 40 wherein said macular degeneration is due to Stargardt disease.

45. A kit for detecting macular degeneration comprising a first container, wherein said container comprises a first primer that is between 15 and 35 nucleotides and is complementary to a first region of a retina-specific ATP-binding cassette transporter gene comprising a nucleic acid sequence according to claim 1 .

46. The kit of claim 45 wherein the first region of the gene is a coding region.

47. The kit of claim 45 wherein said first region of the gene flanks an exon.

48. The kit of claim 45 further comprising a second container wherein said second container comprises a second primer that is between 15 and 35 nucleotides and is complementary to a second region of a retina-specific ATP-binding cassette transporter gene comprising a nucleic acid sequence according to claim 1.

49. The kit of claim 48 wherein the second region of the gene is a coding region.

50. The kit of claim 48 wherein the second region of the gene flanks an exon.

51. A kit according to claim 45, wherein said first primer is selected from the group consisting of SEQ ID NOS: 40-61, 64, 65, 78, 79, 98-105, 108 and 109.

52. The kit of claim 48 wherein the second primer is selected from the group consisting of SEQ ID NOS: 40-61, 64, 65, 78, 79, 98-105, 108, and 109, but is not identical to the first primer.

53. A kit for detecting macular degeneration comprising a first container, wherein said container comprises a primer pair that is complementary to a first and a second region of a retina-specific ATP-binding cassette transporter gene comprising a nucleic acid sequence according to claim 1 wherein each primer is between 15 and 35 nucleotides.

54. The kit of claim 53 wherein the primer pair flank an exon.

55. The kit of claim 53 wherein the primer pair span a coding region.

56. The kit of claim 53 wherein the primer pair comprises a primer having sequence selected from the group consisting of SEQ ID NOS: 40-61, 64, 65, 78, 79, 98-105, 108, and 109.

57. The kit of claim 45 or 53 further comprising a means for visualizing amplified DNA.

58. The kit of claim 57 wherein said means is a fluorescent dye selected from the group consisting of ethidium bromide, ³²P, and biotin.

59. The kit of claim 45 or 53 further comprising one or more size markers.

60. The kit of claim 45 or 53 further comprising one or more positive or negative controls.

61. The kit of claim 45 or 53 further comprising one or more restriction enzymes.

62. An isolated oligonucleotide comprising a nucleic acid sequence complementary to a nucleic acid sequence according to claim 1.

63. *In vitro* use of an oligonucleotide according to claim 62 to inhibit the expression of retina- specific ATP-binding cassette transporter in a cell.

64. An isolated nucleic acid sequence comprising a nucleic acid sequence selected from the group consisting of: SEQ ID NOs:40-61, 64, 65, 78, 79, 98-105, 108, and 109.

## Patentansprüche

1. Nukleinsäuresequenz, welche für einen Retina-spezifischen ATP-Bindungs-Cassette-Transporter kodiert, wobei die Nukleinsäuresequenz SEQ ID NR:4 umfasst.

2. Nukleinsäuresequenz nach Anspruch 1, wobei der ATP-Bindungs-Cassette-Transporter eine Aminosäure, ausgewählt aus der Gruppe, bestehend aus 818 (G oder E), 863 (G oder A), 931 (V oder M), 1028 (A oder V), 1072 (V oder A), 1087 (E oder K), 2027 (L oder F), 2038 (R oder W), 2050 (V oder L), 2077 (R oder W) und 2106 (R oder C), gemäß der Numerierung der Sequenz in Figur 3, umfasst.

3. Nukleinsäuresequenz nach Anspruch 1, umfassend ein Nukleotid, ausgewählt aus der Gruppe, bestehend aus 2453 (G oder A), 2588 (G oder C), 2791 (G oder A), 3083 (C oder T), 3215 (T oder C), 3259 (G oder A), 6079 (C oder T), 6112 (C oder T), 6148 (G oder C), 6229 (C oder T) und 6316 (C oder T), gemäß der Numerierung der Sequenz in Figur 3.

4. Nukleinsäuresequenz nach Anspruch 3, wobei das Nukleotid zu einer Fehlsinn-Mutation führt.

5. Nukleinsäuresequenz nach Anspruch 3, wobei das Nukleotid zu einer SpleißStelle führt.

6. Nukleinsäuresequenz nach Anspruch 1, umfassend ein Nukleotid, welches zu einer Rahmenverschiebungsmutation führt.

7. Vektor, welcher eine Nukleinsäuresequenz nach Anspruch 1 umfasst.

8. Zellkultur, erhalten durch Transformieren einer Zelle mit einem Expressionsvektor nach Anspruch 7.

9. Wirtszelle, welche befähigt ist, eine Nukleinsäuresequenz nach Anspruch 1 rekombinant zu exprimieren.

10. Zellkultur, welche befähigt ist, eine Nukleinsäuresequenz nach Anspruch 1 rekombinant zu exprimieren.

11. Protein-Präparation, umfassend ein rekombinant exprimiertes Polypeptid, mit einer Aminosäuresequenz, welche durch eine Nukleinsäuresequenz nach Anspruch 1 kodiert wird.

12. Pharmazeutische Zusammensetzung, umfassend eine wirksame Menge einer Nukleinsäuresequenz nach Anspruch 1 und einen pharmazeutisch verträglichen Träger.

13. Isoliertes Polypeptid, umfassend einen Retina-spezifischen ATP-Bindungs-Cassette-Transporter, wobei die Nukleinsäuresequenz nach einem der Ansprüche 1 bis 3 für das Polypeptid kodiert.

14. Isoliertes Polypeptid nach Anspruch 13, wobei das Polypeptid eine mit Makuladegeneration verbundene Mutation umfasst.

15. Transgener, nicht-humaner Säuger, umfassend eine rekombinante Sequenz, welche eine Nukleinsäuresequenz nach Anspruch 1 umfasst, die in den Säuger oder einen Vorfahren des Säugers eingeführt wird, oder umfassend ein unterdrücktes Retina-spezifisches ATP-Bindungs-Cassette-Transporter-Gen, welches SEQ ID NR:4 umfasst.

16. Verfahren zum Screenen für ein Mittel, welches Makuladegeneration moduliert, umfassend das Vereinigen eines gereinigten Retina-spezifischen ATP-Bindungs-Cassette-Transporters, welcher eine Aminosäuresequenz umfasst, die durch eine Nukleinsäuresequenz nach Anspruch 1 kodiert wird, mit mindestens einem Mittel, welches im Verdacht steht, einen Retina-spezifischen ATP-Bindungs-Cassette-Transporter zu verändern, und die Beobachtung einer Veränderung der Aktivität des gereinigten Retina-spezifischen ATP-Bindungs-Cassette-Transporters.

17. Verfahren zum Screenen für ein Mittel nach Anspruch 16, wobei das Verfahren ein Verfahren zum Screenen für ein Mittel ist, das Makuladegeneration hemmt, wobei das Verfahren das Vereinigen des gereinigten Retina-spezifischen ATP-Bindungs-Cassette-Transporters mit mindestens einem Mittel, welches im Verdacht steht, einen Retina-spezifischen ATP-Bindungs-Cassette-Transporter zu aktivieren, und die Beobachtung einer Aktivierung des gereinigten ATP-Bindungs-Cassette-Transporters, umfasst.

18. Verfahren nach Anspruch 17, wobei der gereinigte Retina-spezifische ATP-Bindungs-Cassette-Transporter von einem Patienten stammt, welcher unter Verdacht steht, Makuladegeneration aufzuweisen.

19. Verfahren nach Anspruch 17, wobei die Aktivierung des gereinigten Retina-spezifischen ATP-Bindungs-Cassette-Transporters durch die Hemmung eines mit Makuladegeneration verbundenen Charakteristikums, ausgewählt aus der Gruppe, bestehend aus Hemmung der zentral-visuellen Beeinträchtigung, Hemmung fortschreitender bilateraler Atrophie des Makula-retinalen Pigment-Epithels, Hemmung fortschreitender bilateraler Atrophie des Neuroepithels, Hemmung von Makula-Flecken, Hemmung mittelretinaler peripherer Flecken und Hemmung Retina-spezifischer ATP-Bindungs-Cassette-Transporter Transkripte in Photorezeptorzellen, beobachtet wird.

20. Verfahren nach Anspruch 19, wobei die Makuladegeneration aus der Gruppe, bestehend aus Stargadt-Krankheit, Fundus flavimaculatus und altersbezogener Makuladegeneration, ausgewählt wird.

21. Verfahren nach Anspruch 17, weiter umfassend den Schritt des Amplifizierens der Nukleinsäuresequenz, welche für einen Retina-spezifischen ATP-Bindungs-Cassette-Transporter kodiert, unter Verwendung eines Oligonukleotid-Primers, welcher spezifisch mit der Sequenz hybridisiert.

22. Verfahren nach Anspruch 17, weiter umfassend den Schritt des Amplifizierens der Nukleinsäuresequenz, welche für einen Retina-spezifischen ATP-Bindungs-Cassette-Transporter kodiert, unter Verwendung eines Oligonukleotid-Primers, welcher spezifisch mit 17 bis 25 Nukleotiden der Sequenz hybridisiert.

23. Verfahren zum Screenen für ein Mittel nach Anspruch 16, wobei das Verfahren ein Verfahren zum Screenen für ein Mittel ist, das Makuladegeneration aktiviert, wobei das Verfahren das Vereinigen des gereinigten Wildtyp-Retina-spezifischen ATP-Bindungs-Cassette-Transporters mit mindestens einem Mittel, welches unter Verdacht steht, einen Retina-spezifischen ATP-Bindungs-Cassette-Transporter zu aktivieren, und die Beobachtung einer Hemmung des gereinigten Wildtyp-ATP-Bindungs-Cassette-Transporters, umfasst.

24. Verfahren nach Anspruch 23, wobei die Hemmung des gereinigten Retina-spezifischen ATP-Bindungs-Cassette-Transporters durch ein mit Makuladegeneration verbundenes Charakteristikum beobachtet wird, ausgewählt aus der Gruppe, bestehend aus zentral-visueller Beeinträchtigung, bilateraler Atrophie des Makula-retinalen Pigment-Epithels, bilateraler Atrophie des Neuroepithels, Makula-Flecken, mittelretinaler peripherer Flecken und Retina-spezifischer ATP-Bindungs-Cassette-Transporter-Transkripte in Photorezeptorzellen.

25. *In vitro* -Verfahren zum Diagnostizieren eines Individuums mit Makuladegeneration oder Mangel daran, umfassend das Nachweisen des Vorhandenseins oder der Abwesenheit einer mit Makuladegeneration verbundenen Mutation in einer für Retina-spezifische ATP-Bindungs-Cassette-Transporter kodierenden Nukleinsäure nach Anspruch 1 in einer Gewebeprobe des Individuums.

26. Verfahren nach Anspruch 25, wobei die Gewebeprobe aus der Gruppe, bestehend aus Blut, Haut, Serum, Speichel, Sputum, Schleim, Knochenmark, Urin, Lymphe, einer Träne, Chorion und Fruchtwasser, ausgewählt wird.

27. Verfahren nach Anspruch 25, wobei die Mutation aus der Gruppe, bestehend aus 2453 (G→A), 2588 (G→C), 2791 (G→A), 2884 del C, 3083 (C→T), 3259 (G→A), 5041 del 15, 5196 +2(T→C), 6005 +1(G→T), 6079 (C→T), 6112 (C→T), 6148 (G→C), 6229 (C→T) und 6316 (C→T), gemäß der Numerierung der Sequenz in Figur 3, ausgewählt wird.

28. Verfahren nach Anspruch 25, wobei die Mutation zu einem Aminosäuresequenz-Unterschied, ausgewählt aus der Gruppe, bestehend aus G818E, G863A, V931M, A1028V, V1072A, E1087K, VVAIC1681del, L2027F, R2038W, V2050L, R2077W und R2106C, gemäß der Numerierung der Sequenz aus Figur 3, führt.

29. Verfahren nach Anspruch 25, wobei die Mutation aus der Gruppe, bestehend aus einer Fehlsinn-Mutation, einer intragenetischer Deletion, einer intragenetischer Insertion, einer Rahmenverschiebungsmutation und einer Spleiß-Stellen-Mutation, ausgewählt ist.

30. Verfahren nach Anspruch 29, wobei die Mutation eine Fehlsinn-Mutation ist.

31. Verfahren nach Anspruch 29, wobei die Mutation eine Rahmenverschiebungsmutation ist.

32. Verfahren nach Anspruch 29, wobei die Mutation eine Spleißstellenmutation ist.

33. Verfahren nach Anspruch 25, weiter umfassend das Extrahieren von RNA oder DNA aus der Probe.

34. Verfahren nach Anspruch 25, weiter umfassend den Schritt des Amplifizierens der Nukleinsäuresequenz, welche für einen Retina-spezifischen ATP-Bindungs-Cassette-Transporter kodiert.

35. Verfahren nach Anspruch 34, wobei der Amplifizierungsschritt das Ausführen der Polymerase-Kettenreaktion umfasst.

36. Verfahren nach Anspruch 25, weiter umfassend den Schritt des Amplifizierens der Nukleinsäuresequenz, welche für einen Retina-spezifischen ATP-Bindungs-Cassette-Transporter kodiert, unter Verwendung eines Oligonukleotid-Primers, der spezifisch mit der Nukleinsäuresequenz hybridisiert.

37. Verfahren nach Anspruch 25, weiter umfassend den Schritt des Amplifizierens der Nukleinsäuresequenz, welche für einen Retina-spezifischen ATP-Bindungs-Cassette-Transporter kodiert, unter Verwendung eines Primers mit einer Nukleinsäuresequenz, ausgewählt aus der Gruppe, bestehend aus SEQ ID NRn: 40-61, 64, 65, 78, 79, 98-105, 108 und 109.

38. Verfahren nach Anspruch 34, weiter umfassend den Schritt des Vergleichens der amplifizierten Nukleinsäuresequenz von dem Individuum mit einer Kontroll-Nukleinsäuresequenz, welche für einen Wildtyp-Retina-spezifischen ATP-Bindungs-Cassette-Transporter kodiert, wobei eine Mutation in der amplifizierten Nukleinsäuresequenz des Individuums, verglichen mit der Nukleinsäuresequenz, welche für einen Wildtyp-Retina-spezifischen ATP-Bindungs-Cassette-Transporter kodiert, Makuladegeneration anzeigt.

39. *In vitro*-Verfahren zum Diagnostizieren von Makuladegeneration in einem Individuum, umfassend das Nachweisen des Vorhandenseins einer mutierten Version eines Retina-spezifischen ATP-Bindungs-Cassette-Transporters, für den eine Nukleinsäuresequenz nach Anspruch 1 kodiert.

40. Verwendung einer Nukleinsäuresequenz nach Anspruch 1, oder eines Fragments davon, zur Herstellung eines Medikaments für die Behandlung von Makuladegeneration.

41. Verwendung nach Anspruch 40, wobei das Nukleotid für einen Wildtyp-ATP-Bindungs-Cassette-Transporter mit einer Aminosäuresequenz kodiert, welche durch SEQ ID NR:4 kodiert wird.

42. Verwendung nach Anspruch 40, wobei das Medikament zur Behandlung eines Menschen ist.

43. Verwendung nach Anspruch 40, wobei die Makuladegeneration durch Stargadt-Krankheit, Fundus flavimaculatus, alters-bezogene Makuladegeneration, Retinitis pigmentosa, kombinierte Stäbchen- und Zäpfchen-Dystrophien, Zäpfchen-Dystrophien, Musterdystrophie, Ochsenaugen-Makulopathie, bedingt wird.

44. Verwendung nach Anspruch 40, wobei die Makuladegeneration durch Stargadt-Krankheit bedingt wird.

45. Kit zum Nachweis von Makuladegeneration, umfassend einen ersten Behälter, wobei der Behälter einen ersten Primer umfasst, welcher zwischen 15 und 35 Nukleotide aufweist und komplementär zu einem ersten Bereich eines Retina-spezifischen ATP-Bindungs-Cassette-Transporter-Gens ist, welches eine Nukleinsäuresequenz nach Anspruch 1 umfasst.

46. Kit nach Anspruch 45, wobei der erste Bereich des Gens ein kodierender Bereich ist.

47. Kit nach Anspruch 45, wobei der erste Bereich des Gens ein Exon flankiert.

48. Kit nach Anspruch 45, weiter umfassend einen zweiten Behälter, wobei der zweite Behälter einen zweiten Primer umfasst, welcher zwischen 15 und 35 Nukleotide aufweist und komplementär zu einem zweiten Bereich eines Retina-spezifischen ATP-Bindungs-Cassette-Transporter-Gens ist, welches eine Nukleinsäuresequenz nach Anspruch 1 umfasst.

49. Kit nach Anspruch 48, wobei der zweite Bereich des Gens ein kodierender Bereich ist.

50. Kit nach Anspruch 48, wobei der zweite Bereich des Gens ein Exon flankiert.

51. Kit nach Anspruch 45, wobei der erste Primer aus der Gruppe, bestehend aus SEQ ID NRn: 40-61, 64, 65, 78, 79, 98-105, 108 und 109, ausgewählt ist.

52. Kit nach Anspruch 48, wobei der zweite Primer aus der Gruppe, bestehend aus SEQ ID NRn: 40-61, 64, 65, 78, 79, 98-105, 108 und 109, ausgewählt ist, aber nicht mit dem ersten Primer identisch ist.

53. Kit zum Nachweis von Makuladegeneration, umfassend einen ersten Behälter, wobei der Behälter ein Primer-Paar umfasst, welches komplementär zu einem ersten und einem zweiten Bereich eines Retina-spezifischen ATP-Bindungs-Cassette-Transporter-Gens ist, welches eine Nukleinsäuresequenz nach Anspruch 1 umfasst, wobei jeder Primer zwischen 15 und 35 Nukleotide aufweist.

54. Kit nach Anspruch 53, wobei das Primer-Paar ein Exon flankiert.

55. Kit nach Anspruch 53, wobei das Primer-Paar einen kodierenden Bereich umspannt.

56. Kit nach Anspruch 53, wobei das Primer-Paar einen Primer umfasst, welcher eine Sequenz, ausgewählt aus der Gruppe, bestehend aus SEQ ID Rn: 40-61, 64, 65, 78, 79, 98-105, 108 und 109, aufweist.

57. Kit nach Anspruch 45 oder 53, weiter umfassend ein Mittel zur Visualisierung amplifizierter DNA.

58. Kit nach Anspruch 57, wobei das Mittel ein fluoreszierender Farbstoff, ausgewählt aus der Gruppe, bestehend aus Ethidiumbromid, ³²P und Biotin, ist.

59. Kit nach Anspruch 45 oder 53, weiter umfassend einen oder mehrere Größenmarker.

60. Kit nach Anspruch 45 oder 53, weiter umfassend einen oder mehrere positive oder negative Kontrollen.

61. Kit nach Anspruch 45 oder 53, weiter umfassend ein oder mehrere Restriktionsenzyme.

62. Isoliertes Oligonukleotid, umfassend eine Nukleinsäuresequenz, welche komplementär zu einer Nukleinsäuresequenz nach Anspruch 1 ist.

63. *In vitro*-Verwendung eines Oligonukleotids nach Anspruch 62, zur Hemmung der Expression Retina-spezifischen ATP-Bindungs-Cassette-Transporters in einer Zelle.

64. Isolierte Nukleinsäuresequenz, umfassend eine Nukleinsäuresequenz, ausgewählt aus der Gruppe, bestehend aus SEQ ID NRn: 40-61, 64, 65, 78, 79, 98-105, 108 und 109.

## Revendications

1. Une séquence d'acide nucléique codant un transporteur à cassette de fixation à l'ATP spécifique de la rétine dans laquelle l'acide nucléique consiste en la séquence SEQ ID NO:4.

2. La séquence d'acide nucléique selon la revendication 1 dans laquelle le transporteur à cassette de fixation à l'ATP comprend un acide aminé choisi parmi le groupe consistant en 818 (G ou E), 863 (G ou A), 931 (V ou M), 1028 (A ou V), 1072 (V ou A), 1087 (E ou K), 2027 (L ou F), 2038 (R ou W), 2050 (V ou L), 2077 (R ou W), et 2106 (R ou C) suivant la numérotation de la séquence dans la Figure 3.

3. La séquence d'acide nucléique de la revendication 1 comprenant un nucléotide choisi parmi le groupe consistant en 2453 (G ou A), 2588 (G ou C), 2791 (G ou A), 3083 (C ou T), 3215 (T ou C), 3259 (G ou A), 6079 (C ou T), 6112 (C ou T), 6148 (G ou C), 6229 (C ou T), et 6319 (C ou T) suivant la numérotation de la séquence dans la Figure 3.

4. La séquence d'acide nucléique de la revendication 3 dans laquelle le nucléotide donne une mutation non-sens.

5. La séquence d'acide nucléique de la revendication 3 dans laquelle le nucléotide donne un site d'épissage.

6. La séquence d'acide nucléique de la revendication 1 comprenant un nucléotide qui donne une mutation entraînant un décalage du cadre de lecture.

7. Un vecteur comprenant une séquence d'acide nucléique selon la revendication 1.

8. Une culture cellulaire obtenue en transformant une cellule par un vecteur d'expression selon la revendication 7.

9. Une cellule hôte capable d'exprimer par génie génétique une séquence d'acide nucléique selon la revendication 1.

10. Une culture cellulaire capable d'exprimer par génie génétique une séquence d'acide nucléique selon la revendication 1.

11. Une préparation protéique consistant en un polypeptide exprimé par génie génétique qui a une séquence d'acides aminés codée par une séquence d'acide nucléique selon la revendication 1.

12. Une composition pharmaceutique consistant en une quantité efficace d'une séquence d'un acide nucléique selon la revendication 1, et en un véhicule acceptable pharmaceutiquement.

13. Un polypeptide isolé consistant en un transporteur à cassette de fixation à l'ATP spécifique de la rétine lequel polypeptide est codé par la séquence d'acide nucléique de l'une quelconque des revendications 1 à 3.

14. Le polypeptide isolé selon la revendication 13 dans laquelle le polypeptide comprend une mutation associée à la dégénérescence maculaire.

15. Un mammifère transgénique non humain comportant une séquence recombinante consistant en une séquence d'un acide nucléique selon la revendication 1 qui est introduite dans ce mammifère ou dans un ancêtre de ce mammifère, ou qui comprend un gène réprimé codant pour le transporteur à cassette de fixation à l'ATP spécifique de la rétine comprenant la séquence SEQ ID NO:4.

16. Un procédé de détection d'un agent qui module la dégénérescence maculaire dans lequel on combine un transporteur à cassette de fixation à l'ATP spécifique de la rétine consistant en une séquence d'acides aminés codée par une séquence d'acide nucléique selon la revendication 1 et au moins un agent dont on suspecte qu'il altère le transporteur à cassette de fixation à l'ATP spécifique de la rétine et on observe une altération de l'activité de ce transporteur à cassette de fixation à l'ATP spécifique de la rétine purifié.

17. Un procédé de détection d'un agent selon la revendication 16 dans lequel le procédé est un procédé pour détecter un agent qui inhibe la dégénérescence maculaire, procédé dans lequel on combine le transporteur à cassette de fixation à l'ATP spécifique de la rétine purifié et au moins un agent dont on suspecte qu'il active le transporteur à cassette de fixation à l'ATP spécifique de la rétine et on observe une activation du transporteur à cassette de fixation à l'ATP spécifique de la rétine purifié.

18. Le procédé selon la revendication 17 dans lequel le transporteur à cassette de fixation à l'ATP spécifique de la rétine purifié provient d'un patient dont on suspecte qu'il souffre de dégénérescence maculaire.

19. Le procédé de la revendication 17 dans lequel on observe l'activation du transporteur à cassette de fixation à l'ATP spécifique de la rétine purifié par une inhibition d'une caractéristique associée à la dégénérescence maculaire choisie parmi le groupe consistant en une inhibition de la détérioration de la vision centrale, une inhibition de l'atrophie bilatérale progressive de l'épithélium pigmentaire de la rétine maculaire, une inhibition de l'atrophie bilatérale progressive du neuroépithélium, une inhibition des tâches maculaires, une inhibition des tâches périphériques au centre de la rétine, et une inhibition des transcrits du transporteur à cassette de fixation à l'ATP spécifique de la rétine dans les cellules photoréceptrices.

20. Le procédé de la revendication 19 dans lequel la dégénérescence maculaire est choisie à partir du groupe consistant de la maladie de Stargardt, du fundus flavimaculatus, et des dégénérescences maculaires liées à l'âge.

21. Le procédé de la revendication 17 comprenant de plus l'étape d'amplification de la séquence d'acide nucléique codant pour le transporteur à cassette de fixation à l'ATP spécifique de la rétine en utilisant une amorce oligonucléotidique qui s'hybride spécifiquement à cette séquence.

22. Le procédé de la revendication 17 comprenant de plus l'étape d'amplification de la séquence d'acide nucléique codant pour le transporteur à cassette de fixation à l'ATP spécifique de la rétine en utilisant une amorce oligonucléotidique qui s'hybride spécifiquement à de 17 nucléotides à 25 nucléotides de cette séquence.

23. Un procédé de détection d'un agent selon la revendication 16 dans lequel le procédé est un procédé de détection d'un agent qui active la dégénérescence maculaire, procédé dans lequel on combine le transporteur à cassette de fixation à l'ATP spécifique de la rétine purifié de type sauvage et au moins un agent dont on suspecte qu'il active la dégénérescence maculaire, puis on observe une inhibition de ce transporteur à cassette de fixation à l'ATP spécifique de la rétine purifié de type sauvage.

24. Le procédé de la revendication 23 dans lequel l'inhibition du transporteur à cassette de fixation à l'ATP spécifique de la rétine purifié est observée par une caractéristique associée à la dégénérescence maculaire choisie à partir du groupe consistant en une détérioration de la vision centrale, une atrophie bilatérale de l'épithélium pigmentaire de la rétine maculaire, une atrophie bilatérale du neuroépitéhlium, des tâches maculaires, des tâches périphériques du centre de la rétine, et des transcrits du transporteur à cassette de fixation à l'ATP spécifique de la rétine dans les cellules photoréceptrices.

25. Un procédé *in vitro* pour diagnostiquer chez un individu une dégénérescence maculaire ou son absence, comprenant en la détection de la présence ou de l'absence d'une mutation associée à la dégénérescence maculaire dans un acide nucléique qui code pour un transporteur à cassette de fixation à l'ATP spécifique de la rétine selon la revendication 1 dans un échantillon tissulaire de cet individu.

26. Le procédé de la revendication 25 dans lequel l'échantillon tissulaire est choisi parmi le groupe consistant du sang, de la peau, du sérum, de la salive, du crachat, de la muqueuse, de la moelle osseuse, de l'urine, de la lymphe, une larme, le chorion, et le fluide amniotique.

27. Le procédé de la revendication 25 dans lequel la mutation est choisie parmi le groupe consistant en : 2453 (G→A), 2588 (G→C), 2791 (G→ A), 2884 del C, 3083 (C→T), 3259 (G→A), 5041del15, 5196 +2(T→C), 6005 + 1 (G→ T), 6079 (C→ T), 6112 (C→T), 6148 (G→ C), 6229 (C→ T), et 6319 (C→ T) suivant la numérotation de la séquence dans la Figure 3.

28. Le procédé de la revendication 25 dans lequel la mutation donne une différence dans la séquence d'acides aminés choisie à partir du groupe consistant en G818E, G863A, V931M, A1028V, V1072A, E1087K, VVAIC1681del, L2027F, R2038W, V2050L, R2077W, et R2106C suivant la numérotation de la séquence dans la Figure 3.

29. Le procédé de la revendication 25 dans lequel la mutation est choisie à partir du groupe consistant en une mutation non-sens, une délétion intragénique, une insertion intragénique, une mutation entraînant un décalage du cadre de lecture, et une mutation d'un site d'épissage.

30. Le procédé de la revendication 29 dans lequel la mutation est une mutation non-sens.

31. Le procédé de la revendication 29 dans lequel la mutation est une mutation entraînant un décalage du cadre de lecture.

32. Le procédé de la revendication 29 dans lequel la mutation est une mutation d'un site d'épissage.

33. Le procédé de la revendication 25 consistant de plus à extraire de l'ARN ou de l'ADN de l'échantillon.

34. Le procédé de la revendication 25 comprenant de plus une étape d'amplification de la séquence d'acide nucléique codant pour le transporteur à cassette de fixation à l'ATP spécifique de la rétine.

35. Le procédé de la revendication 34 dans lequel l'étape d'amplification consiste à effectuer une réaction d'amplification en chaîne par polymérase.

36. Le procédé de la revendication 25 comprenant de plus une étape d'amplification de la séquence d'acide nucléique codant pour un transporteur à cassette de fixation à l'ATP spécifique de la rétine en utilisant une amorce oligonucléotidique qui s'hybride spécifiquement à cette séquence d'acide nucléique.

37. Le procédé de la revendication 25 comprenant de plus une étape d'amplification de la séquence d'acide nucléique codant pour un transporteur à cassette de fixation à l'ATP spécifique de la rétine en utilisant une amorce qui a une séquence d'acide nucléique choisie parmi le groupe consistant en des séquences SEQ ID NO: 40 à 61, 64, 65, 78, 79, 98 à 105, 108 et 109.

38. Le procédé de la revendication 34 comprenant de plus l'étape de comparaison de la séquence d'acide nucléique amplifiée à partir de l'individu à une séquence d'acide nucléique témoin codant pour le transporteur à cassette de fixation à l'ATP spécifique de la rétine de type sauvage dans laquelle une mutation de la séquence amplifiée à partir de l'individu par rapport à la séquence d'acide nucléique qui code pour le transporteur à cassette de fixation à l'ATP spécifique de la rétine de type sauvage indique une dégénérescence maculaire.

39. Un procédé *in vitro* pour diagnostiquer une dégénérescence maculaire chez un individu consistant à détecter la présence d'une version mutée d'un transporteur à cassette de fixation à l'ATP spécifique de la rétine codé par une séquence d'acide nucléique de la revendication 1.

40. Utilisation d'une séquence d'acide nucléique selon la revendication 1, ou de l'un de ses fragments, pour la fabrication d'un médicament destiné au traitement de la dégénérescence maculaire.

41. L'utilisation de la revendication 40 dans laquelle le nucléotide code pour un transporteur à cassette de fixation à l'ATP spécifique de la rétine de type sauvage qui a une séquence aminoacide codée par SEQ ID NO:4.

42. L'utilisation de la revendication 40 dans laquelle le médicament est destiné au traitement d'un humain.

43. L'utilisation de la revendication 40 dans laquelle la dégénérescence maculaire est due à la maladie de Stargardt, le fundus flavimaculatus, et les dégénérescences maculaires liées à l'âge, la rétinite pigmentaire, des distrophies combinées des bâtonnets et des cônes, des dystrophies des cônes, la dystrophie maculaire réticulée ("pattern dystrophy"), ou une dépigmentation en oeil de boeuf.

44. L'utilisation de la revendication 40 dans laquelle la dégénérescence maculaire est due à la maladie de Stargardt.

45. Une trousse de détection de la dégénérescence maculaire consistant en un premier récipient, dans laquelle ce récipient contient une première amorce qui a une longueur comprise entre 15 et 35 nucléotides et qui est complémentaire d'une première région d'un gène d'un transporteur à cassette de fixation à l'ATP spécifique de la rétine comprenant une séquence d'acide nucléique selon la revendication 1.

46. La trousse de la revendication 45 dans laquelle la première région du gène est une région codante.

47. La trousse de la revendication 45 dans laquelle la première région du gène flanque un exon.

48. La trousse de la revendication 45 qui consiste de plus en un second récipient dans laquelle ce second récipient contient une seconde amorce qui a une longueur comprise entre 15 et 35 nucléotides et qui est complémentaire d'une seconde région d'un gène d'un transporteur à cassette de fixation à l'ATP spécifique de la rétine comprenant une séquence d'acide nucléique selon la revendication 1.

49. La trousse de la revendication 48 dans laquelle la seconde région du gène est une région codante.

50. La trousse de la revendication 48 dans laquelle la seconde région du gène flanque un exon.

51. Une trousse selon la revendication 45, dans laquelle la première amorce est choisie parmi le groupe consistant des séquences SEQ ID NO: 40 à 61, 64, 65, 78, 79, 98 à 105, 108 et 109.

52. La trousse de la revendication 48, dans laquelle la seconde amorce est choisie parmi le groupe consistant des séquences SEQ ID NO: 40 à 61, 64, 65, 78, 79, 98 à 105, 108 et 109, mais n'est pas identique à la première amorce.

53. Une trousse de détection de la dégénérescence maculaire consistant en un premier récipient, dans laquelle ce récipient contient une paire d'amorces qui sont complémentaires d'une première et d'une seconde région d'un gène d'un transporteur à cassette de fixation à l'ATP spécifique de la rétine comprenant une séquence d'acide nucléique selon la revendication 1 dans laquelle chaque amorce a une longueur comprise entre 15 et 35 nucléotides.

54. La trousse de la revendication 53 dans laquelle la paire d'amorces flanque un exon.

55. La trousse de la revendication 53 dans laquelle la paire d'amorces englobe une région codante.

56. La trousse de la revendication 53 dans laquelle la paire d'amorce consiste en une amorce ayant une séquence choisie parmi le groupe consistant des séquences SEQ ID NO: 40 à 61, 64, 65, 78, 79, 98 à 105, 108 et 109.

57. La trousse de la revendication 45 ou 53 comprenant de plus un moyen pour visualiser l'ADN amplifié.

58. La trousse de la revendication 57 dans laquelle le moyen est un colorant fluorescent choisi parmi le groupe consistant du bromure d'éthidium, du ³²P et de la biotine.

59. La trousse de la revendication 45 ou 53 comprenant de plus un ou plusieurs marqueurs de poids moléculaire.

60. La trousse de la revendication 45 ou 53 comprenant de plus un ou plusieurs témoins positifs ou négatifs.

61. La trousse de la revendication 45 ou 53 comprenant de plus un ou plusieurs enzymes de restriction.

62. Un oligonucléotide isolé comprenant une séquence d'acide nucléique complémentaire à une séquence d'un acide nucléique selon la revendication 1.

63. Utilisation *in vitro* d'un oligonucléotide selon la revendication 62 pour inhiber l'expression d'un transporteur à cassette de fixation à l'ATP spécifique de la rétine dans une cellule.

64. Une séquence d'acide nucléique isolé comprenant une séquence d'acide nucléique choisie parmi le groupe consistant des séquences SEQ ID NO: 40 à 61, 64, 65, 78, 79, 98 à 105, 108 et 109.
